# EUROPEAN PATENT SPECIFICATION

(11) **EP 0 918 872 B1**
(45) Date of publication and mention of the grant of the patent: **20.02.2008**
(21) Application number: 97937087.1
(22) Date of filing: 01.08.1997
(51) Int. Cl.: C12N 15/62, A61K 39/395, A61K 38/17, A61K 47/48, A61K 51/10, C07K 16/30, C07K 16/46, C07K 16/00, C12N 15/13, C12N 1/21, C12N 5/10, C07K 19/00

(54) **A METHOD FOR INHIBITING IMMUNOGLOBULIN-INDUCED TOXICITY RESULTING FROM THE USE OF IMMUNOGLOBULINS IN THERAPY AND IN VIVO DIAGNOSIS**
EIN VERFAHREN ZUR INHIBIERUNG IMMUNGLOBULININDUZIERTER TOXIZITÄT AUFGRUND VON DER VERWENDUNG VON IMMUNOGLOBINEN IN THERAPIE UND IN VIVO DIAGNOSTIK
PROCEDE SERVANT A INHIBER LA TOXICITE PROVOQUEE PAR LES IMMUNOGLOBULINES PROVENANT DE L'UTILISATION D'IMMUNOGLOBULINES EN THERAPIE ET EN DIAGNOSTIC IN VIVO

(30) Priority: 02.08.1996 US 23033 P
(43) Date of publication of application: 02.06.1999
(73) Proprietor: Bristol-Myers Squibb Company, Princeton, New Jersey 08543-4000 (US); Rosok, Mae Joanne, Seattle, WA 98155 (US)
(72) Inventor: ROSOK, Mae Joanne, Seattle, WA 98155 (US); YELTON, Dale, E., Seattle, WA 98112 (US)
(74) Representative: Josif, Albert
(86) International application number: PCT/US1997/013562
(87) International publication number: WO 1998/005787

(56) References cited:
- EP-A- 0 699 756
- S. GILLIES ET AL.: "Antigen binding and biological activities of engineered mutant chimeric antibodies with human tumor specificities." HUMAN ANTIBODIES AND HYBRIDOMAS, vol. 1, no. 1, 1990, STONEHAM, MA, USA, pages 47-54, XP002050448
- G. SCHREIBER ET AL.: "An unmodified anticarcinoma antibody, BR96, localizes to and inhibits the outgrowth of human tumors in nude mice." CANCER RESEARCH, vol. 52, no. 12, 15 June 1992, BALTIMORE, MD, USA, pages 3262-3266, XP002050449
- A. DUNCAN ET AL.: "The binding site for C1q on IgG." NATURE, vol. 332, no. 6166, 21 April 1988, LONDON, GB, pages 738-740, XP002050450 cited in the application
- J. LUND ET AL.: "Human FcgammaRI and FcgammaRII interact with distinct but overlapping sites on human IgG." THE JOURNAL OF IMMUNOLOGY, vol. 147, no. 8, 15 October 1991, BALTIMORE, MD, USA, pages 2657-2662, XP002050451 cited in the application
- Y. XU ET AL.: "Residue at position 331 in the IgG1 and IgG4 CH2 domains contributes to their differential ability to bind and activate complement." THE JOURNAL OF BIOLOGICAL CHEMISTRY, vol. 269, no. 5, 4 February 1994, BALTIMORE, MD, USA, pages 3469-3474, XP002050452 cited in the application
- T. MICHAELSEN ET AL.: "One disulfide bond in front of the second heavy chain constant region is necessary and sufficient for effector functions of human IgG3 without a genetic hinge." PROCEEDINGS OF THE NATIONAL ACADEMY OF SCIENCES OF THE USA, vol. 91, no. 20, 27 September 1994, WASHINGTON, DC, USA, pages 9243-9247, XP002050453
- L. TAN ET AL.: "Influence of the hinge region on complement activation, C1q binding, and segmental flexibility in chimeric human immunoglobulins." PROCEEDINGS OF THE NATIONAL ACADEMY OF SCIENCES OF THE USA, vol. 87, no. 1, January 1990, WASHINGTON, DC, USA, pages 162-166, XP002050454
- CANFIELD S. ET AL.,: J. EXP. MED., vol. 173, 1991, pages 1483-1491,
- MORGAN A. ET AL.: IMMUNOLOGY, vol. 86, 1995, pages 319-324,

## Description

### TECHNICAL FIELD OF THE INVENTION

The present invention relates to methods for inhibiting or reducing immunoglobulin-induced toxicity resulting from therapy or in vivo diagnosis. Specifically, in lieu of using unmodified antibodies or recombinant binding proteins for in vivo use, the invention provides the use of modified antibodies or recombinant binding proteins which have been structurally altered in the constant domain so that upon administration immunoglobulin-induced toxicity is reduced or inhibited.

### BACKGROUND OF THE INVENTION

Over the years investigators have attempted to harness the immune system for therapeutic use. Immunoglobulin (Ig) molecules which constitute an important part of the immune system are of great interest because they (1) react with a diverse family of ligands, (2) possess different effector functions and (3) are of great biological importance. Despite its potential, a persistent problem with immunoglobulin immunotherapy has been, among other problems, the toxic effect to normal cells of using antibodies which recognize both normal and diseased cells. This problem is far-reaching because the majority of antibodies presently available recognize a target located on both normal and diseased cells (Slavin-Chiorini, et al., Int. J. Cancer 53: 97-103 (1993)).

The constant region can promote cell death through antibody dependent cell mediated cytotoxicity (ADCC) or by complement dependent cytotoxicity (CDC). Despite the deletion of portions of the constant region, particularly the CH₂ domain, the antigen binding function can be retained (D. Yelton, M. Scharf, Mutant monoclonal antibody with alterations in biological functions, J. Exp. Methods. 156:1131-1148 (1982)).

Others have generated a CH₂-deleted antibody (Mueller et al., Proc. Natl. Acad. Sci. USA 87: 5702-5705 (1990)). Their findings provide that the CH₂-deleted antibody was cleared from the blood of tumor-bearing mice much faster than the corresponding intact antibody. Other in vivo findings also confirmed that a CH₂-deleted antibody, designated ch14.18DCH2, is a potentially useful reagent for radioimmunodetection of human tumors because of its reduced immunogenicity, increased target specificity, and rapid clearance from circulation (Mueller et al., Proc. Natl. Acad. Sci. USA 87: 5702-5705 (1990).

G. J. Schreiber et al, Cancer Research vol. 52, 1992, p. 3262-3266 disclose the anticarcinogenic murine BR96 antibody F(ab')₂ IgG3 polypeptide and an IgG1 class switched variant of the original IgG3 BR96 antibody which have no antibody-dependent cellular cytotoxicity (ADCC) and no complement-dependent cytotoxicity (CDC) effector functions. S. D. Gillies et al, Human Antibodies and Hybridomas, vol. 1, 1990, p. 47-54 disclose antigen binding and biological activities of engineered mutant chimeric antibodies with human tumor specificities. The constant region of the IgG chain was structurally altered by either deleting the CH2 domain or point mutating two cysteine residues at the hinge region of the IgG molecule. The CH2 deletion led to a drastically reduced ADCC and CDC activity. The cysteine mutation resulted in a greatly reduced ADCC activity and a reduced, but still significant, ability to mediate CDC. G. J. Weiner et al, Journal of Immunology, vol. 152, 1994, p. 2385-2392 disclose a bispecific anti-CD3 x antitumor F(ab')₂ antibody being used for immune therapy. The F_{c} part of the antibody has been removed to reduce the toxic side effects encountered in preliminary patient studies, such as ADCC and non-specific T cell activation. A. R. Duncan et al, Nature, vol. 332, 1988, p. 738-740 disclose that certain point mutations at the residues 318 (Glu), 320 (Lys) and 322 (Lys) on the CH2 domain of different IgG isotypes reduce CDC and that there are IgG isotypes such as IgG4 which are non-lytic and therefore do not induce a CDC response. Y. Xu et al, The Journal of Biological Chemistry, vol. 269, 1994, p. 3469-3474 disclose that the C-terminal region of the CH2 domain (residues 292-340) appears to be responsible for the isotype-specific differences in complement binding and induction of the CDC response. In addition, the residue at position 331 was analyzed and found to be important for C1q binding and complement activation in human IgG1. A point mutation from Pro³³¹ to Ser³³¹ abolished this activity whereas a Pro³³¹ point mutation in the IgG4 isotype which is usually inactive restored the lytic activity of said isotype. J. Lund et al, The Journal of Immunology, vol. 147, 1991, p. 2657-2662 disclose the FcγRI and FcγRII binding sites on the CH2 domain on the human IgG3 isotype being responsible for the toxic ADCC response in subjects treated with antibodies. The binding sites partially overlap at the region of residues 234 to 239 on human IgG1 and IgG3 isotypes. A binding to human IgG2 cannot be detected. Furthermore, a point mutation at position 235 resulted in the greatest reduction of FcγRI binding and point mutations at positions 234 and 237 resulted in the greatest reduction of FcγRII binding. A. Morgan et al, Immunology, 1995, vol. 86, p. 319-324 disclose that changing the leucine 235 in the CH2 region of IgG1 abolished human complement lysis and changing the glycine at 237 to alanine of IgG1 reduced human complement lysis. Exchanging the whole region 233-236 with the sequence found in human IgG2 abolished human complement lysis. In contrast, a change in the previously described Clq-binding motif, from lysine at 320 to alanine, had no effect on IgG 1-mediated complement lysis. S. M. Canfield et al, J. Exp. Med., vol. 173, June 1991, p. 1483-1491 disclose an IgG antibody having two mutations at positions 234 and 331 having a reduced Fcy receptor binding activity and therefore a reduced ADCC response.

Generally, whole antibody molecules are composed of two heavy (H) and two light (L) chains which are held together by covalent bonds (disulfide) and non-covalent interactions. Each chain contains a variable region (V) and a constant region (C). The variable regions at the amino termini of the two chains form the antigen binding region. The constant region of the H chain has three components or domains. Occasionally, the first constant region domain (CH₁) interacts with the C region of the L chain through hydrophobic interactions and generally a disulfide bond, depending on isotype. The next C region stretch is the hirige-acting disulfide bond stably introduced between two H chains. The second constant region domain (CH₂) is adjacent to the hinge region. CH₂ contains sequences important for effector functions of the antibody, such as the sequences responsible for complement fixation, and Fc receptor binding The third constant region domain (CH₃) is located at the carboxyl terminus of the H chain, and is considered to play an important role in H chain assembly as well as some C region functions.

Today many antibodies in clinical trials are directed against tumor associated antigens. Most tumor associated antigens are not tumor specific but are also generally found on the cell surface of some normal, non-tumorigenic cells. The clinical use of some antibodies directed against tumor associated antigens are limited because of the toxicity associated with their use. Therefore, there is a need for methods for inhibiting toxicity associated with immunoglobulin use in the field of disease therapy (e.g., therapy for tumors, kidney disease, and the like) and in vivo diagnosis.

We addressed this need by discovering methods for inhibiting or reducing toxicity to normal cells generally associated with immunoglobulin immunotherapy or in vivo diagnosis, wherein the immunoglobulin recognizes both diseased and normal cells. Our discovery involves generating immunoglobulin molecules or Ig fusion proteins having structurally altered constant regions which inhibit or reduce immunoglobulin-induced toxicity.

### SUMMARY OF THE INVENTION

Disclosed herein are methods for inhibiting immunoglobulin-induced toxicity by using known immunoglobulin or Ig fusion protein molecules which are structurally altered in their constant regions so that the resulting structurally altered immunoglobulin or Ig fusion protein molecules exhibit reduced or inhibited toxicity in vivo compared to their original unmodified counterparts.

The present invention first provides a BR96 antibody having a human IgG1 constant region which has been structurally altered in the CH2 domain wherein leucine at amino acid position 235 is mutated to alanine; glycine at amino acid position 237 is mutated to alanine; glutamic acid at amino acid position 318 is mutated to serine; lysine at amino acid position 320 is mutated to serine; and lysine at amino acid position 322 is mutated to serine.

The present invention also provides a BR96 antibody having a human IgG1 constant region which has been structurally altered in the CH2 domain wherein leucine at amino acid position 235 is mutated to alanine; glycine at amino acid position 237 is mutated to alanine; and proline at amino acid position 331 is mutated to alanine.

In addition, the present invention provides a BR96 antibody having a human IgG1 constant region which has been structurally altered in the CH2 domain wherein leucine at amino acid position 235 is mutated to alanine; glycine at amino acid position 237 is mutated to alanine; glutamic acid at amino acid position 318 is mutated to serine; lysine at amino acid position 320 is mutated to serine; lysine at amino acid position 322 is mutated to serine; and proline at amino acid position 331 is mutated to alanine.

Other aspects of the invention are claimed in appended claims 4-18. Further embodiments not encompassed by the claimed invention are also disclosed in the present specification.

Structural alteration of the constant region may be effected in a number of ways as long as it results in reducing or inhibiting immunoglobulin-induced toxicity.

Structural alteration of the constant region is effected by deletion of the entire constant region. In another embodiment, only the CH₂ domain is deleted. In another embodiment, only that portion of the CH₂ domain that binds the Fc receptor is deleted. In yet another embodiment, only that portion of the CH₂ domain that binds the complement component C1q is deleted. Alternatively, in another embodiment, multiple deletions in discrete Fc receptor and complement component binding domains are effected.

Alternatively, structural alteration is effected by single or multiple mutations in the CH₂ domain such as amino acid insertions and substitutions. The mutation or mutations must result in inhibiting immunoglobulin-induced toxicity. By way of example, the amino acids in multiple toxicity associated domains in the constant region can be altered so as to render the constant region unable to mediate a ADCC response or activate complement thereby inhibiting immunoglobulin induced toxicity resulting from immunotherapy. Alternatively, multiple amino acids in a single toxicity associated domain in the constant region can be altered.

Further alternatively, structural alteration can be effected by isotype switching resulting in an altered immunoglobulin molecule that either does not induce toxicity or induces some limited toxicity but does not cause a harmful effect. For example, isotype switching can result in the constant region being unable to mediate a CDC or ADCC response or some other activity which mediates toxicity.

### BRIEF DESCRIPTION OF THE FIGURES

Figure 1 is a line graph showing plasma clearance in high Le^{Y} expressing dogs using chimeric BR96 versus constant region mutant of cBR96-2.
Figure 2 is a schematic diagram of a plasmid designated pTWD-cJVK.L1 including the chimeric (c)BR96-light chain (SEQ ID NO. 11).

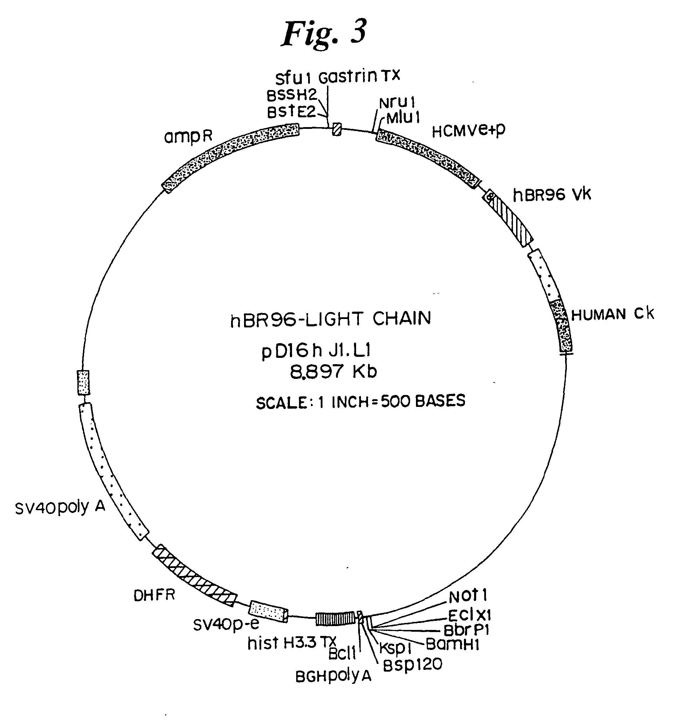

Figure 3 is a schematic diagram of a plasmid designated pD16hJ1.L1 including the human (h)BR96-light chain (SEQ ID NO. 13).
Figure 4 is a schematic diagram of a plasmid, designated pD17-hJm14-dCH2.H1, of hBR96-2A (i.e., human mutant BR96 having the H1, H2, and H3 mutations and the CH₂ deletion (PCT Application No. 95/305444, published March 6, 1996)).
Figure 5 is a schematic diagram of a plasmid, designated pD17-cJ-dCH2.H1, of cBR96-A (SEQ ID NO. 10) (i.e., chimeric BR96 having the CH₂ deletion (PCT Application No. 95/305444, published March 6,1996)).

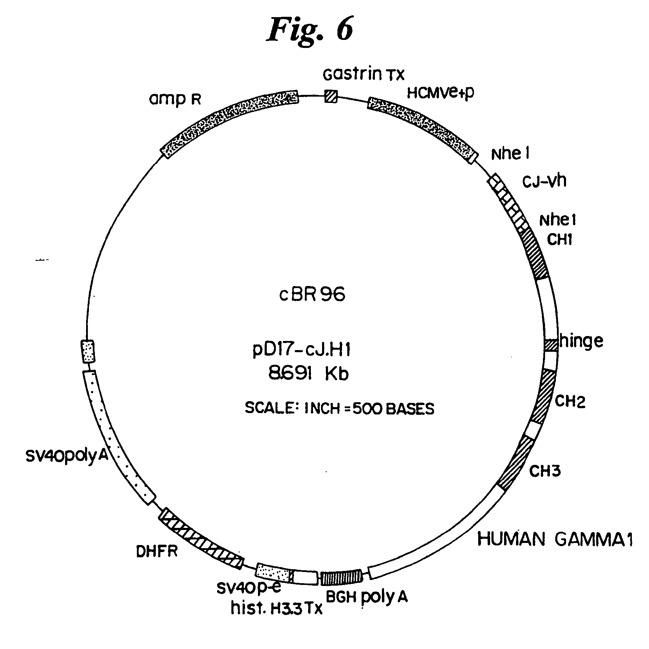

Figure 6 is a schematic diagram of a plasmid, designated pD17-cJ.H1, of cBR96.

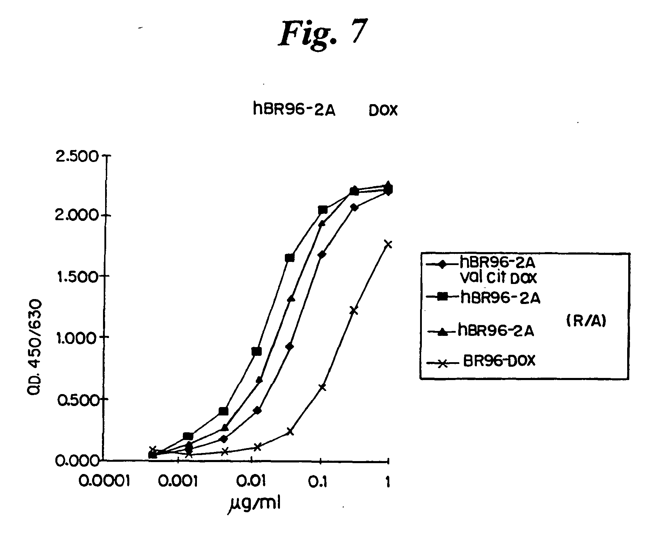

Figure 7 is a line graph showing the results of an ELISA assay of (1) hBR96-2A-Dox to Le^{y} (closed diamond), (2) hBR96-2A to Le^{y} (96:0006A2 R/A)(closed square), (3) hBR96-2A to Le^{y} (96:0006B R/A)(closed triangle), and BR96-Dox to Le^{y} (X).

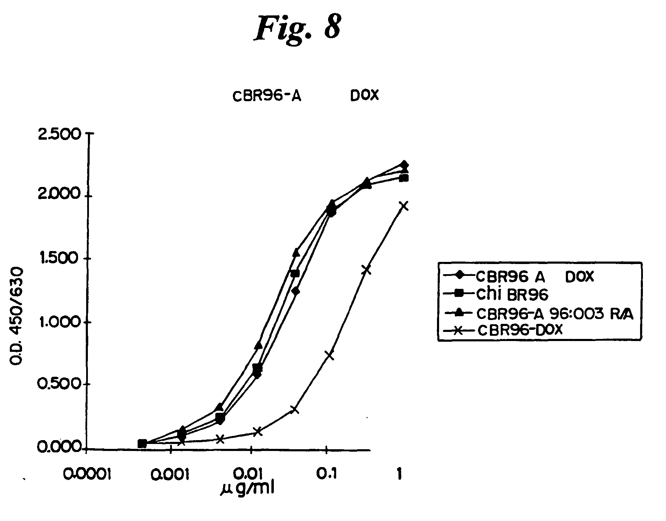

Figure 8 is a line graph showing the results of an ELISA assay of (1) BR96-A-Dox to Le^{y} (closed diamond), (2) chiBR96 to Le^{y} (closed square), (3) cBR96-A to Le^{y} (96:0003 R/A)(closed triangle), and cBR96-Dox to Le^{y} (X).

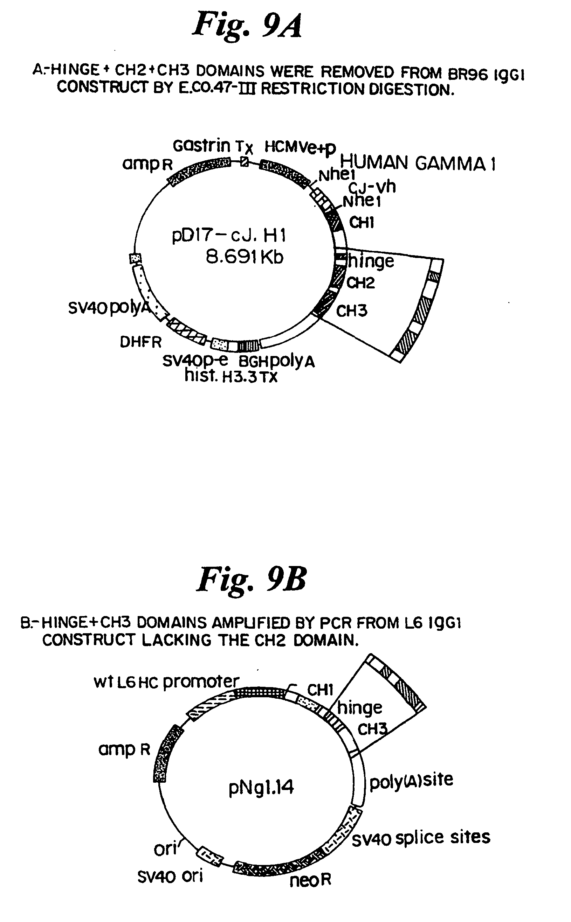

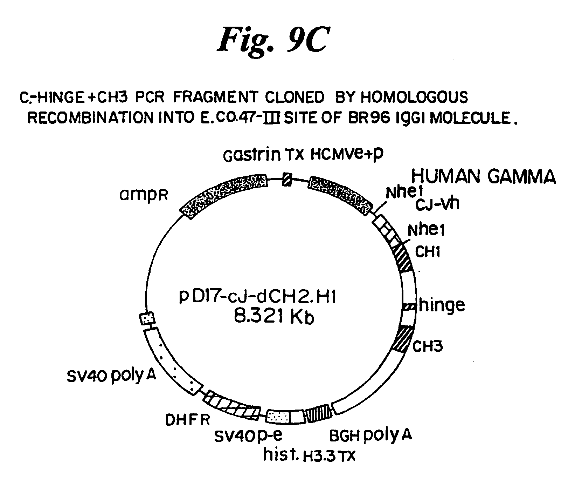

Figures 9a-c are schematic diagrams showing the steps for deleting a CH₂ domain.

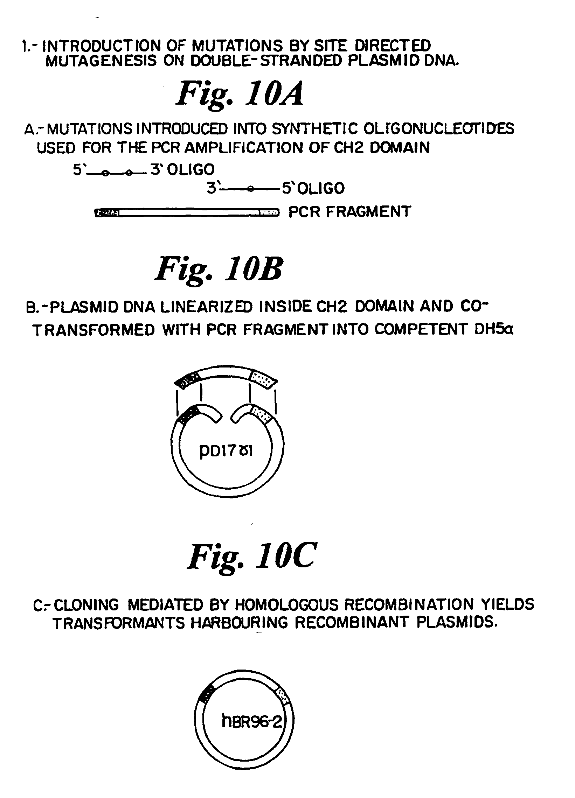

Figures 10a-c are schematic diagrams showing the construction of BR96 IgG1 CH₂ domain point mutations.
Figure 11 is a schematic diagram showing the construction of the pNgl/14 vector.
Figure 12 is a schematic diagram showing the construction of pD17-hBR96-2.
Figure 13 is a schematic diagram showing the construction of pD17-hJm14-dCH2.H1.

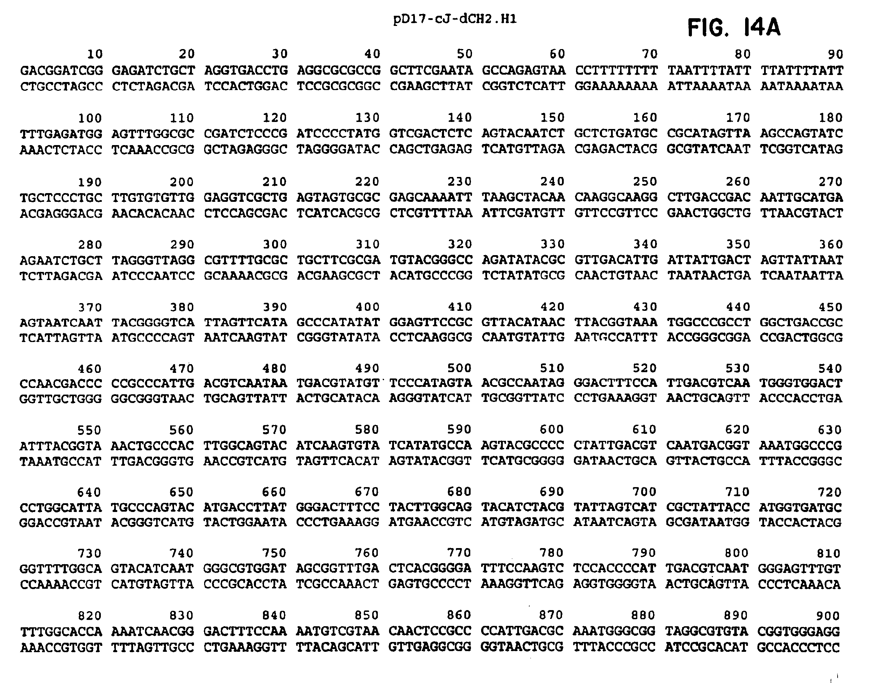

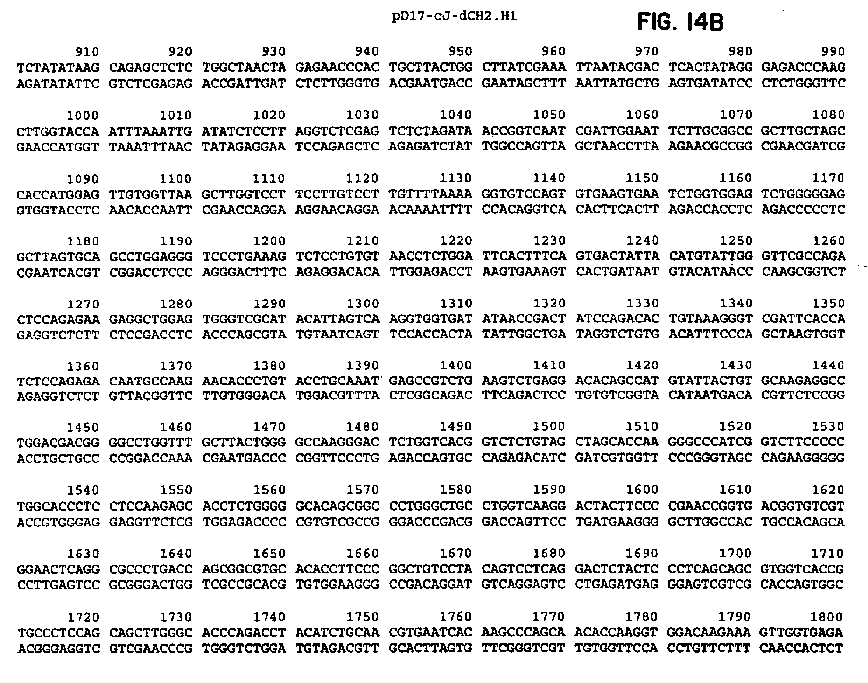

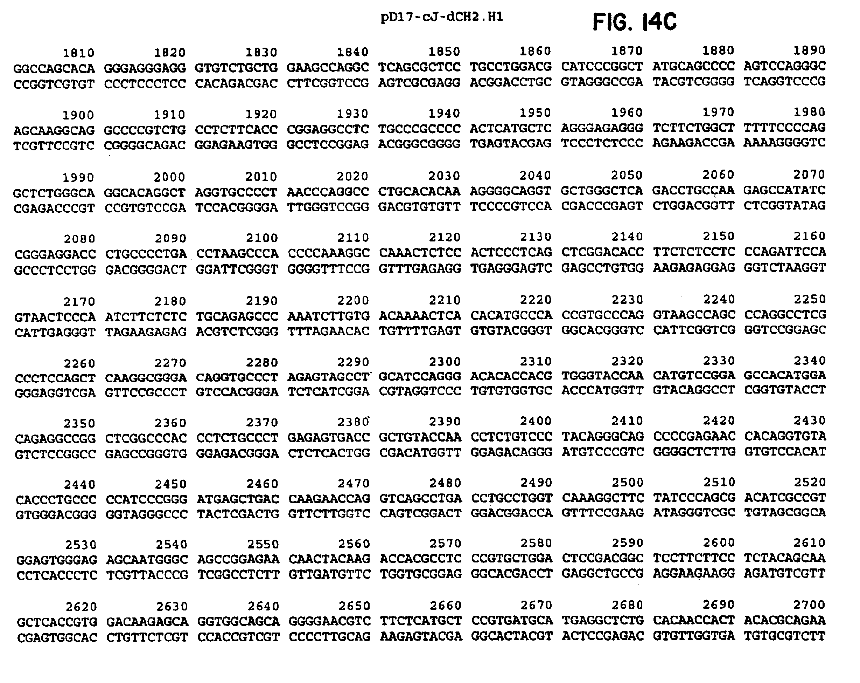

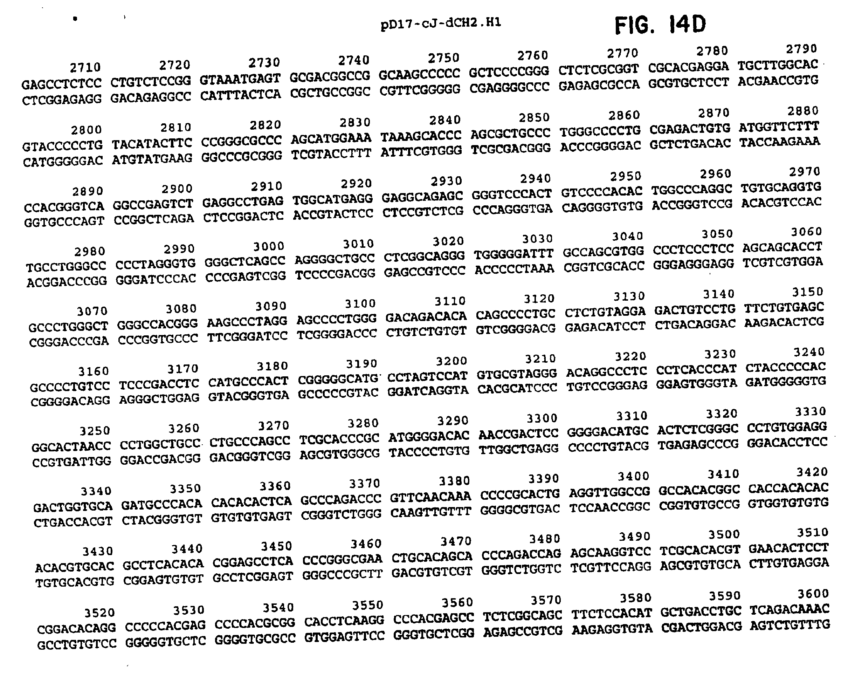

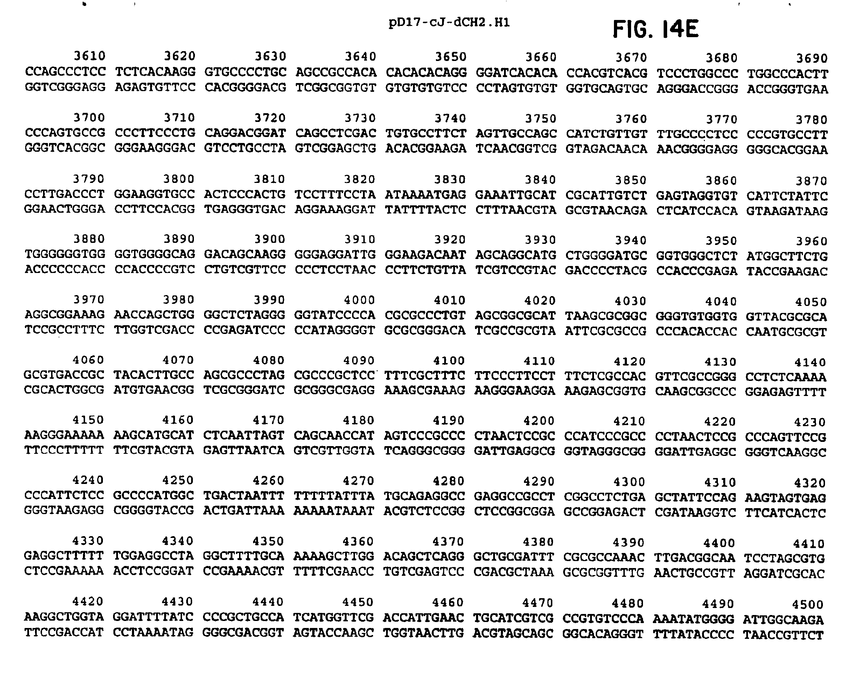

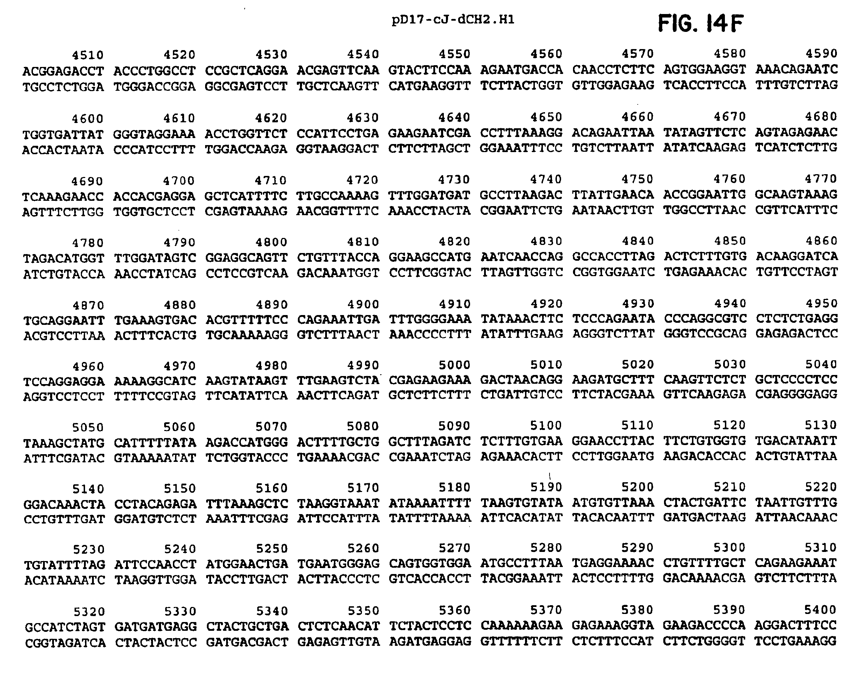

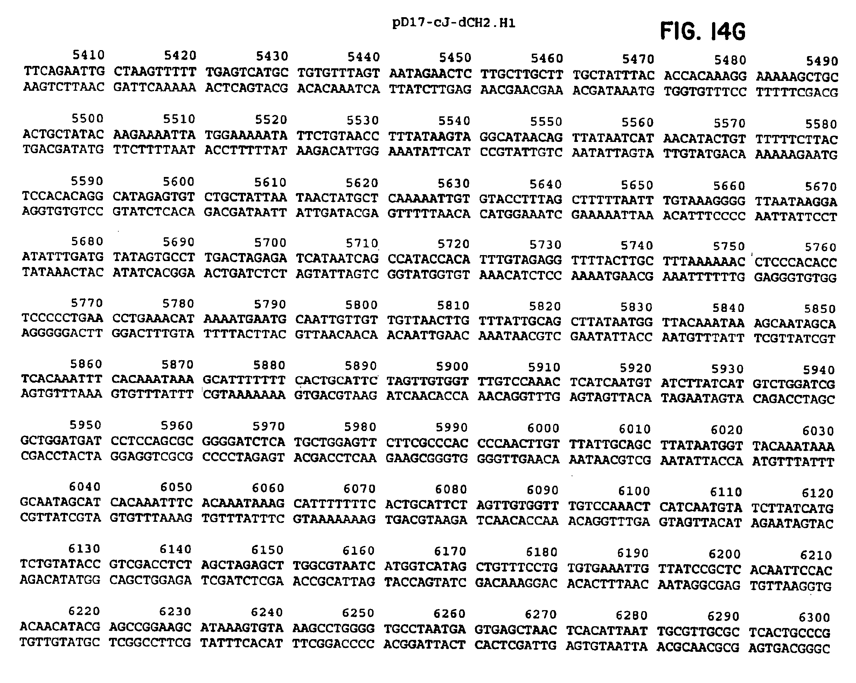

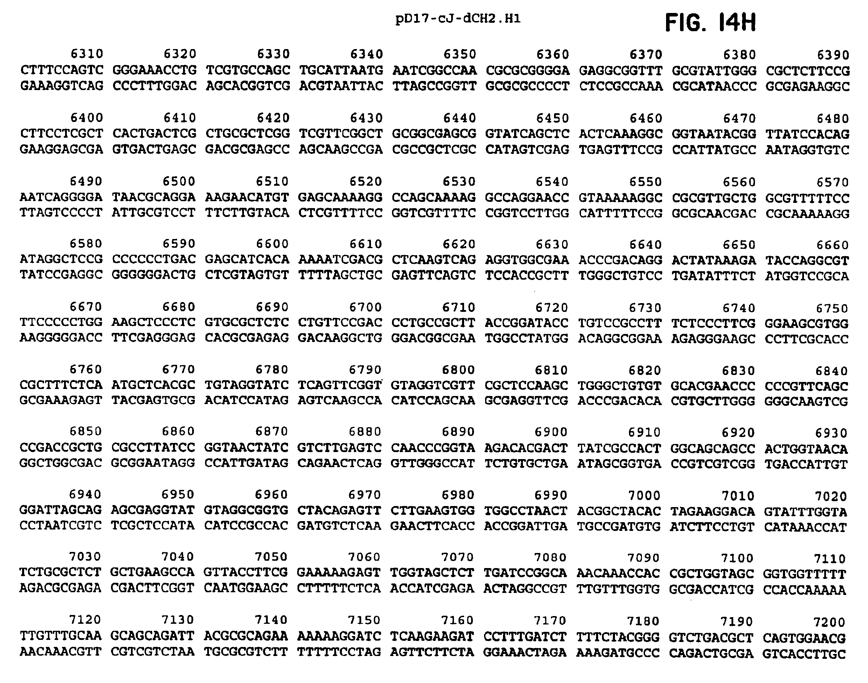

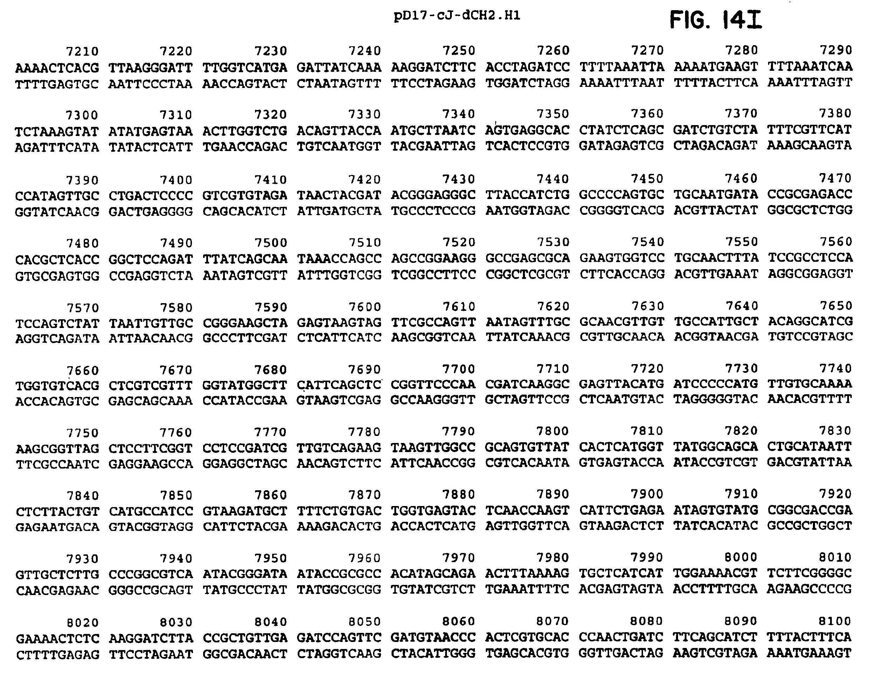

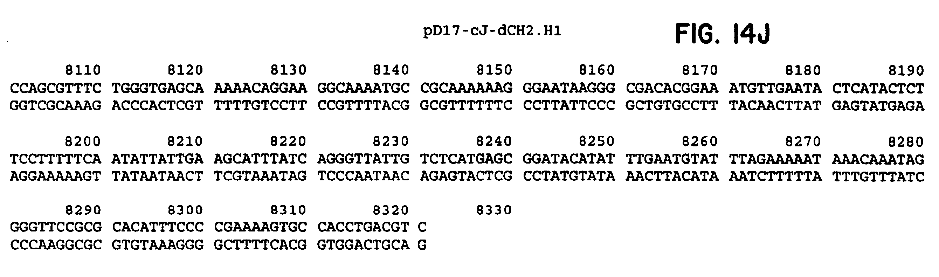

Figures 14A-J are the nucleic acid sequence of pD17-cJ-dCH2.H1, the plasmid shown in Figure 5, chimeric BR96 having the CH₂ deletion.

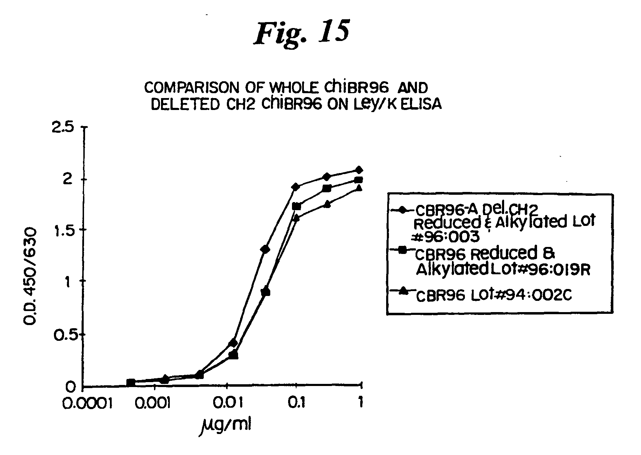

Figure 15 is a line graph showing the results of an ELISA assay comparing whole chiBR96 and deleted CH₂ chiBR96 on Le^{y}.

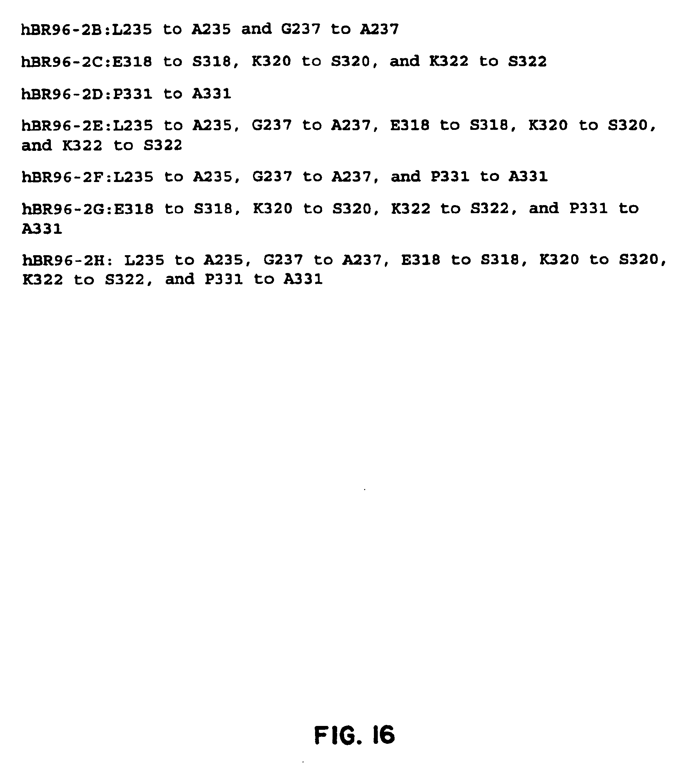

Figure 16 is a description of the seven structural alterations.

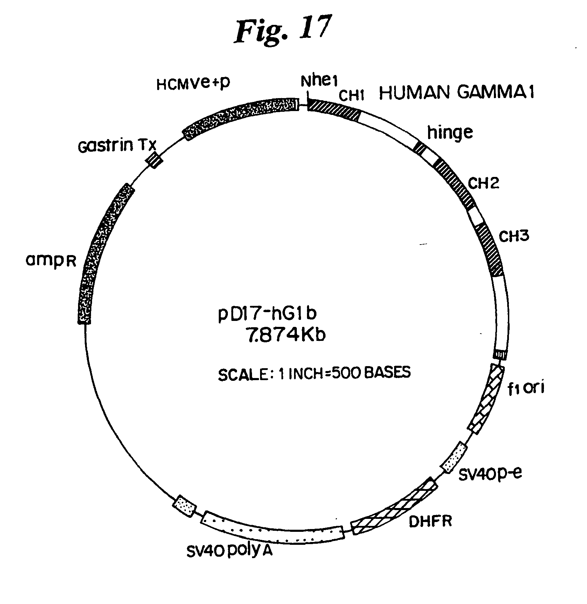

Figure 17 is a schematic diagram of a plasmid designated pD I 7-hG 1 b.
Figures 18A-F are the nucleic acid sequence pf pD 17-hJm 14.H 1.

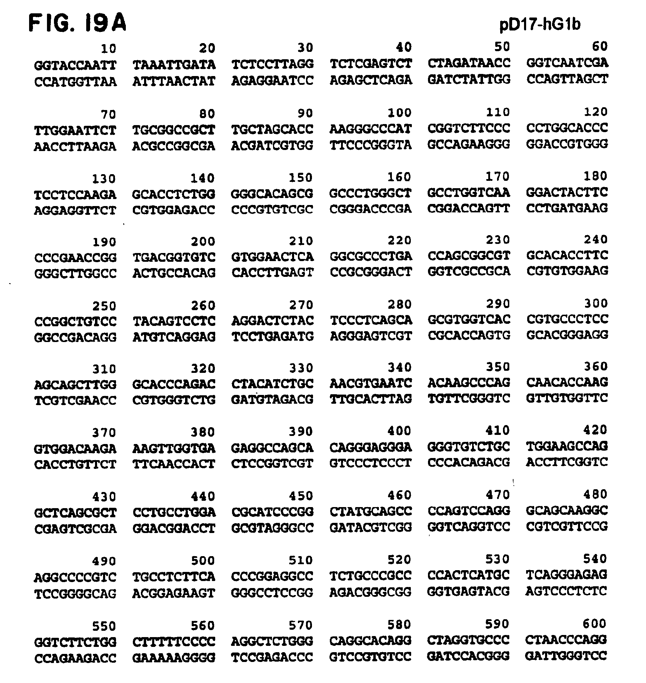

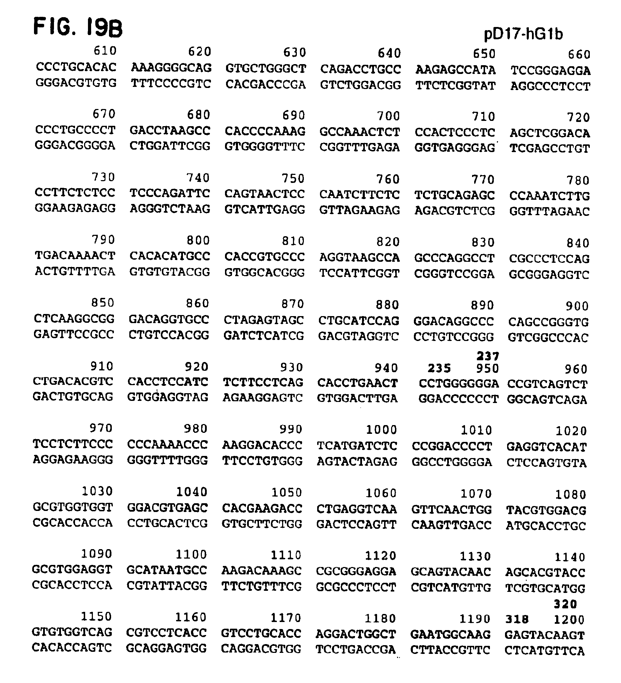

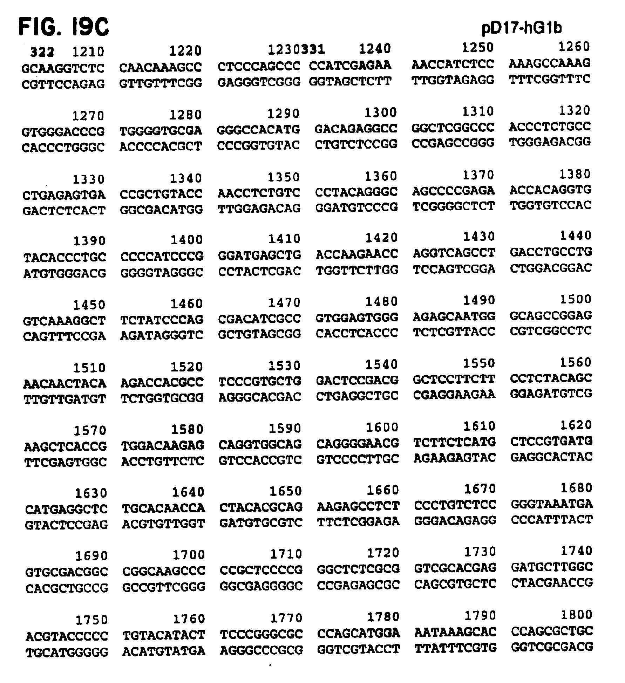

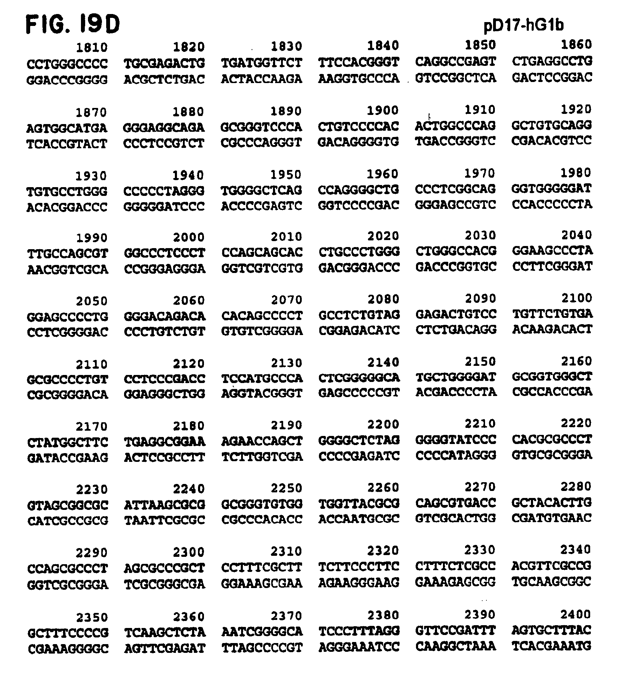

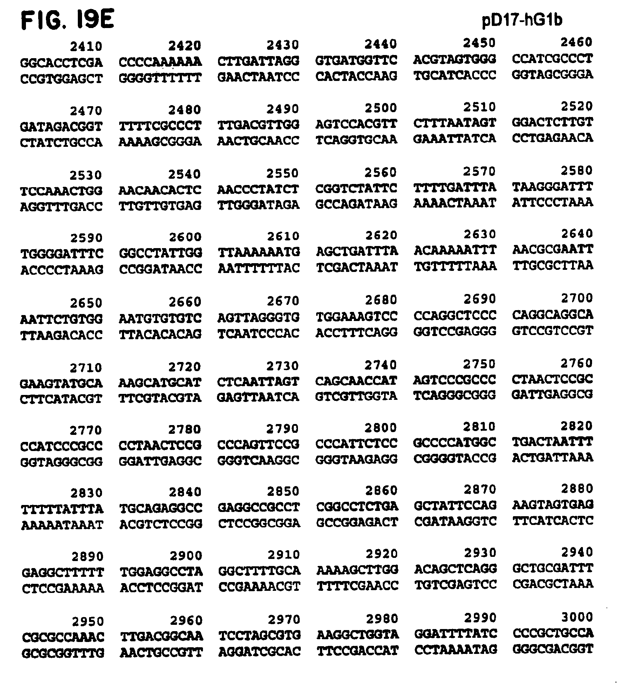

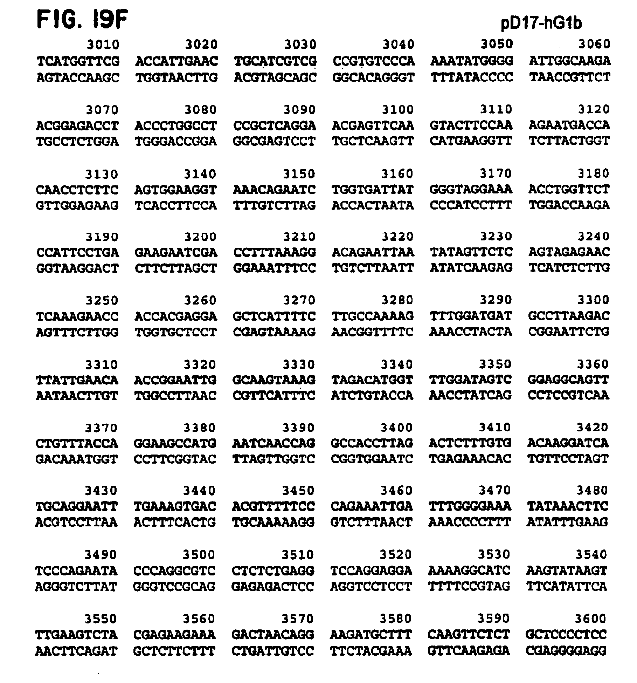

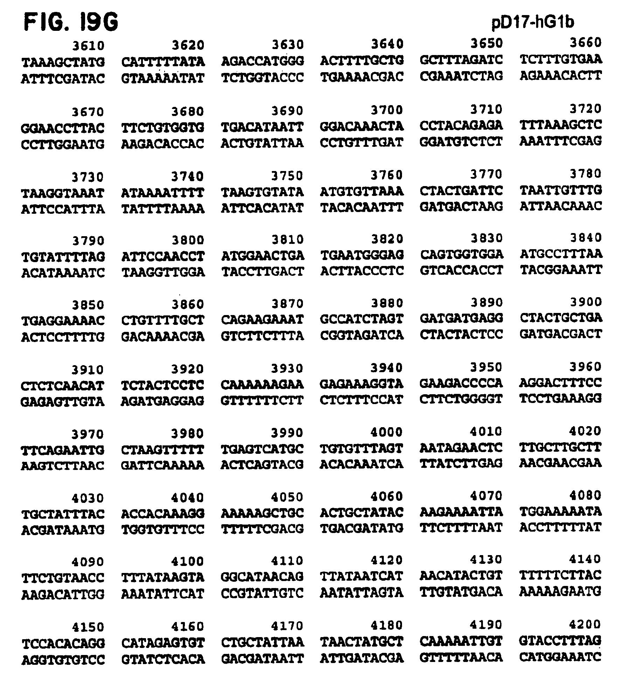

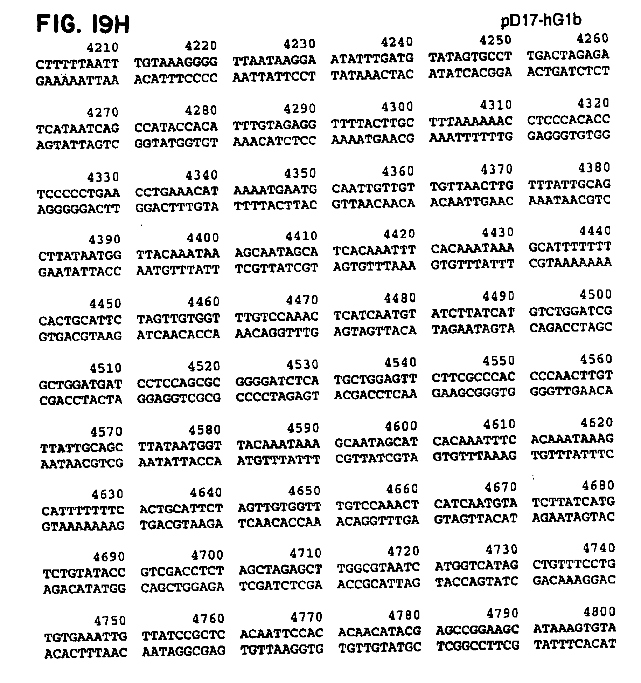

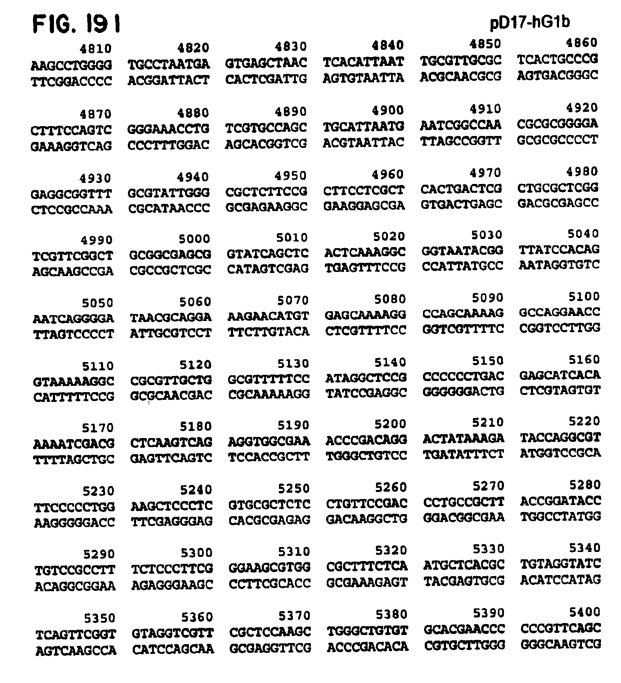

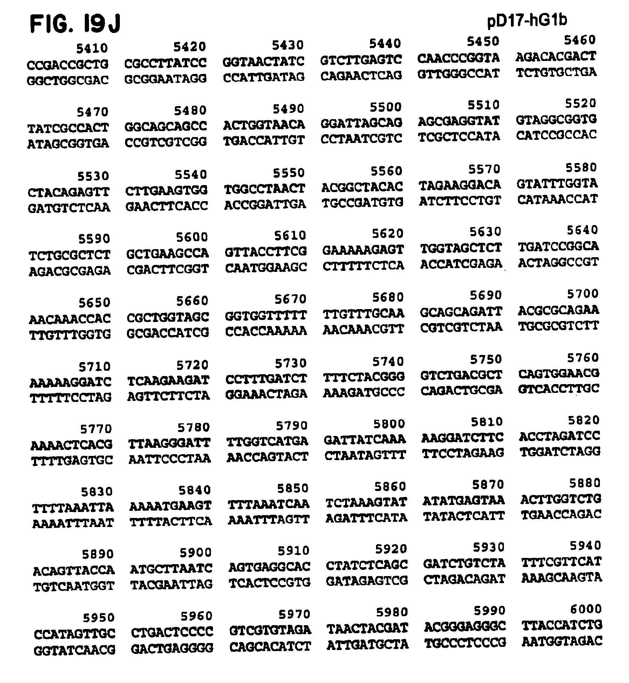

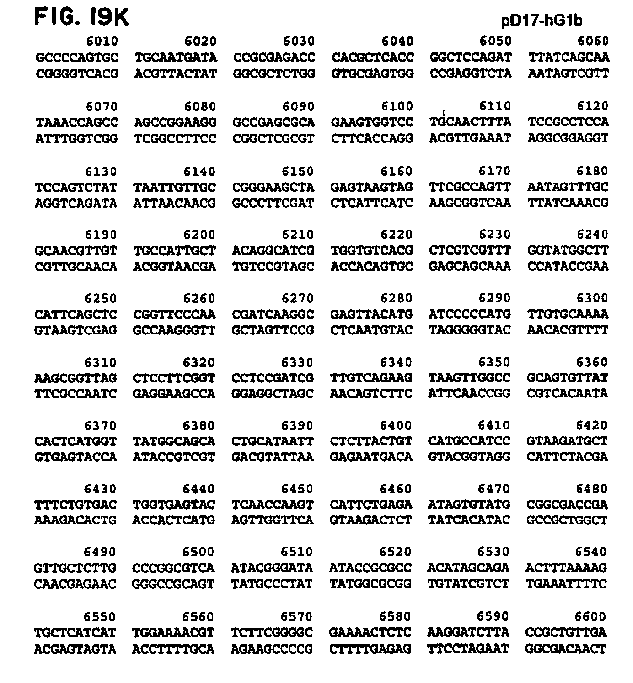

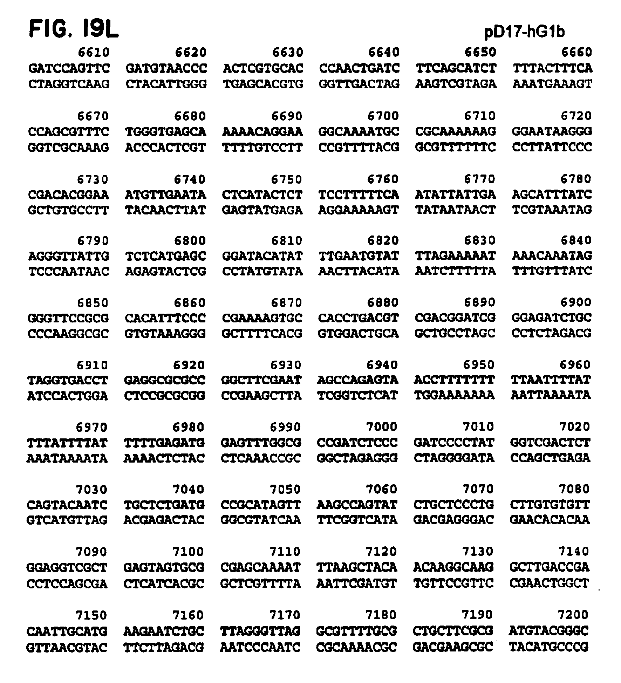

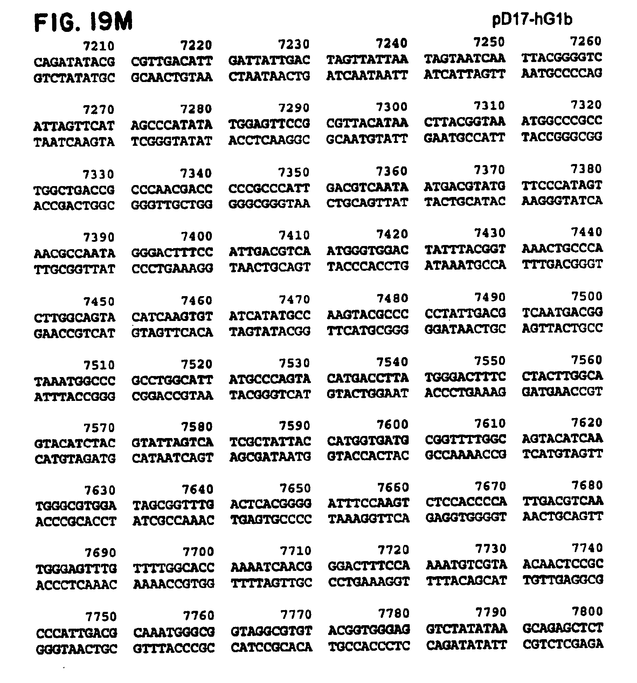

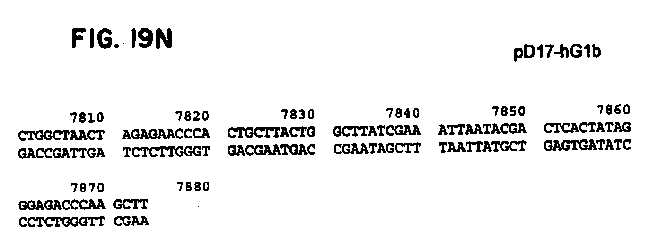

Figures 19A-N are the nucleic acid sequence of pD17-hG1b.
Figure 20 is a line graph showing complement dependent cytotoxicity. In the legend, the closed square is hBR96-1; closed diamond is hBR96-2B; closed circle is hBR96-2C; closed triangle is hBR96-2D; open square is hBR96-2H; open circle is hBR96-2A and open triangle is 2B8, *anti-Pseudonomas aeruginosa* flagella type b mAb, negative control.
Figure 21 is a line graph showing antibody dependent cell-mediated cytotoxity. In the legend, the closed square is hBR96-1; closed diamond is hBR96-2B; closed circle is hBR96-2C; closed triangle is hBR96-2D; open square is hBR96-2H; open circle is hBR96-2A and open triangle is 2B8, *anti-Pseudonomas aeruginosa* flagella type b monoclonal antibody (mAb), negative control.

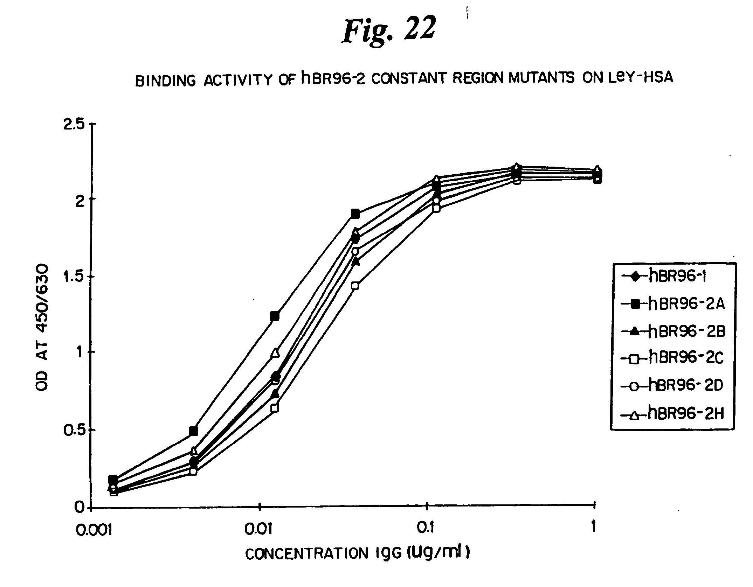

Figure 22 is a line graph showing binding activity of hBR96-2 constant region mutants on LeY-HSA. In the legend, the solid diamond is hBR96-1; solid square is hBR96-2A (CH2 deletion); solid triangle is hBR96-2B (235, 237 mutations); open square is hBR96-2C (318, 320, 322 mutations); open circle is hBR96-2D (331 mutation); and open triangle is hBR96-2H (235, 237, 318, 320, 322, 331 mutations).

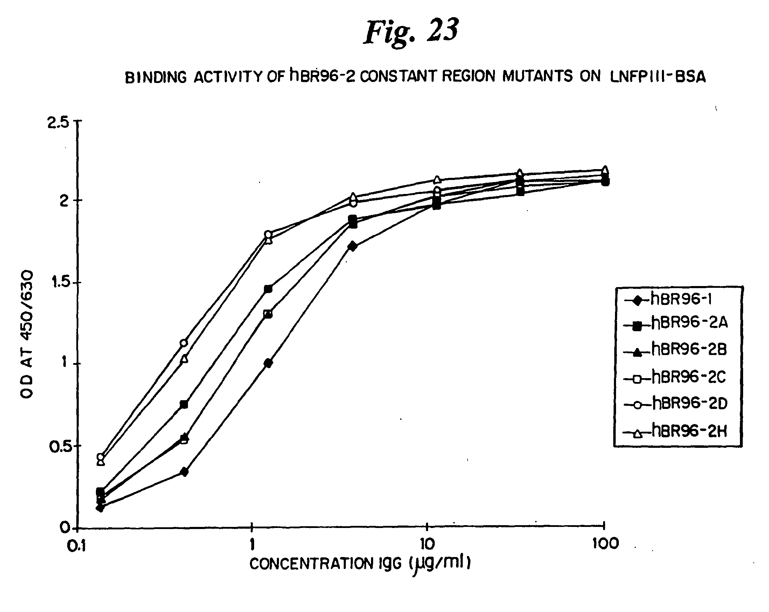

Figure 23 is a line graph showing binding activity of hBR96-2 constant region mutants on LNFPIII-BSA. LNFPIII is a lacto-N-fucopentasose, a Lewis X trisaccharide with an additional lactose spacer (V Labs, Covington, LA). In the legend, the solid diamond is hBR96-1; solid square is hBR96-2A (CH2 deletion); solid triangle is hBR96-2B (235, 237 mutations); open square is hBR96-2C (318, 320, 322 mutations); open circle is hBR96-2D (331 mutation); and open triangle is hBR96-2H (235, 237, 318, 320, 322, 331 mutations).

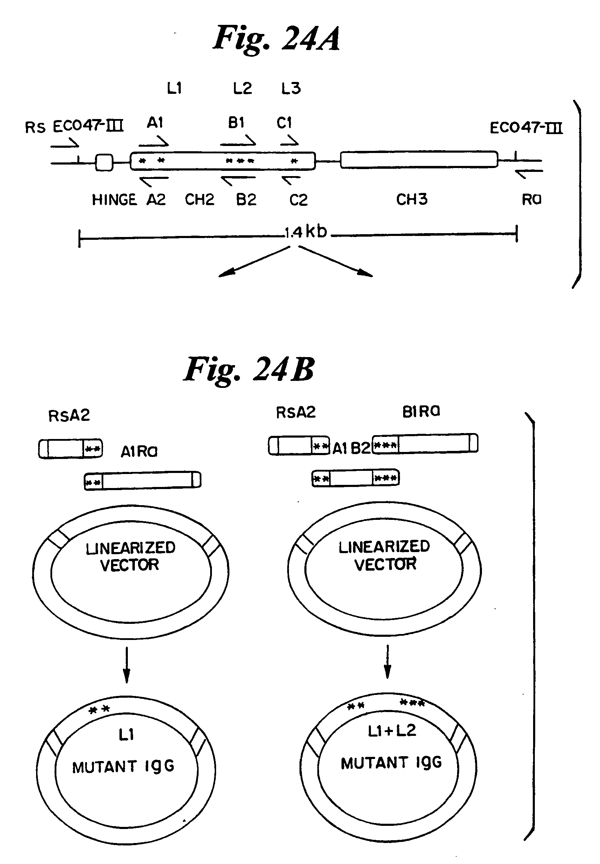

Figures 24A and 24B provide a strategy for introducing multiple mutations by RPCR. (A) Diagram of he 1.4 kpb IgG heavy chain region showing the hinge CH₂ and CH₃ domains as boxed regions. Site-specific mutations to be introduced into CH₂ positions L1, L2, and L3 are encoded by complementary sets of mutant PCR primers (A1 and A2; B1 and B2; and Cl and C2). The asterisks (*) indicate the number of amino acid changes introduced at each L position. The two PCR primers, Rs (Recombination -sense) and Ra (Recombination-antisense), flank the Eco-47-III restriction sites and mediate homologous recombination with vector ends. The 3' ends of the oligonucleotides are represented by arrowheads. (B) A three-way homologous recombination event between fragments RsA2, AIRa and the linearized vector produces the L mutant IgG. Two distally located sets of mutations (L and L2) are simultaneously introduced by increasing the number of recombining PCR produces as is shown in the four-way recombination of RsA2, A1B2, B1Ra with vector.
Figure 25 is a gel showing Eco-47-III restriction endonuclease analysis of DNAs prepared from colonies generated by multiple PCR fragment RPCR. Lane M: 1kb ladder DNA marker (GIBCO/BRL Life Science Technology). Lanes 1-12: Twelve randomly selected colonies resulting from quadruple homologous recombination events were used to prepare plasmid and digested with Eco47-III. Clones 1, 2, 6 and 9 contain the fully assembled 1.4 kpb insert.
Figure 26 provides the amino acid sequence for hBR96-2 heavy-chain variable region and the human IgG1 constant region.
Figure 27 provides the amino acid sequence for hBR96-2A heavy-chain variable region and the human IgG1 constant region without the CH₂ domain.
Figure 28 provides the amino acid sequence for chi BR96 heavy-chain variable region and the human IgG1 constant region without the CH₂ domain.

### DETAILED DESCRIPTION OF THE INVENTION

### DEFINITIONS

As used herein the term "inhibiting immunoglobulin-induced toxicity" means to reduce or alleviate symptoms generally associated with toxicity caused by immunoglobulin or Ig fusion protein therapy, e.g., toxicity mediated by effector functions of the Fc receptor. For example, BR96 antibody recognizes and binds BR96 antigen which is found at some levels in the gastrointestinal tract and at elevated levels in tumors (as compared to the gastrointestinal tract of normal tissues). The binding of BR96 antibody to BR96 antigen in vivo causes symptoms associated with gastrointestinal toxicity. These symptoms include rapid onset of vomiting, often with blood, and nausea. In humans the bleeding is limited to the fundus of the stomach, causing erosion of the superficial mucosa of the stomach.

The pathology of the wound is limited and resolves. However, the extreme nature of the nausea and vomiting, unrelieved by anti-emetics, defines it as the dose-limiting toxicity. For highly elevated levels of other antigens found in the central nervous system (CNS), liver, and other locations, the toxicity will be characterized by symptoms other than those described above.

As used herein the term "immunoglobulin molecule" can be produced by B cells or be generated through recombinant engineering or chemical synthetic means. Examples of immunoglobulin molecules include (1) antibodies, e.g., polyclonal and monoclonal antibodies, chimeric or humanized, and (2) recombinant Ig containing binding proteins, e.g., Ig fusion proteins. Recombinant Ig containing binding proteins include cell surface proteins, e.g., CD antigens (in one embodiment, CTLA4), to which an Ig tail is joined.

As used herein the terms "structurally altered" or "structural alteration" means manipulating the constant region so that the resulting molecule or protein exhibits a diminished ability to induce toxicity. Structural alteration can be by chemical modification, proteolytic alteration, or by recombinant genetic means. Recombinant genetic means may include, but is not limited to, the deletion, insertion and substitution of amino acid moieties.

As used herein the terms "multiple toxicity associated domains" means more than one discrete toxicity associated domain. As there appear to be at least two toxicity associated domains in the immunoglobulin molecule, one roughly localized to amino acids 231-238 and another roughly localized to amino acids 310-331, an example of the structural alteration of multiple toxicity associated domains comprises the insertion, substitution or deletion of amino acid residues in both of these domains. This definition excludes structural alterations targeting a single toxicity associated domain.

Merely by way of example, the constant region of the immunoglobulin molecule can be structurally altered so that the molecule no longer mediates a CDC or ADCC response. However, the methods of the invention encompasses the use of structurally altered immunoglobulin molecules regardless of whether it mediates a CDC or ADCC response. The underlying requirement is that the altered molecule must inhibit immunoglobulin-induced toxicity.

The present invention first provides a BR96 antibody having a human IgG1 constant region which has been structurally altered in the CH2 domain wherein leucine at amino acid position 235 is mutated to alanine; glycine at amino acid position 237 is mutated to alanine; glutamic acid at amino acid position 318 is mutated to serine; lysine at amino acid position 320 is mutated to serine; and lysine at amino acid position 322 is mutated to serine.

The present invention also provides a BR96 antibody having a human IgGI constant region which has been structurally altered in the CH2 domain wherein leucine at amino acid position 235 is mutated to alanine; glycine at amino acid position 237 is mutated to alanine; and proline at amino acid position 331 is mutated to alanine.

In addition, the present invention provides a BR96 antibody having a human IgGI constant region which has been structurally altered in the CH2 domain wherein leucine at amino acid position 235 is mutated to alanine; glycine at amino acid position 237 is mutated to alanine; glutamic acid at amino acid position 318 is mutated to serine; lysine at amino acid position 320 is mutated to serine; lysine at amino acid position 322 is mutated to serine; and proline at amino acid position 331 is mutated to alanine.

Other aspects of the invention are claimed in appended claims 4-18. Further embodiments not encompassed by the claimed invention are also disclosed in the present specification.

Structural alteration can be effected in a number of ways. For example, structural alteration can be effected by deletion of the entire constant region.

Alternatively, structural alteration can be effected by deletion of the entire CH₂ domain of the constant region. In this instance, deletion of the entire CH₂ domain may render the molecule unable to (1) bind an Fc receptor thereby eliminating the molecule's possibility of mediating antibody-dependent cellular cytotoxicity (ADCC), (2) bind C1q, or (3) activate complement.

Alternatively, structural alteration can be effected by deletion of only that portion of the CH₂ domain that binds the Fc receptor or complement.

Further alternatively, a single mutation or multiple mutations such as substitutions and insertions in the CH₂ domain can be made. The underlying requirement of any mutation is that it must inhibit, diminish, or block immunoglobulin-induced toxicity. For example, this can be achieved by mutating the constant region such that the altered molecule is rendered unable to mediate a CDC response or an ADCC response, or to activate complement.

Alternatively, structural alteration can be effected by isotype switching (also known as class switching) so that the altered molecule does not induce toxicity in the subject. In one embodiment, the constant region of the immunoglobulin is structurally altered so that it no longer binds the Fc receptor or a complement component, e.g., switching a molecule's original IgG isotype from IgG1 to IgG4. Isotype switching can be effected regardless of species, i.e., an isotype from a non-human being can be switched with an isotype from a human being (E.D. Finkelman et al. (1990) Annu. Rev. Immunol. 8:303-333; T. Honjo et al. (1979) Cell 18: 559-568; T. Honjo et al. In "Immunoglobulin Genes" pp. 124-149 Academic Press, London)).

As used herein the term "Ig fusion protein" means any recombinantly produced antigen or ligand binding domain having a constant region which can be structurally altered.

As used herein "cytotoxic agent" includes antimetabolites, alkylating agents, anthracyclines, antibiotics, anti-mitotic agents, and chemotherapeutic agents. Specific examples within these groups include but are not limited to ricin, doxorubicin, daunorubicin, taxol, ethidium bromide, mitomycin, etoposide, tenoposide, vincristine, vinblastine, colchicine, supporin, gelonin, PE40, bryodin, dihydroxy anthracin dione, actinomycin D, and 1-dehydrotestosterone.

As used herein the term "BR96" refers to (1) the whole BR96 monoclonal antibody disclosed in PCT No. 95/305444, published March 6, 1996, (2) chimeric BR96 monoclonal antibody disclosed in PCT No. 95/305444, published March 6, 1996, or (3) BR96 mutant molecules disclosed in PCT No. 95/305444, published March 6, 1996.

As used herein, "treating" means to (1) provide tumor regression so that the tumor is not palpable for a period of time (standard tumor measurement procedures may be followed (A.B. Miller et al. "Reporting results of cancer treatment" Cancer 47:207-214 (1981)); (2) stabilize the disease; or (3) provide any clinically beneficial effects.

As used herein, an "effective amount" is an amount of the antibody, immunoconjugate, or recombinant molecule which kills cells or inhibits the proliferation thereof.

As used herein, "administering" means oral administration, administration as a suppository, topical contact, intravenous, intraperitoneal, intramuscular or subcutaneous administration, or the implantation of a slow-release device such as a miniosmotic pump, to the subject.

As used herein, "pharmaceutically acceptable carrier" includes any material which when combined with the antibody retains the antibody's specificity or efficacy and is non-reactive with the subject's immune system. Examples include, but are not limited to, any of the standard pharmaceutical carriers such as a phosphate buffered saline solution, water, emulsions such as oil/water emulsion, and various types of wetting agents. Other carriers may also include sterile solutions, tablets including coated tablets and capsules.

Typically such carriers contain excipients such as starch, milk, sugar, certain types of clay, gelatin, stearic acid or salts thereof, magnesium or calcium stearate, talc, vegetable fats or oils, gums, glycols, or other known excipients. Such carriers may also include flavor and color additives or other ingredients. Compositions comprising such carriers are formulated by well known conventional methods.

As used herein, "mutation" means a single amino acid or nucleic acid mutation or multiple mutations by whatever means, e.g., homologous recombination, error prone PCR, or site directed mutagenesis.

In order that the invention herein described may be more fully understood, the following description is set forth.

### METHODS OF THE PRESENT INVENTION

Disclosed herein is a method for inhibiting immunoglobulin-induced toxicity resulting from the use of immunoglobulin during therapy or in vivo diagnosis. For example, the methods of the invention would be useful to minimize the toxicity associated with prolonged clinical exposure to immunoglobulin use during or after tumor imaging with radiolabeled antibodies.

In accordance with the practice of this invention, the subject includes, but is not limited to, human, equine, porcine, bovine, murine, canine, feline, and avian subjects. Other warm blooded animals are also included in this invention.

This method comprises administering an immunoglobulin molecule to the subject. The immunoglobulin can be IgG, IgM, or IgA. IgG is preferred.

In one embodiment of the invention, the immunoglobulin molecule recognizes and binds Le^{y}. In another embodiment, the immunoglobulin recognizes and binds Le^{x}. In a further embodiment, the immunoglobulin is a monoclonal antibody BR96 produced by the hybridoma deposited on February 22,1989 with the American Type Culture Collection (ATCC), 12301 Parklawn Drive, Rockville, MD 20852 and accorded ATCC Accession No.: HB 10036. In yet another embodiment, the immunoglobulin is a chimeric antibody ChiBR96 produced by the hybridoma deposited on May 23, 1990, with the ATCC, 12301 Parklawn Drive, Rockville, MD 20852 and accorded ATCC Accession No.: HB 10460.

In accordance with the practice of the invention, the immunoglobulin can be a bispecific antibody with a binding specificity for two different antigens, one of the antigens being that with which the monoclonal antibody BR96 produced by the hybridoma having the identifying characteristics of HB 10036 as deposited with the ATCC binds. Also, in accordance with the practice of the invention, the immunoglobulin can be an anti-idiotypic antibody.

As required by the invention, at least a portion of the constant region of the immunoglobulin molecule is structurally altered. Structural alteration can be effected by a number of means. In one embodiment, the entire constant region, i.e., CH₁, CH₂, and CH₃ domains, can be deleted.

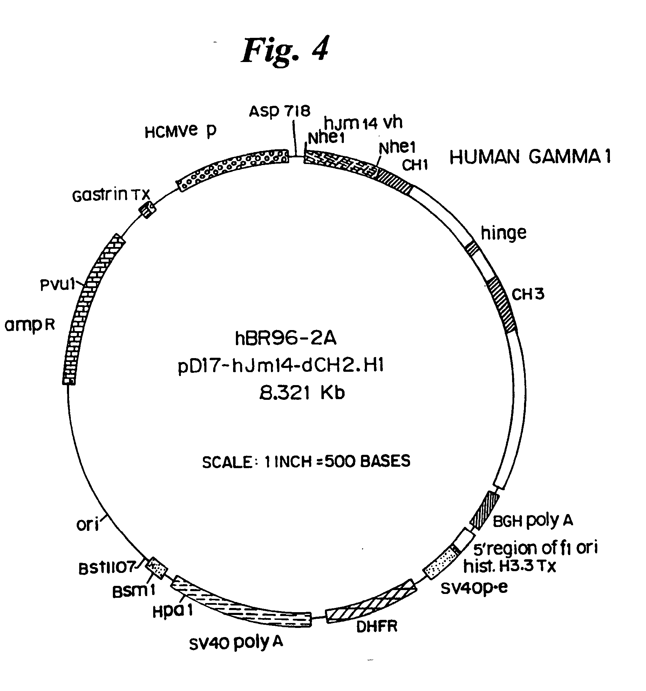

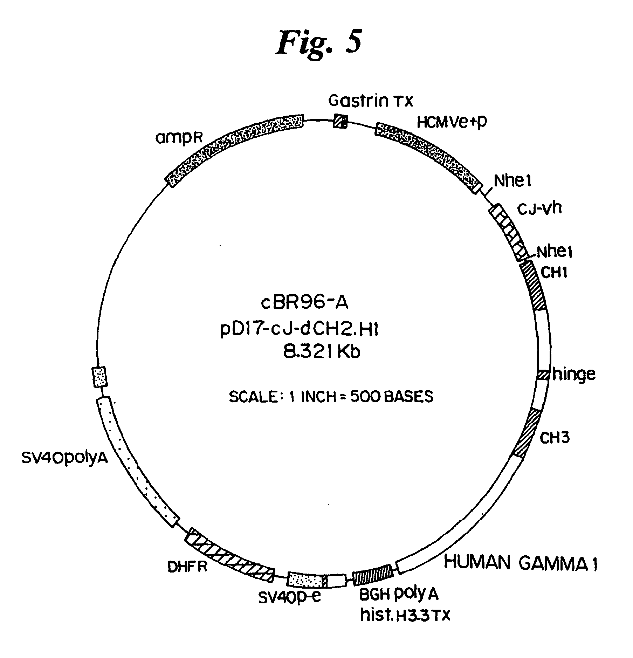

In another embodiment, only the CH₂ domain is deleted from the immunoglobulin molecule (e.g., cBR96-A (Figure 5), hBR96-2A (Figure 4). In this embodiment, the CH₂ deletion may result in a molecule unable to bind the Fc receptor or a complement component.

In another embodiment, only that portion of the CH₂ domain which binds the complement component C1q is deleted. In yet another embodiment, mutations in specific portions of the CH₂ domain are made. For example, the immunoglobulin molecule may be modified by structurally altering multiple toxicity associated domains in the constant region so that immunoglobulin-induced toxicity is inhibited. A discussion of such mutations are further found hereinafter.

Regardless of the means, the underlying requirement for any structural alteration of the constant region is that immunoglobulin-induced toxicity is substantially reduced or inhibited. In one embodiment, immunoglobulin-induced toxicity is inhibited by structurally altering the constant region such that the molecule's ability to mediate a CDC response or ADCC response and/or activate the complement cascade is prevented or inhibited. Methods for determining whether the molecule is able to inhibit a CDC response are well known, e.g., one method involves a ⁵¹Cr-release test (H. Garrigues et al. Int. J. Cancer 29:511 (1982); I. Hellström et al. PNAS 82:1499 (1985)). Methods for determining whether the molecule is able to inhibit an ADCC response are well known (I. Hellström et al. PNAS 82:1499 (1985)). Methods for determining whether the molecule is able to activate a complement cascade are well known.

Disclosed herein is a method comprising administering to the subject an Ig fusion protein having a structurally altered constant region. Structural alteration of the constant region may include deletion of the entire C region or portions thereof, e.g., alteration of the CH₂ domain so that the altered molecule no longer binds the Fc receptor or a complement component.

Disclosed herein is a method for inhibiting immunoglobulin-induced toxicity resulting from immunotherapy in a subject. The method comprises administering to the subject an antibody which has been modified so that at least a portion of the constant region has been structurally altered as discussed supra. In one embodiment, the antibody recognizes and binds Le^{y}. In another embodiment, the antibody recognizes and binds to Le^{x}.

The antibody can be monoclonal antibody BR96 produced by the hybridoma having the identifying characteristics of HB 10036 as deposited with the ATCC. Alternatively, the antibody can be chimeric antibody ChiBR96 produced by the hybridoma having the identifying characteristics of HB 10460 as deposited with the ATCC. Further, the antibody can be a bispecific antibody with a binding specificity for two different antigens, one of the antigens being that with which the monoclonal antibody BR96 produced by the hybridoma having the identifying characteristics of HB 10036 as deposited with the ATCC binds.

Additionally, disclosed herein is a method for inhibiting immunoglobulin-induced toxicity resulting from immunotherapy for a disease in a subject. The disease will vary with the antigen sought to be bound. Examples of diseases include but are not limited to immunological diseases, cancer, cardiovascular diseases, neurological diseases, dermatological diseases or kidney disease.

This method comprises the following steps. Step one provides selecting an antibody for a target. Generally, the target is associated with the disease and the antibody directed to the target is known. For example, the target can be the BR96 antigen and the antibody selected is BR96.

Step two of this method provides structurally altering the constant region of the antibody so selected so that immunoglobulin induced toxicity is inhibited. Inactivation can include any of the means discussed above. For example, inactivation can be effected by structurally altering multiple toxicity associated domains in the CH₂ domain of the constant region of the Ig protein so selected.

Step three of this method provides administering the structurally altered antibody of step two to the subject under conditions that the structurally altered antibody recognizes and binds the target and that such binding directly or indirectly alleviates symptoms associated with the disease.

In one embodiment step one provides selecting an Ig fusion protein for a target. Further, the method provides mutating the Ig fusion protein so selected by structurally altering the CH₂ domain of the constant region of the Ig protein by the same means discussed above.

Disclsoed herein are methods to treat human carcinoma. For example, the immunoglobulin, antibody, or Ig fusion protein discussed above can be used in combination with standard or conventional treatment methods such as chemotherapy, radiation therapy or can be conjugated or linked to a therapeutic drug, or toxin, as well as to a lymphokine or a tumor-inhibitory growth factor, for delivery of the therapeutic agent to the site of the carcinoma.

Techniques for conjugating therapeutic agents to immunoglobulins are well known (see, e.g., Arnon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies '84: -Biological And Clinical Applications, Pinchera et al. (eds.), pp. 475-506 (1985); and Thorpe et al., "The Preparation And Cytotoxic Properties Of Antibody-Toxin Conjugates", Immunol. Rev., 62:119-58 (1982)).

Alternatively, the structurally altered antibody or Ig fusion protein can be coupled to high-energy radiative agents, e.g., a radioisotope such as ¹³¹I; which, when localized at the tumor site, results in a killing of several cell diameters (see, e.g., Order, "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985)). According to yet another embodiment, the structurally altered BR96 antibody can be conjugated to a second antibody to form an antibody heteroconjugate for the treatment of tumor cells as described by Segal in United States Patent 4,676,980.

Still other therapeutic applications for the structurally altered antibody or Ig fusion protein of the invention include conjugation or linkage, e.g., by recombinant DNA techniques or protein chemical techniques, to an enzyme capable of converting a prodrug into a cytotoxic drug and the use of that antibody-enzyme conjugate in combination with the prodrug to convert the prodrug to a cytotoxic agent at the tumor site (see, e.g., Senter et al., "Anti-Tumor Effects Of Antibody-alkaline Phosphatase", Proc. Natl. Acad. Sci. USA, 85:4842-46 (1988); "Enhancement of the in vitro and in vivo Antitumor Activities of Phosphorylated Mitomycin C and Etoposide Derivatives by Monoclonal Antibody-Alkaline Phosphatase Conjugates", Cancer Research 49:5789-5792 (1989); and Senter, "Activation of Prodrugs by Antibody-Enzyme Conjugates: A New Approach to Cancer Therapy," FASEB J. 4:188-193 (1990)).

It is apparent therefore that the present invention encompasses pharmaceutical compositions including immunoglobulin molecules, antibodies, and Ig fusion proteins all having structurally altered CH₂ domains, and their use in methods for treating human carcinomas. For example, the invention includes pharmaceutical compositions for use in the treatment of human carcinomas comprising a pharmaceutically effective amount of a structurally altered BR96 and a pharmaceutically acceptable carrier.

The compositions may contain the structurally altered antibody or Ig fusion protein or antibody fragments, either unmodified, conjugated to a therapeutic agent (e.g., drug, toxin, enzyme or second antibody). The compositions may additionally include other antibodies or conjugates for treating carcinomas (e.g., an antibody cocktail).

The compositions of the invention can be administered using conventional modes of administration including, but not limited to, intrathecal, intravenous, intraperitoneal, oral, intralymphatic or administration directly into the tumor. Intravenous administration is preferred.

The composition of the invention can be in a variety of dosage forms which include, but are not limited to, liquid solutions or suspensions, tablets, pills, powders, suppositories, polymeric microcapsules or microvesicles, liposomes, and injectable or infusible solutions. The preferred form depends upon the mode of administration and the therapeutic application.

The compositions of the invention also preferably include conventional pharmaceutically acceptable carriers and adjuvants known in the art such as human serum albumin, ion exchangers, alumina, lecithin, buffer substances such as phosphates, glycine, sorbic acid, potassium sorbate, and salts or electrolytes such as protamine sulfate.

In accordance with the practice of the invention, the pharmaceutical carrier can be a lipid carrier. The lipid carrier can be a phospholipid. Further, the lipid carrier can be a fatty acid. Also, the lipid carrier can be a detergent. As used herein, a detergent is any substance that alters the surface tension of a liquid, generally lowering it.

In one example of the invention, the detergent can be a nonionic detergent. Examples of nonionic detergents include, but are not limited to, polysorbate 80 (also known as Tweec®80 or (polyoxyethylenesorbitan monooleate), Brij®, and Triton® (for example Triton®WR-1339 and Triton®A-20).

Alternatively, the detergent can be an ionic detergent. An example of an ionic detergent includes, but is not limited to, alkyltrimethylammonium bromide.

Additionally, in accordance with the invention, the lipid carrier can be a liposome. As used in this application, a "liposome" is any membrane bound vesicle which contains any molecules of the invention or combinations thereof.

The most effective mode of administration and dosage regimen for the compositions of this invention depends upon the severity and course of the disease, the patient's health and response to treatment and the judgment of the treating physician.

The interrelationship of dosages for animals of various sizes and species and humans based on mg/m² of surface area is described by Freireich, E.J., et al. Cancer Chemother., Rep. 50 (4): 219-244 (1966). Adjustments in the dosage regimen can be made to optimize the tumor cell growth inhibiting and killing response, e.g., doses can be divided and administered on a daily basis or the dose reduced proportionally depending upon the situation (e.g., several divided doses can be administered daily or proportionally reduced depending on the specific therapeutic situation).

### THE MOLECULES OF THE INVENTION

The present invention first provides a BR96 antibody having a human IgG1 constant region which has been structurally altered in the CH2 domain wherein leucine at amino acid position 235 is mutated to alanine; glycine at amino acid position 237 is mutated to alanine; glutamic acid at amino acid position 318 is mutated to serine; lysine at amino acid position 320 is mutated to serine; and lysine at amino acid position 322 is mutated to serine.

The present invention also provides a BR96 antibody having a human IgG1 constant region which has been structurally altered in the CH2 domain wherein leucine at amino acid position 235 is mutated to alanine; glycine at amino acid position 237 is mutated to alanine; and proline at amino acid position 331 is mutated to alanine.

In addition, the present invention provides a BR96 antibody having a human IgG constant region which has been structurally altered in the CH2 domain wherein leucine at amino acid position 235 is mutated to alanine; glycine at amino acid position 237 is mutated to alanine; glutamic acid at amino acid position 318 is mutated to serine; lysine at amino acid position 320 is mutated to serine; lysine at amino acid position 322 is mutated to serine; and proline at amino acid position 331 is mutated to alanine.

Other aspects of the invention are claimed in appended claims 4-18. Further embodiments not encompassed by the claimed invention are also disclosed in the present specification.

Disclosed herein are structurally altered BR96 or BR96 Ig fusion proteins. Structurally altered BR96 antibodies or Ig fusion proteins have the variable region of BR96 and a modified constant region. This modification provides structurally altered BR96 antibodies or Ig fusion proteins with the ability to inhibit immunoglobulin-induced toxicity.

Various embodiments of structurally altered BR96 or BR96 Ig fusion proteins have been made.

In one embodiment, designated cBR96-A, the entire CH₂ domain of cBR96 was deleted. CBR96-A is expressed by the plasmid having the sequence shown in SEQ. ID. NO. 10. cBR96 is expressed by a plasmid having the sequence in SEQ ID NO. 9.

In another embodiment, designated hBR96-2A, the entire CH₂ domain of hBR96 was deleted. hBR96-A is expressed by the plasmid having the sequence shown in SEQ. ID. NO. 12. hBR96 is a mutant BR96 having the H1, H2, and H3 mutations described in PCT Application No. 95/305444, published March 6, 1996.

In yet another embodiment, designated hBR96-2B, the leucine residue located at amino acid position 235 is mutated to alanine. Additionally, the glycine residue located at amino acid position 237 is mutated to alanine. The amino acid position numbering used is described in Kabat et al. Sequences of Proteins of Immunological Interest 5th Edition (1991) United States Department of Health and Human Services.

In a further embodiment, designated hBR96-2C, the glutamic acid residue at position 318 is mutated to serine; the lysine residue located at position 320 is mutated to serine; and the lysine residue located at position 322 is mutated to serine using standard protocols (Alexander R. Duncan and Greg Winter "The binding site for C1q on IgG" Nature 332:738 (1988)).

In another embodiment, designated hBR96-2D, the proline residue at position 331 is mutated to alanine (M-H. Tao et al., "Structural features of human immunoglobulin G that determine isotype-specific differences in complement activation" J. Exp. Med. 178:661-667 (1993); Y. Xu et al., "Residue at position 331 in the IgG1 and IgG4 domains contributes to their differential ability to bind and activate complement" J. Biol. Chem. 269:3469-3474 (1994)).

In an additional embodiment, designated hBR96-2E, the leucine residue at position 235 is mutated to alanine; the glycine residue located at position 237 is mutated to alanine; the glutamic acid residue located at position 318 is mutated to serine; the lysine residue located at position 320 is mutated to serine; and the lysine residue located at position 322 is mutated to serine (A. Morgan et al., "The N-terminal end of the CH2 domain of chimeric human IgG1 anti-HLA-DR is necessary for C1q, Fc(gamma)RI and Fc(gamma)RIII binding" Immunol. 86:319-324 (1995)).

In yet a further embodiment, designated hBR96-2F, the leucine residue located at position 235 is mutated to alanine; the glycine residue located at position 237 is mutated to alanine; and the proline residue located at position 331 is mutated to alanine.

In yet another embodiment, designated hBR96-2G, the glutamic acid residue located at position 318 is mutated to serine; the lysine residue located at position 320 is mutated to serine; the lysine residue located at position 322 is mutated to serine; and the proline residue located at position 331 is mutated to alanine.

In another embodiment, designated hBR96-2H, the leucine residue located at position 235 is mutated to alanine; the glycine residue located at position 237 is mutated to alanine; the glutamic acid residue at position 318 is mutated to serine; the lysine residue located at position 320 is mutated to serine; the lysine residue located at position 322 is mutated to serine; and the proline residue located at position 331 is mutated to alanine.

Depending on its form, a structurally altered BR96 antibody or fusion protein can be a monofunctional antibody, such as a monoclonal antibody, or bifunctional antibody, such as a bispecific antibody or a heteroantibody. The uses of structurally altered BR96, i.e., as a therapeutic or diagnostic agent, will determine the different forms of structurally altered BR96 which is made.

Several options exists for antibody expression. Immunoexpression libraries can be combined with transfectoma technology, i.e., the genes for the Fab molecules derived from the immunoglobulin gene expression library can be connected to the desired constant-domain exons. These recombinant genes can then be transfected and expressed in a transfectoma that would secrete an antibody molecule.

Once produced, the polypeptides of the invention can be modified, i.e., by amino acid modifications within the molecule, so as to produce derivative molecules. Such derivative molecules would retain the functional property of the polypeptide, namely, the molecule having such substitutions will still permit the binding of the polypeptide to the BR96 antigen or portions thereof.

It is a well-established principle of protein chemistry that certain amino acid substitutions, entitled "conservative amino acid substitutions," can frequently be made in a protein without altering either the conformation or the function of the protein.

Amino acid substitutions include, but are not necessarily limited to, amino acid substitutions known in the art as "conservative".

Such changes include substituting any of isoleucine (I), valine (V), and leucine (L) for any other of these hydrophobic amino acids; aspartic acid (D) for glutamic acid (E) and vice versa; glutamine (Q) for asparagine (N) and vice versa; and serine (S) for threonine (T) and vice versa.

Other substitutions can also be considered conservative, depending on the environment of the particular amino acid and its role in the three-dimensional structure of the protein. For example, glycine (G) and alanine (A) can frequently be interchangeable, as can alanine and valine (V).

Methionine (M), which is relatively hydrophobic, can frequently be interchanged with leucine and isoleucine, and sometimes with valine. Lysine (K) and arginine (R) are frequently interchangeable in locations in which the significant feature of the amino acid residue is its charge and the differing pK's of these two amino acid residues are not significant. Still other changes can be considered "conservative" in particular environments.

In one embodiment of the present invention, the polypeptide is substantially pure, i.e., free of other amino acid residues which would inhibit or diminish binding of the polypeptide to its target and would inhibit or reduce gastrointestinal toxicity which are normally exhibited during or after antibody therapy.

### NUCLEIC ACID MOLECULES ENCODING THE PRESENT INVENTION

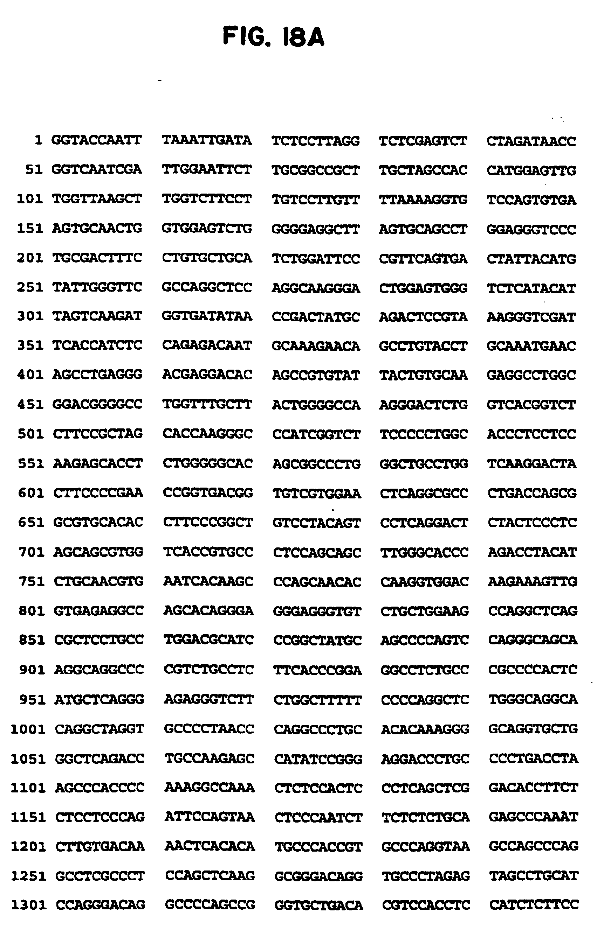

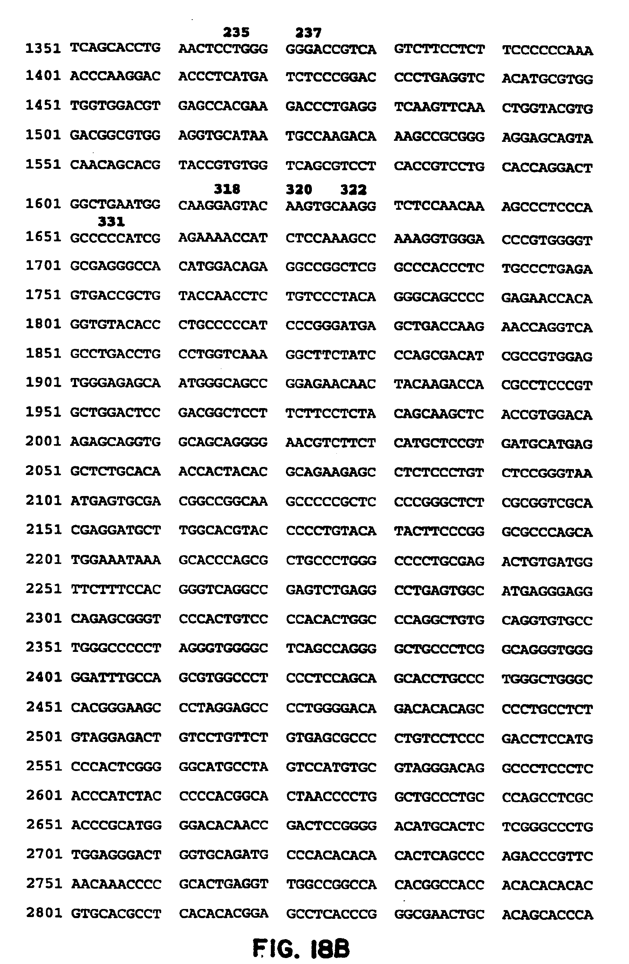

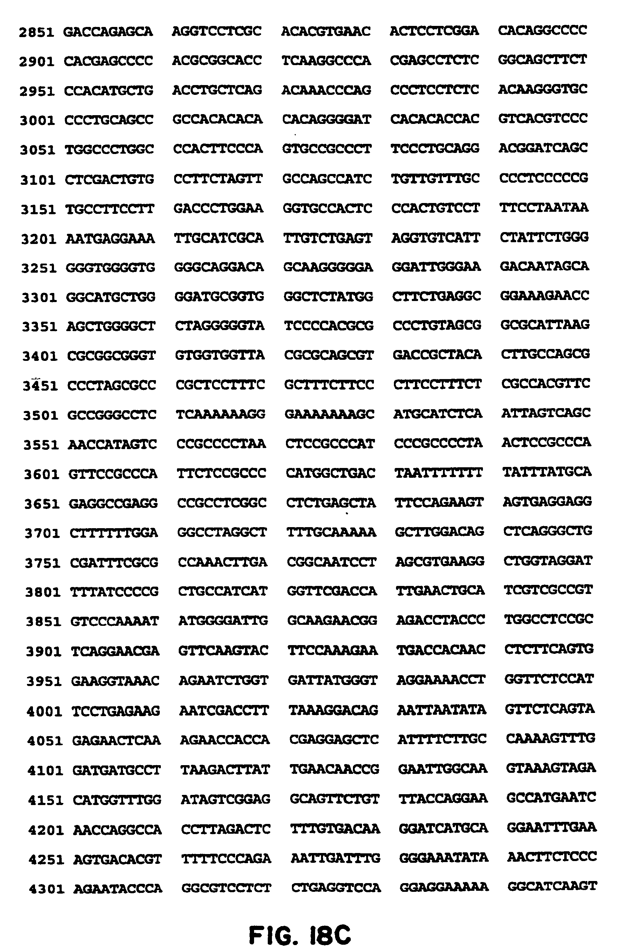

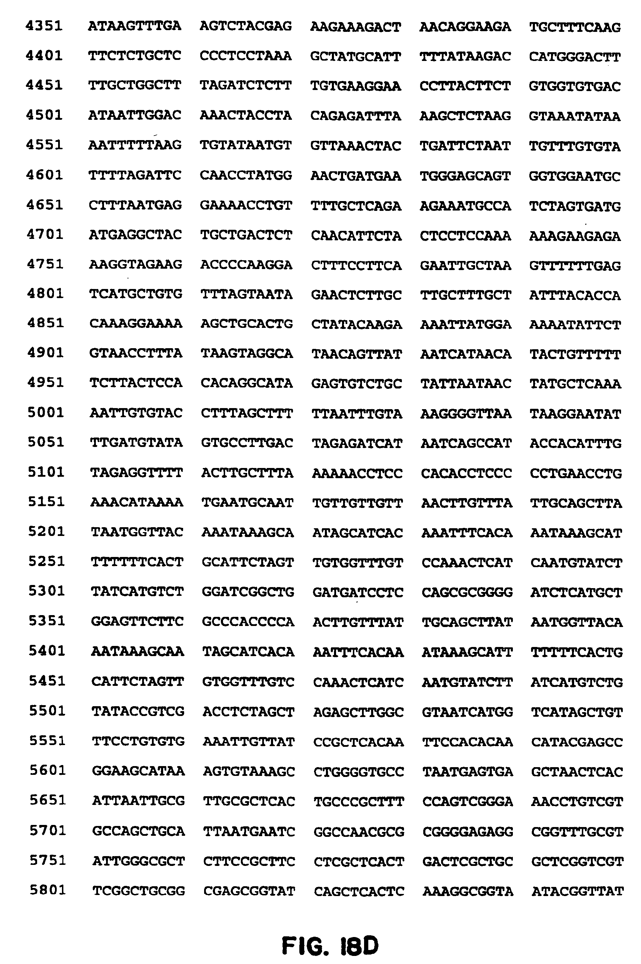

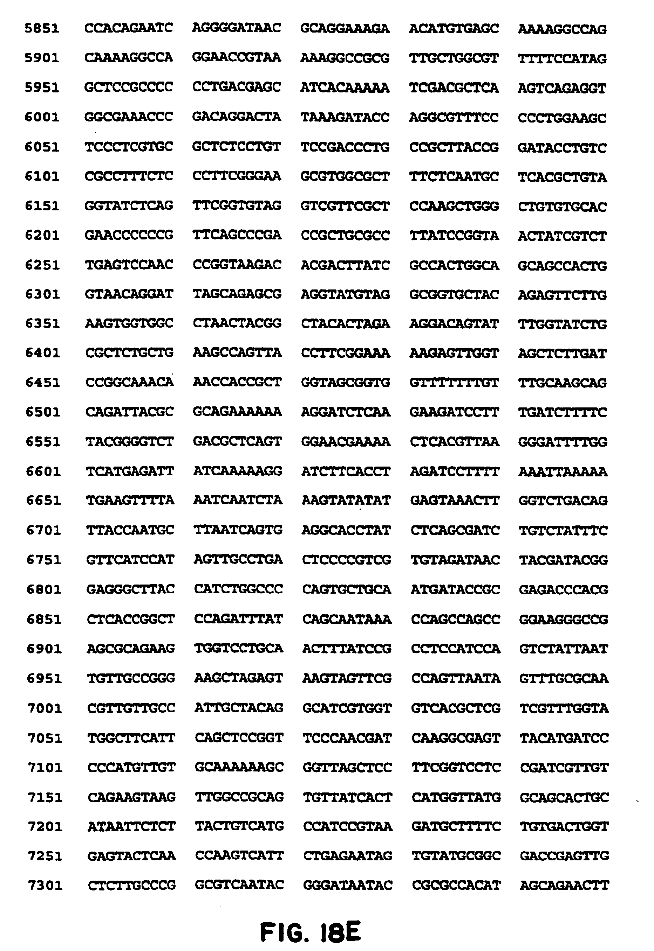

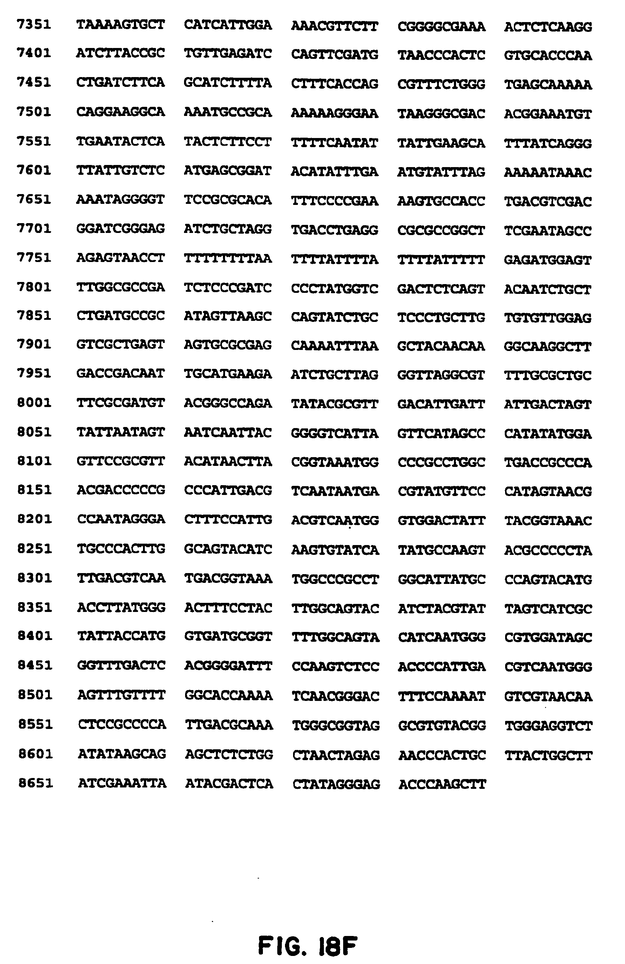

The nucleotide sequences and the amino acid sequences of the variable and constant regions of BR96 are known. The sequence for the immunoglobulin constant region is known and provided in Figure 18. Specific mutations in the constant region of the BR96 antibody were made. Nucleic acid molecules encoding the seven mutants described above (hBR96-2B through hBR96-2H) are as follows.

In hBR96-2B, alanine at amino acid positions 235 and 237 is encoded by codons GCU, GCC, GCA, or GCG.

In hBR96-2C, serine at positions 318, 320, and 322 is encoded by UCU, UCC, UCA, or UGG.

In hBR96-2D, alanine at position 331 is encoded by codons GCU, GCC, GCA, or GCG.

In hBR96-2E, alanine at positions 235 and 237 is encoded by codons GCU, GCC, GCA, or GCG. Serine at positions 318, 320, and 322 is encoded by UCU, UCC, UCA, or UGG.

In hBR96-2F, alanine at positions 235, 237, and 331 is encoded by codons GCU, GCC, GCA, or GCG.

In hBR96-2G, serine at positions 318, 320, 322 is encoded by UCU, UCC, UCA, or UGG. Further, the alanine at position 331 is encoded by codons GCU, GCC, GCA, or GCG.

In hBR96-2H, alanine at positions 235, 237, and 331 is encoded by codons GCU, GCC, GCA, or GCG. Additionally, serine at positions 318, 320, 322 is encoded by UCU, UCC, UCA, or UGG.

Any of the above can be deoxyribonucleic acid (DNA), e.g., complementary DNA (cDNA), or ribonucleic acid (RNA).

### IMMUNOCONJUGATES

Immunoconjugates (having whole antibody or Ig fusion proteins) may be constructed using a wide variety of chemotherapeutic agents such as folic acid and anthracyclines (Peterson et al., "Transport And Storage Of Anthracyclines In Experimental Systems And Human Leukemia", in Anthracycline Antibiotics In Cancer Therapy, Muggia et al. (Eds.), p. 132 (Martinus Nijhoff Publishers (1982); Smyth et al., "Specific Targeting of Chlorambucil to Tumors With the Use of Monoclonal Antibodies", J. Natl. Cancer Inst., 76:503-510 (1986)), including doxorubicin (DOX) (Yang and Reisfeld "Doxorubicin Conjugated with a Monoclonal Antibody Directed to a Human Melanoma-Associated Proteoglycan Suppresses Growth of Established Tumor xenografts in Nude Mice PNAS (USA)" 85:I189-1193 (1988)), Daunomycin (Arnon and Sela "In Vitro and in vivo Efficacy of Conjugates of Daunomycin With Anti-Tumor Antibodies" Immunol. Rev., 65:5-27 (1982)), and morpholinodoxorubicin (Mueller et al., "Antibody Conjugates With Morpholinodoxorubicin and Acid-Cleavable Linkers", Bioconjugate Chem., 1:325-330 (1990)).

BR96 has been conjugated to doxorubicin and has been shown to be effective in therapy of certain cancers or carcinomas (Trail, P.A., Willner, D., Lasch, S.J., Henderson, A.J., Casazza, A.M., Firestone, R.A., Hellström, I., and Hellström, K.E. Cure of xenografted human carcinomas by BR96-doxorubicin immunoconjugates. Science, 261:212-215, 1993).

In accordance with the practice of the invention, structurally altered BR96 can be used in forms including unreduced IgG, reduced structurally altered IgG, and fusion proteins (PCT Application No. 95/305444, published March 6, 1996).

Suitable therapeutic agents for use in making the immunoconjugate includes Pseudomonas exotoxin A (PE) in either the native PE or LysPE40 form. LysPE40 is a truncated form containing a genetically modified amino terminus that includes a lysine residue for conjugation purposes. Doxorubicin is also a suitable therapeutic agent.

Additional examples of therapeutic agents include, but are not limited to, antimetabolites, alkylating agents, anthracyclines, antibiotics, and anti-mitotic agents.

Antimetabolites include methotrexate, 6-mercaptopurine, 6-thioguanine, cytarabine, 5-fluorouracil decarbazine.

Alkylating agents include mechlorethamine, thiotepa chlorambucil, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin.

Anthracyclines include daunorubicin (formerly daunomycin) and doxorubicin (also referred to herein as adriamycin). Additional examples include mitozantrone and bisantrene.

Antibiotics include dactinomycin (formerly actinomycin), bleomycin, mithramycin, and anthramycin (AMC).

Antimitotic agents include vincristine and vinblastine (which are commonly referred to as vinca alkaloids).

Other cytotoxic agents include procarbazine, hydroxyurea, asparaginase, corticosteroids, mytotane (O,P'-(DDD)), interferons.

Further examples of cytotoxic agents include, but are not limited to, ricin, bryodin, gelonin, supporin, doxorubicin, taxol, cytochalasin B, gramicidin D, ethidium bromide, etoposide, tenoposide, colchicine, dihydroxy anthracin dione, 1-dehydrotestosterone, and glucocorticoid.

Clearly analogs and homologs of such therapeutic and cytotoxic agents are encompassed by the present invention. For example, the chemotherapuetic agent aminopterin has a correlative improved analog namely methotrexate.

Further, the improved analog of doxorubicin is an Fe-chelate. Also, the improved analog for 1-methylnitrosourea is lomustine. Further, the improved analog of vinblastine is vincristine. Also, the improved analog of mechlorethamine is cyclophosphamide.

### METHODS FOR MAKING MOLECULES OF THE INVENTION

There are multiple approaches to making site specific mutations in the CH₂ domain of an immunoglobulin molecule. One approach entails PCR amplification of the CH₂ domain with the mutations followed by homologous recombination of the mutated CH₂ into the vector containing the desired immunoglobulin, e.g., hBR96-2. For example, hBR96-2B and hBR96-2D have been made by this method.

Another approach would be to introduce mutations by site-directed mutagenesis of single-stranded DNA. For example, vector pD 17-hG 1 b, which contains only the constant region of IgG1 and not the V domain of hBR96, has the fl origin of replication. This gives the vector the properties of a phagemid and site-directed mutagenesis experiments can be performed according to the methods of Kunkel, et al. (Kunkel, T.A., J.D. Roberts, and R.A. Zakour, 1987 Methods Enzymol. 154:367-383) as provided in the Bio-Rad Muta-Gene® phagemid *in vitro* mutagenesis kit, version 2. For example, hBR96-2B, -C, -D, -E, -F, -G, and -H were made by this method.

In order that the invention described herein may be more fully understood, the following examples are set forth. It should be understood that these examples are for illustrative purposes only and are not to be construed as limiting the scope of this invention in any manner.

### EXAMPLE 1

The following standard ELISA protocol was used.

**Materials:** Immulon2 96 well plates and Genetic Systems Specimen Diluent Concentrate (10x); antibody conjugate was Goat Anti Human Kappa-HRP Mouse Adsorbed, Southern Biotech. at 1:10,000 in Genetic Systems Conjugate Diluent (1x); Genetic Systems EIA Chromogen Reagent (TMB) (1:100); Genetic Systems EIA Buffered Substrate (1x); primary antibody or antigen were AffiniPure F(ab')₂ Fragment Goat Anti Human IgG Fc Fragment specific (Jackson Immuno Research), Goat Anti Human Kappa-UNLB (Southern Biotechnology Associates), Le^{y}-HSA (Alberta Research Council).

**Methods:** Dilute primary antibody or antigen to 1.0 µg/ml in 0.05M Carb/Bicarb buffer. Add 100µl of the diluted solution per well in Immulon 2 plates. Seal plates and incubate O.N. at 4°C.

Block plates by flicking them and blotting on paper towels. Add 200µl/well of Genetic Systems, Specimen Diluent Concentrate (1x). Incubate at least 1 hour at room temperature and then dump the contents of the plates. Wash the plates 3x in saline/Tween. Blot to dry. Allow the plates to dry at R.T. (45 min. to 1 hour). Seal and store the plates at 4°C.

Test samples as follows. Dilute samples and standards in Specimen Diluent at 1:10. Perform serial dilutions in separate round bottom plates. Transfer 100µl/well of final dilutions to antigen coated assay plates; then incubate O.N. at 4°C. Wash plates 3x with saline/Tween.

For conjugation add 100 µl/well of antibody-HRP conjugate in Genetic Systems Conjugate Diluent (1x). Incubate plates at Room Temp. for 60 min. Wash plates 3x in saline/Tween.

Add 100 µl/well of Genetic Systems EIA Chromogen Reagent (TMB) 1:100 in EIA Buffered Substrate (1x). Incubate at R.T. for 15 min. and stop with IN H₂SO₄ 100 µl/well. Read plate at 450/630nm in EIA plate reader.

### EXAMPLE 2

Construction of CH₂ deleted BR96 molecules

Strategy for Deleting CH₂ Domains: To construct CH₂ deleted BR96 molecules, the hinge, CH₂ and CH₃ domains were removed from chimeric BR96 and humanized BR9696-2 IgG1 molecules by an Eco47-III restriction digestion in non-coding regions. The hinge and CH₃ domains were amplified by polymerase chain reaction (PCR) from a human IgG1 (pNγ1.14) molecule lacking the CH₂ domain. Two oligonucleotides (Sense 49mer, Antisense 50mer) homologous to the sequences of IgG1 constant region at both sides preserving E.co47-III sites were synthesized. The amplified hinge and CH₃ domain PCR fragments were added into Eco47-III sites on BR96 IgG1 molecules by in vivo homologous recombination (P. Bubeck et al., Nucleic Acid Research (1993) 21:3601-3602). The new BR96 IgG1 molecules were verified by restriction mapping and sequencing.

A sewing PCR strategy was used for the construction of CH₂ deleted human IgG 1 (pNγ1.14) (Robert M. Horton, et al. (1990) Biotech 8 (5)P, 528).

The CH₁ domain was amplified as a 580 bp fragment with a sense oligonucleotide (5' TGG CAC CGA **AAG CTT** TCT GGG GCA GGC CAG GCC TGA 3') (primer A) and an antisense oligonucleotide (5' **TCC GAG CAT GTT GGT ACC CAC GTG GTG GTC GAC** GCT GAG CCT GGC TTC GAG CAG ACA 3') (primer B) from a linearized human IgG1 constant region vector (pNγ1.7). The PCR fragment extends from the 5' end of the Hind-III site (in bold) through the Cel-II, Sal-I, Dra-III, Kpn-I, 6 bp nucleotide spacer and Mro-I sites (in bold) at the 3' end of the CH₁ domain.

The CH₃ domain was then partially amplified (to the Xba-I site) with a sense primer (5' **GTC GAC CAC CAC GTG GGT ACC AAC ATG TCC GGA** GCC ACA TGG ACA GAG GCC GGC T 3') (primer C) and an antisense primer (5' CTG GTT CTT GTT CAT CTC CTC **TCT AGA** TGG 3') (primer D) from a linearized human IgGI constant region vector (pNγ1.7). A PCR fragment (about 150 bp) with Sal-I, Dra-III, Kpn-I, 6 nucleotide spacer and Mro-I sites (in bold) on its 5' end, extends only through the Xba-1 site (in bold) within the CH₃ domain.

The CH₁ and CH₃ partial PCR fragments were combined in a PCR without any primer. The reaction was run through two full cycles of denaturation and reannealing to allow the fragments to combine at the homologous region at the 3' ends. Primers A and D (described above) were added to the reaction and the PCR cycle was completed. The polymerase extends the DNA with primer A and primer D, yielding a full- length (660 bp) PCR fragment. The newly extended PCR fragment is arranged from the 5' end to the 3' end in the following order: Hind-III - CH₁ - Cel-II - Sal-I - Dra-III - Kpn-I - 6 bp spacer - Mro-I - CH₃ partial - Xba-1.

The combined PCR fragment, with the CH₁ and partial CH₃ domains, was then cloned by a blunt end ligation into a Sma-I site on a pEMBL18 vector and the sequence was confirmed by dideoxy sequencing (Sanger et al. (1977) PNAS (USA) 74:5463-5466).

To transfer the CH₁ and partial CH₃ into a mammalian expression vector, both the pEMBL18 and pNγ1.7 vectors were digested with Hind-III and Xba-I. The Hind-III and Xba-I fragment was ligated into the same sites on a linearized pNγ1.7 vector. The new construct, with CH₁ and a full CH₃ domain, was designated the pNγ1.10 vector.

The hinge fragment was amplified from a Hind-III digested pNγ1.7 vector with the primers designed to flank the hinge exon with a Sal-I and a Dra-III cloning site at each end. These sites also exist between the CH₁ and CH₃ domains of the pNγ1.10 construct. The sense oligonucleotide (5' ACC ATG **GTC GAC** CTC AGA CCT GCC AAG AGC CAT ATC 3') with a 6 bp spacer and a Sal-I cloning site (in bold) and the antisense oligonucleotide (5' CAT GGT **CAC GTG** GTG TGT CCC TGG ATG CAG GCT ACT CTA G 3') with a 6 bp spacer and a Dra-III cloning site (in bold) were used for the amplication of the hinge fragment (250 bp). The hinge region PCR fragment was cloned into a Sma-I site on pEMBL18 by blunt end ligation. Both the pEMBL18 with the hinge domain and the pNγ1.10 with the CH₂ and CH₃ domains were digested with Sal-1 and Dra-III. The digested hinge fragment was cloned into the Sal-1 and Dra-III linearized sites on the pNγ1.10 vector. The new construct, now carrying the CH₁, hinge and CH₃ domains, was designated pNγ1.11.

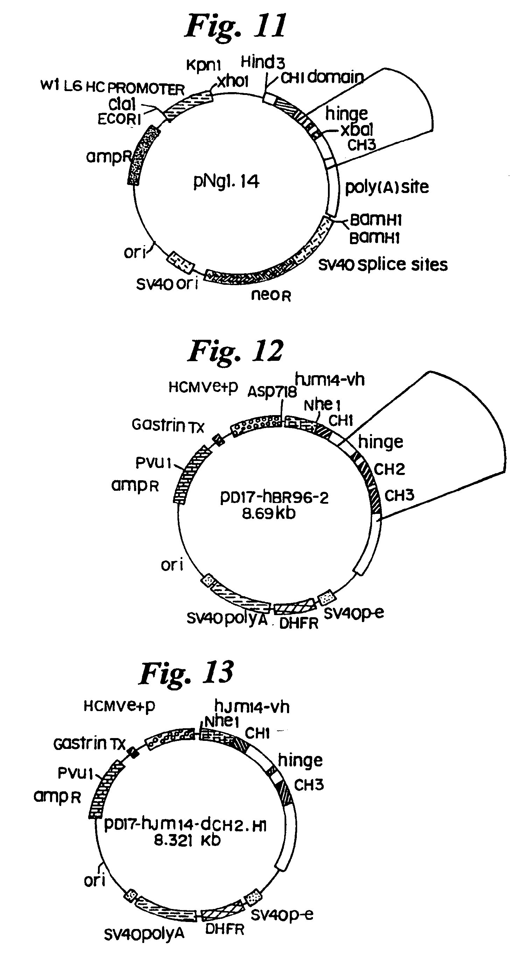

To make the final CH₂ deleted human IgG1 construct, both the pNγ1.11 construct and pNγ1.11 vector were digested with BamH1 and HindIII. A fragment containing the CH₁, hinge and CH₃ domains was cloned into the linearized pNγ1.11 vector. The new constant region IgG1 construct lacks the CH₂ domain and is designated pNγ1.14 (Figure 11).

For digestion of BR96 IgG1 with Eco47-III, a restriction fragment with hinge, CH₂ - and CH₃ domains was identified on the constant region sequence of BR96 IgG1 vector in both chimeric and humanized molecules. The 5' end of this fragment lies inside the intron between CH₁ and hinge and the 3' end is located inside the CH₃ intron of the BR96 IgG1 molecule. The hinge, CH₂ and CH₃ domains (1.368 kb fragment) were removed from BR96 IgG1 molecules by Eco47-III restriction digestion. The Eco47-III is a blunt end cutter. The BR96 IgG1 DNA digested with this enzyme does not require any pretreatment before cloning. Figure 12 is a diagrammatic representation of the pD17-hBR96-2 vector showing the Eco47-III sites used in cloning.

The CH₂ deleted BR96 IgG 1 was then constructed as follows. The hinge and CH₃ domains were amplified from a CH₂ deleted L6 IgG1 (pNγ1.14) construct with a sense oligonucleotide (5' CAGGGAGGGAGGGTGTCTGCTGGAAGCCAGGCTC**AGCGCT**GACCTCAG A 3') homologous to the constant region sequence of IgGI at the 5' end of the Eco47-III site (in bold) and an antisense oligonucleotide (5'GGAAAGAACCATCACAGTCTCGCAGGGG CCCAGGGC**AGCGCT**GGGTGCTT 3') homologous to the constant region sequence of IgG1 at the 3' end of the Eco47-III site (in bold). The Eco47-III site at the 3' end of the pNγ1.14 construct is modified in the cloning process. The Eco47-III site is thus introduced into an antisense primer and used in amplification of the hinge and CH₃ domains.

The pD17-BR96 IgG1 vector was digested with Eco47-III and the hinge, CH₂ and CH₃ domains were removed. The linearized pD17-BR96 IgG1 vector was mixed with equimolar amounts of hinge and CH₃ PCR fragments. Cotransformation of the PCR fragment with linearized DNA into E.coli DH5a competent cells resulted in a recombinant molecule, mediated by homologous recombination in bacteria. This construct lacks the CH₂ domain of BR96 IgG1 molecules, and is designated pD17-BR96-dCH2 (Figure 13).

1.9 grams of CH₂-deleted chimeric BR96 was obtained as raw material from 89L of culture supernatant.

### EXAMPLE 3

Toxicity, localization and clearance of CH₂-deleted chimeric BR96 was tested in vivo as follows.

Three dogs received 400 mg/m² of cBR96-A, the CH₂ deletion mutant of chimeric BR96, and two received chimeric BR96. Both molecules had been mildly reduced and alkylated. This is required to prevent dimerization of the deletion mutant into a tetravalent form. Both control dogs experienced the typical GI toxicity and none of the three receiving the mutant displayed any toxicity. The control dogs and two of the test dogs were sacrificed at 1 hr to obtain duodenal tissue to measure antibody localization. Both control dogs had grossly visible GI pathology, and the test dogs had normal appearing GI tissue. The third dog has continued to show no signs of toxicity.

**Results:** A significant amount of localization of the CH₂ deleted cBR96 (cBR96-A) occurred to the GI tract in dogs treated with 400 mg/m², although the intact chiBR96 localized slightly better. The levels of localization indicate that roughly equivalent amounts of intact and CH₂ deleted cBR96 was delivered to the GI tract in these dogs.

**Table 5. Localization of cBR96 to GI tissue.**

| Group | Animal | Specific | mean |
|---|---|---|---|
| | | Localization | |
| | #271 | 155 | |
| cBR96 | | | 135 |
| | #272 | 114 | |
| | #273 | 126 | |
| cBR96-A | | | 89 |
| | #274 | 52 | |

Using the mean level of specific localization, an amount of cBR96-A equivalent to at least 66% of the amount of cBR96 was delivered to the target organ of toxicity, the duodenum. Based on the dose ranging done with cBR96 in dogs (some clinical signs of toxicity seen at doses of 10 mg/m²), even if this difference is real, it could not explain the difference between significant toxicity and no toxicity, evaluation to date indicated that dogs treated with cBR96-A had no toxicity, pending microscopic histopathologic examination. This evaluation was based on analysis of 2 frozen blocks per dog and 2 sections per block. Replicates were quite good. We also ran historical frozen tissues from dogs treated with native cBR96 or F(ab)2/BR96 and the levels of localization for those tissues were 110 and 0, respectively, consistent with our previous data.

Assuming that there is no toxicity at marginally higher (2X) doses of cBR96-A, these data indicate that the CH₂ domain is associated with the induction of acute gastroenteropathy, and that the removal of this domain prevents the induction of gastroenteropathy mediated by BR96.

This study confirms the results showing that F(ab')2 is not toxic in the dog model and that the toxicity is mediated by the constant region. The CH₂ deletion mutant is a candidate for targeting agents clinically. Because of the very long half-life of chimeric BR96, some decrease in the mutant's half life should be acceptable.

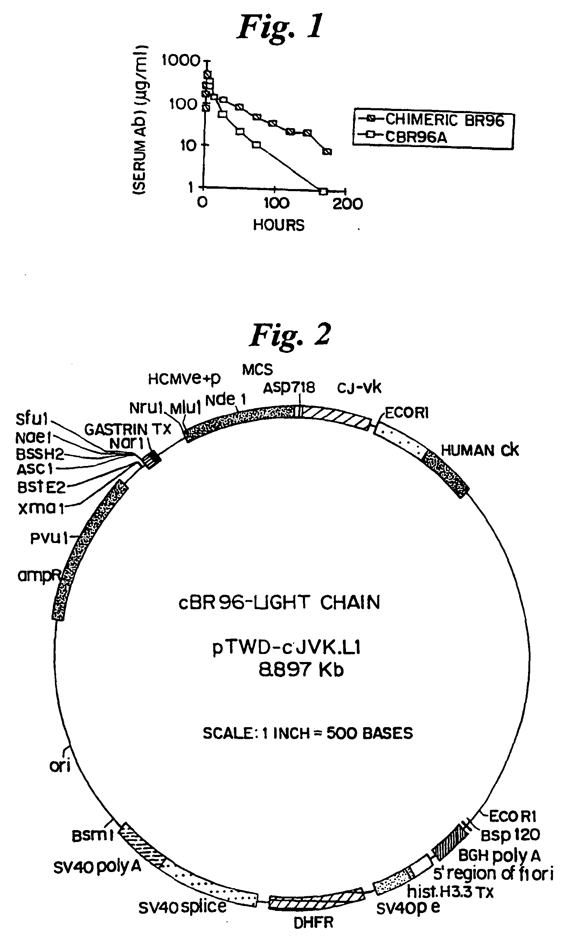

Figure 1 shows the measurement of the clearance of the cBR96-A in high Le^{Y} expressing dogs. The study used chimeric versus constant region mutant of cBR96-2.

CBR96-2 did clear faster than the chimeric BR96. The localization of cBR96-A to the gastrointestinal epithelium is not significantly affected by this more rapid clearance. More than enough of the cBR96-A localized to have caused toxicity.

**Discussion:** The constant region of chimeric IgG is responsible for the GI toxicity seen in clinical trials, e.g. with chiBR96-dox. The GI toxicity seen in the dog model is very similar to the clinical toxicity. Both in man and dog, administration of the unconjugated antibody mediates an acute GI toxicity characterized by rapid onset of vomiting, often with blood.

In man the bleeding is limited to the fundus of the stomach, causing erosion of the superficial mucosa of the stomach. Although the pathology of the wound is limited and resolves, the extreme nature of the nausea and vomiting, unrelieved by anti-emetics, defines it as the dose-limiting toxicity.

This toxicity is mediated in man and dog by the antibody molecule alone. At higher doses of the antibody-dox conjugate, additional toxicity is seen in the dog model, probably due to doxorubicin. Although the intact IgG of BR96 causes toxicity in dog and man, the F(ab')2 molecule (divalent and lacking only in the constant region) is not toxic in dogs. This finding has motivated our attempts at high levels, and improves the affinity and specificity of BR96 for tumor antigen.

The CH₂ domain is known to mediate complement and FcR binding. It was not known that structural alteration of the CH₂ domain would result in immunoglobulin-induced toxicity inhibition.

### Toxicology study of hBR96-2B

The toxicology study of hBR96-2B in high Lewis Y expressor dogs (n=2) showed that a dose of 400 mg/m² did not cause hematemesis nor bloody stools, in contrast to BR96 which consistently causes one or both signs. A dog sacrificed at 24 hrs had normal gross appearance of the GI tract, again in marked contrast to chimeric BR96 which causes hemorrhagic lesions and mucosal erosions.

### EXAMPLE 4

The polymerase chain reaction (PCR) is a widely used and versatile technique for the amplification and subsequent modification of immunoglobulin genes. The rapidity and accuracy with which antibody genes can be modified in vitro has produced an assortment of novel antibody genes can be modified in vitro has produced an assortment of novel antibodies. For example, PCR methods have been used for engineering antibodies with increased affinity to antigen, for "humanizing" antibodies, and for modulating effector function (Marks, J.D., A.D. Griffiths, M. Malmqvist, T. Clackson, J.M. Bye and G. Winter. 1992. Bypassing immunization: high affinity human antibodies by chain shuffling. Bio/Technology 10:779-783; Rosok, M.J., D.E. Yelton, L.J. Harris, J. Bajorath, K.-E. Hellstrom, 1. Hellstrom, G.A. Cruz, K. Kristensson, H. Lin, W.D. Huse and S.M. Glaser. 1996. A combinatorial library strategy for the rapid humanization of anticarcinoma BR96 Fab. J. Biol. Chem. 271:22611-22618; Morgan, A.N., D. Jones, A.M. Nesbitt, L. Chaplin, M.W. Bodmer and S. Emtage. 1995. The N-terminal end of the CH2 domain of chimeric human IgG1 anti-HLA-DR is necessary for Clq, FcγRI and FcγRIII binding. Immunology. 86:319-324).

As part of a more comprehensive study, we desired to introduce various site specific mutations in the CH₂ constant domain of human IgG₁. Six specific amino acid residues distributed throughout the CH2 domain previously identified to play a role in immune effector function were marked as targets for mutagenesis (Morgan, A.N., D. Jones, A.M. Nesbitt, L. Chaplin, M.W. Bodmer and S. Emtage. 1995. The N-terminal end of the CH2 domain of chimeric human IgG1 anti-HLA-DR is necessary for Clq, FcγRI and FcγRIII binding. Immunology. 86:319-324; Duncan, A.R. and G. Winter. 1988. The binding site for C1q on IgG. Nature 332:738-740; Tao, M.-H., R.I.F. Smith and S.L. Morrison. 1993. Structural features of human immunoglobulin G that determine isotype-specific differences in complement activation. J.Exp.Med. 178:661-667). five of the six residues were grouped into two clusters-one cluster consisting of two residues, two amino acids apart (Location 1, or L1); and a second cluster consisting of three residues spanning a sequence of five amino acids (L2). The remaining amino acid position (L3) made for the total of six residues. We were interested in constructing a panel of mutant CH₂ domain IgGs consisting of each L mutation by itself as well as in combination with other L mutants (e.g., L1; L1; and L2; L1, L2 and L3; etc.).

Various *in vitro* methods have been described where PCR is used to simultaneously introduce distally located site-specific mutations within a gene sequence (Ho, S.N., H.D. Hunt, RM. Horton, J.K. Pullen and L.R Pease. 1989. Site-directed mutagenesis by overlap extension. Gene 77:51-59; Ge, L. and P. Rudolpf. 1996. Simultaneous introduction of multiple mutations using overlap extention PCR BioTechniques 22:28-30). Alternatively, an *in vivo* procedure termed recombination PCR (RPCR) has also successfully been used for rapidly and efficiently generating distally located site-specific mutations (Jones, D.H. and S.C. Winistorfer. 1993. Use of polymerase chain reaction for making recombinant constructs. p.241-250. In B.A. White (Ed.), Methods in Molecular Biology, Vol. 15. Humana Press Inc., Totowa, NJ, Jones, D.H. And B.H. Howard. 1991. A rapid method for recombination and site-specific mutagenesis by placing homologous ends on DNA using polymerase chain reaction. BioTechniques 10:62-66). RPCR uses *E. Coli's* recombination machinery to generate intact circular recombinant plasmids from a transfected mixture of linear PCR-generated product and linearized vector. *In vivo* recombination is mediated through the joining of nucleotide sequences designed into the 5' ends of both PCR primers that are homologous to DNA sequences encoded by the vector. In this report we describe an extension of the RPCR procedure for simultaneously introducing complex combinations of mutations into an antibody CH₂ domain.

Humanized BR96 variable region heavy and light chain genes, previously cloned and co-expressed as an assembled active Fab fragment in an M13 phage expression vector, provided the starting material (Rosok, M.J., D.E. Yelton, L.J. Harris, J. Bajorath, K.-E. Hellstrom, I. Hellstrom, G.A. Cruz, K. Kristensson, H. Lin, W.D. Huse and S.M. Glaser. 1996. A combinatorial library strategy for the rapid humanization of anticarcinoma BR96 Fab. J. Biol. Chem. 271:22611-22618). The heavy and light chain V genes were amplified by PCR from a single-stranded M 13 DNA template and subcloned by *in vivo* recombination (Jones, D.H. And B.H. Howard. 1991. A rapid method for recombination and site-specific mutagenesis by placing homologous ends on DNA using polymerase chain reaction. BioTechniques 10:62-66) into vectors pD17-hG1a and pD16-hCκ, to form pBR96-hGla and pBR96-hCκ respectively. pD17-hGla and pD16-hCκ are eukaryotic immunoglobulin expression vectors derived from pcDNA3 (Invitrogen, San Diego, CA). The plasmid pBR96-hG1a was further modified by site-directed mutagenesis to introduce two Eco47-III restriction sites flanking the immunoglobulin hinge-CH₂-CH₃ domains using standard procedures. The recipient vector was then prepared by digesting pBR96-hG 1 a with Eco47-III, isolating the vector backbone by agarose gel electrophoresis followed by extracting the vector DNA from the excised gel slice using the Qiagen Gel Extraction kit (Qiagen, Chatsworth, CA).

The strategy for introducing multiple mutations within the immunoglobulin CH₂ gene, shown in Figure 24, relies on the *in vivo* homologous recombination of several independently amplified PCR products with each other as well as with the pBR96-hG1a vector DNA. For introducing mutations at two distal locations two PCR products are synthesized (Figure 24B). One end of each PCR product is for recombining with an homologous end of the linear vector, and the other end, encoding the mutation(s) of interest, is for recombining with the neighboring PCR product. As shown in Figure 24B, additional distally-located mutations can be introduced into a target sequence by increasing the number of PCR products proportionately. The recombination of neighboring PCR-products always occurs across the regions containing the desired mutations, therefore the oligonucleotide primers encoding these ends (e.g., A1, A2) contain complementary mutant residues. The mutagenic PCR primers contain at least 15 nucleotides of wild-type sequence flanking each side of the mutant residues for either priming the polymerization reaction or mediating recombination. Two 49-nucleotide long PCR sense and anti-sense primers (Rs and Ra) contain sequences for recombining with the end regions of the Eco47-III digested pBR96-hG1a vector.

Each L mutation was amplified in a separate PCR reaction. The reaction conditions were 250 ng intact pBR96-hG1a DNA template, 10 ul of 1X *Pfu* buffer (Stratagene, Inc. San Diego, CA), 10 nmol dNTPs, 200ng each of the appropriate PCR primers, 10% dimethysulfoxide (ATCC, Rockville, MD) and 2.5 units cloned *Pfu* DNA polymerase in a 100ul reaction volume. Samples were first denatured at 95° C for 5 min, cooled to 45°C for 5 min, and extended at 72°C for 1 min followed by 25 cycles of denaturation at 94°C for 45 sec, annealing at 45°C for 45 sec, extension at 72°C for 1 min/kb, followed by a final extension at 72°C for 7 min in a Perkin-Elmer DNA Thermal Cycler (Norwalk, CT). The amplified products were purified from a 1% agarose gel, extracted with Qiagen Gel Extraction kit and the recovered DNA quantitated. 50 ng of each PCR product was mixed with 25 ng of the Eco47-III digested pBR96-hG1a vector, transfected into Max competent E. coli DH5α according to the manufacturer's procedure (GIBCO BRL/Life Technologies, Gaithersburg, MD), and the entire transfection reaction plated onto selective LB agar plates containing 100 ug/ml ampicillin.

The results of several cloning experiments are summarized in the Table that follows. Typically the transformations produced from 80 to 200 bacterial colonies. Individual colonies were selected and grown overnight in 2 ml liquid cultures for isolation of miniprep plasmid DNA (Qiagen) and analysis by Eco47-III restriction endonuclease mapping. Among 24 independent transformants analyzed from triple homologous recombination events (two PCR products plus vector) 11 clones contained the predicted 1.4 kpb DNA insert.

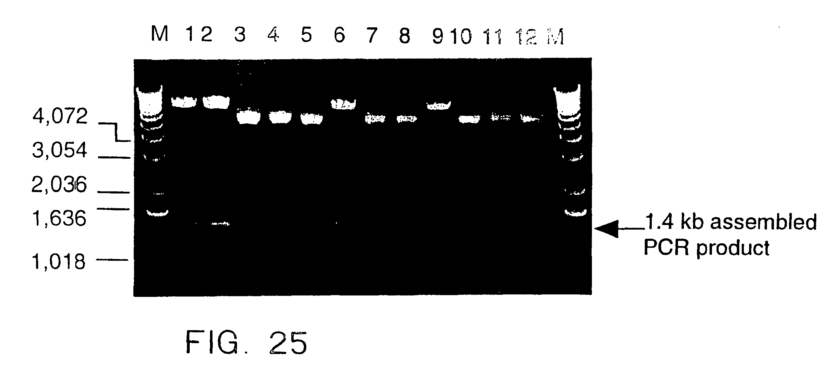

Figure 25 shows a sample diagnostic restriction analysis of DNA prepared from clones derived from quadruple homologous recombination events (three PCR products plus vector). Additional sampling of clones resulting from quadruple recombination yielded a cloning efficiency of 29% (7 clones containing inserts/24 clones sampled). At this point, due to the small sampling sizes, we do not know whether the differences in the cloning efficiencies observed between the triple and quadruple recombination events are meaningful.

To evaluate the expression of Le^{γ} -binding activity of the CH₂ mutant IgGs, miniprep DNAs from 6 clones derived from the triple recombination reaction and 6 clones derived from the quadruple recombination reaction exhibiting the predicted diagnostic Eco47-III restriction patterns were isolated, mixed with pBR96- hC_{κ} DNA and used to co-transfect COS7 cells. 48 hour spent supernatants from 3 ml cultures were assayed for total IgG production and for Leγ binding activity by enzyme-linked immunosorbent assay (EIA) as described (Yelton, D.E., M.J. Rosok, G.A. Cruz, W.L. Cosand, J. Bajorath, I. Hellstom, K.-E. Hellstorm, W.D. Huse and S.M. Glaser. 1995. Affinity maturation of the BR96 anti-carcinoma antibody by codon-based mutagenesis. J.Immunol. 155:1994-2004). All twelve cultures were found to secrete approximately 2-3 ug/ml Le^{γ} -reactive IgG. The spectrum of Leγ binding activities were all similar to that of native humanized BR96 IgG indicating that the homologously recombined antibodies did not acquire any gross mutations that could affect antigen binding. To confirm that the desired CH₂ mutations had been incorporated, and to evaluate the recombined genes for misincorporated nucleotides, four of the clones producing functional antibody were sequenced using Sequenase Version 2 DNA Sequencing Kit (United States Biochemical). One clone was found to contain a single nucleotide change within the forward PCR primer used for mediating recombination with vector DNA. We are uncertain whether this error occurred during chemical synthesis of the oligonucleotide primer or is a result of misincorporation during the PCR reaction, despite the fact that we used a thermostable polymerase with proofreading activity.

A RPCR procedure for homologously recombining up to three separate PCR-generated mutated antibody sequence products into a eukaryotic expression vector for the rapid construction of engineered IgG molecules is described herein. The advantage of this approach is the ability to simultaneously introduce multiple distally-located mutations with PCR products synthesized by a single round of PCR. Recombinant DNAs are produced with a reasonably high cloning efficiency and fidelity of correct nucleotide sequences. The ability to efficiently rejoin several distinct PCR products should permit combinatorial strategies for constructing complexly mutated protein domains as well as broadening the number and location of desired mutations.

Analysis of transformants generated by multiple-fragment RPCR.

| Mutant IgGs Constructed | PCR Fragments in reaction | HR^{a} events | Colonies Analyzed | Cloning Efficiency^{b} |
|---|---|---|---|---|
| 2 | 2 | triple | 24 | 45% |
| 2 | 3 | quadruple | 24 | 33% |
| ^{a}HR-homologous recombination | | | | |
| ^{b}cloning efficiency (number of clones containing 1.4kbp insert/total number of colonies | | | | |

### EXAMPLE 5

This example provides two methods for introducing site specific mutations into the CH2 domain of human IgG1 constant region containing vectors.

One method involves PCR amplification of a segment or segments of the constant region, wherein mutations are introduced using appropriately constructed oligonucleotides. The vector receiving the fragment(s) is digested with a restriction enzyme to linearize the vector. PCR amplification primers are designed so that the 5' ends of the PCR fragments can hybridize to the DNA sequence of the vectors. If more than one PCR fragment is amplified, then common sequences to the two fragments are introduced by oligonucleotides. Bacteria are transfected with the PCR fragments and with the digested vector. The fragments and vector can recombine by homologous recombination using the bacteria's recombination machinery. Bacterial colonies are selected and the DNA is analyzed by size and restriction map as a preliminary determination that the vector and fragment(s) recombined correctly. Correct insertion of fragments with the mutations is confirmed by dideoxynucleotide sequence analysis. DNA is then introduced into mammalian cells as described for the CH2 deleted antibody, and the expressed antibody analyzed for binding and functional activity.

By way of example, mutations Leu to Ala at residue 235 in CH2 and Gly to Ala at residue 237 were introduced by the procedure disclosed in Example 4. The heavy chain vector used for this procedure was pD17-hG1a, similar to pD17-BR96 vector described herein except that humanized V regions (Rosok, M.J., D.E. Yelton, L.J. Harris, J. Bajorath, K-E. Hellstrom, I, Hellstrom, G.A. Cruz, K. Kristensson, H. Lin, W.D. Huse, and S.M. Glaser, 1996. J. Biol. Chem 271 37:22611-22618) with three affinity mutations (H1, H2, and H3 mutations) were substituted. pBR96-hG1a contains two Eco47-III restriction sites flanking the Ig hinge-CH2-CH3 domains. The recipient vector was prepared by (1) digesting pBR96-hG1a with *Eco*47-III, (2) isolating the vector by agarose gel electrophoresis, and (3) extracting the vector DNA from the excised gel slice using the Qiagen Gel Extraction kit (Qiagen, Chatsworth, CA). To introduce mutations at a single location, such as for positions 235 and 237, two PCR products were synthesized.

To introduce two distally located mutations, such as for mutant F (also referred to herein as hBR96-2F) with mutations at 235, 237, 331, requires 3 PCR products. The recombination of neighboring PCR products occurs across the regions containing the desired mutations, therefore the oligonucleotide primers encoding these ends contain complementary mutant residues. The mutagenic PCR primers contain at least 15 nucleotides of wild-type sequence flanking each side of the mutant residues for either priming the polymerization reaction or mediating recombination. Two 49-nucleotide long PCR sense and anti-sense primers containing sequences for recombining with the end regions of the *Eco*47-III digested pBR96-hG1a vector.

PCR amplification used 250 ng intact pBR96-hG1a DNA template, 10 µl of 10X *Pfu* buffer (Stratagene, Inc., San Diego, CA), 10 nmol dNTPs, 200 ng each of the appropriate PCR primers, 10% dimethylsulfoxide (ATCC, Rockville, MD) and 2.5 units cloned *Pfu* DNA polymerase (Stratagen, Inc. San Diego, CA) in 100 µl reaction. Samples were denatured at 95°C for 5 min, annealed at 45°C for 5 min, and extended at 72°C for 1 min followed by 25 cycles of denaturation at 94°C for 45 sec, annealing at 45°C for 45 sec, extension at 72°C for 1 min/kb, and a final extension at 72°C for 7 min. The amplified products were purified from a 1% agarose gel, extracted with the Qiagen Gel Extraction kit and quantitated. 50 mg of each PCR product was mixed with 25 ng of the Eco47-III digested pBR96-hG1a vector and transfected in E.coli MAX Efficiency DH5α^{™} according to the manufacturer's instructions (GIBCO BRL/Life Technologies; Gaithersburg, MD). The entire transfection reaction was plated onto LB agar plated containing 100 µg/ml ampicillin.

Bacterial colonies were selected and grown overnight at 37° C in 2 ml liquid cultures. DNA was isolated and analyzed by Eco47-III restriction endonuclease mapping. Clones with the correct size insert were sequenced (Sequenase Version 2, U.S. Biochemical Corp., Cleveland, OH).

The second method for introducing site specific mutations into the CH₂ domain of human IgG1 involved the method of Kunkel (1987 Methods Enzymology, supra). For this procedure pD17-hG1b DNA with the F1 origin of replication was introduced into electrocompetent E. coli CJ236 dut-ung- (Bio-Rad Laboratories, Hercules, CA) by electroporation according to manufacturer's instructions. PD17-hGlb is a vector having a constant region but no variable region. The F1 ori site allows treatment of this vector as a phagemid.

Bacteria containing the plasmid were selected by ampicillin resistance. Single stranded uridinylated DNA was prepared using the Muta-Gene Phagemid In Vitro Mutagenesis Version 2 protocol (Bio-Rad). Mutations were introduced by site-directed mutagenesis with the appropriate antisense oligonucleotide. For molecules with mutations at more than one location, mutations were introduced by either of the two methods discussed above. One method would be to (1) prepare one mutant, for example, mutant 2C (also referred to herein as BR96-2C) with the mutations at residues 318, 320, 322, (2) isolate ssDNA, and (3) introduce a second mutation set with the appropriate anti-sense oligonucleotide. The second method would be to anneal two antisense oligonucleotides with the same uridinylated ssDNA and screen for mutants with both sets of changes. Mutant 2H (hBR96-2H) was also prepared by a combination of thse methods.

The V region of humanized BR96-2 heavy chain was introduced by the homologous recombination method described above in pD 17-hJm 14.H 1. The pD17-hJm14.H1 plasmid contains the BR96 humanized variable region with the H1/H2/H3 mutations and the plasmid was used to transfect mutant sequences into mammalian cells. The pD17Glb vector containing the Fc mutation(s) was digested with NheI for 3 hr at 37° C and the DNA isolated by methods described above. Insertion of the V region into the vector was determined by size and restriction enzyme mapping and confirmed by sequence analysis.

Transient expression of whole antibodies was performed by transfection of COS cells. For production of antibody, stable transfections of CHO cells were performed (see description of deleted CH2 mutant). All mutants were purified from CHO culture supernatants by protein A chromatography.

The oligonucleotide primers homologous to the vector and used to introduce the constant regions mutations were as follows:
Oligonucleotides homologous to vector sequences:
Oligonucleotides to mutate Leu235 to Ala and Gly237 to Ala (underlined sequences show sites of mutation):
Oligonucleotides to mutate Glu318, Lys320, Lys322 to Ser
Oligonucleotides to mutate Pro331 to Ala:
   **Antis CH2 P331/A/3:** GAT GGT TTT CTC GAT GGC GGC TGG GAG GGC
   **Sense CH2 P33/A:** GCC CTC CCA GCC GCC ATC GAG AAA ACC ATC
Alternative antisense oligo to introduce Ala at 331 by site-directed mutation:
   **CH2P331A:** GAT GGT TTT CTC GAT AGC GGC TGG GAG GGC TTT G
Oligonucleotides to mutate Glu318 to Ser, Lys320 to Ser, Lys322 to Ser, and Pro331 to Ala:

### In vitro Assays of the Mutants

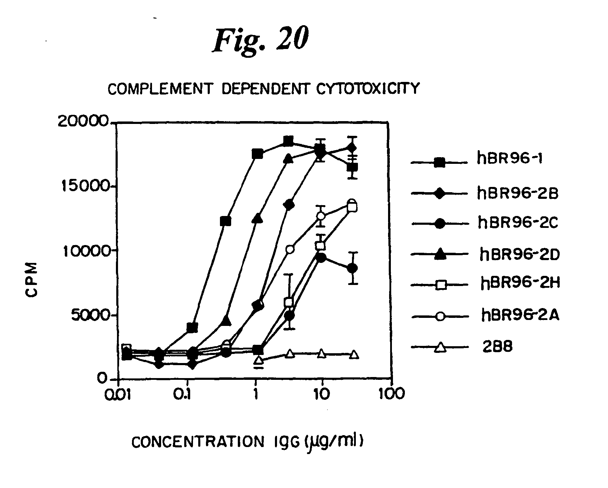

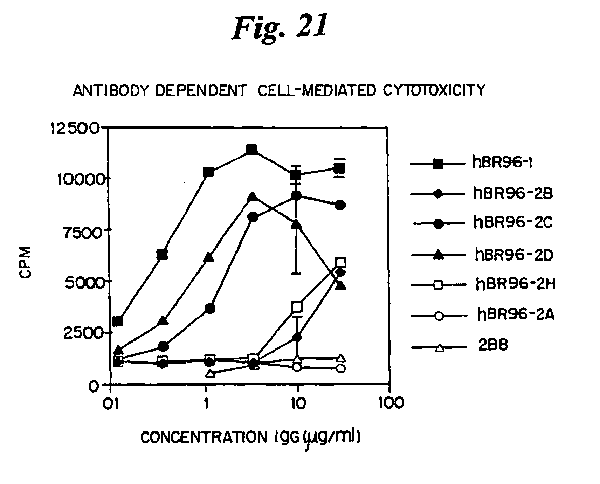

Results of the CDC demonstrate that mutant hBR96-2B has approximately 10 fold less activity than the control hBR96-1 (two affinity mutations, one in H2 and one in H3, refer to previous patent (Figure 20)). The mutants that have the least ability to kill cells in the presence of complement is hBR96-2C with the triple mutations at positions 318, 320, and 322 and the hBR96-2H mutant (least cytotoxic antibodies in the panel) which contains all six mutations at the three different locations. ADCC activity was most affected by the CH2 deleted hBR96-2 molecule (Figure 21). hBR96-2B and -2H lost between 100 and 1000 fold activity to kill in the presence of effector cells. In the ADCC assay the hBR96-2B molecule also lost approximately 10 fold activity (Figure 21).

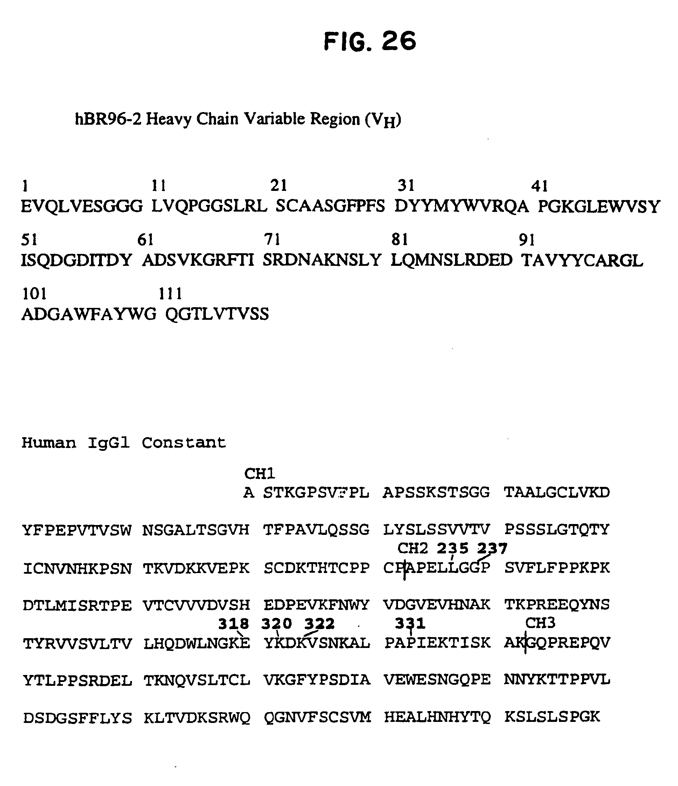

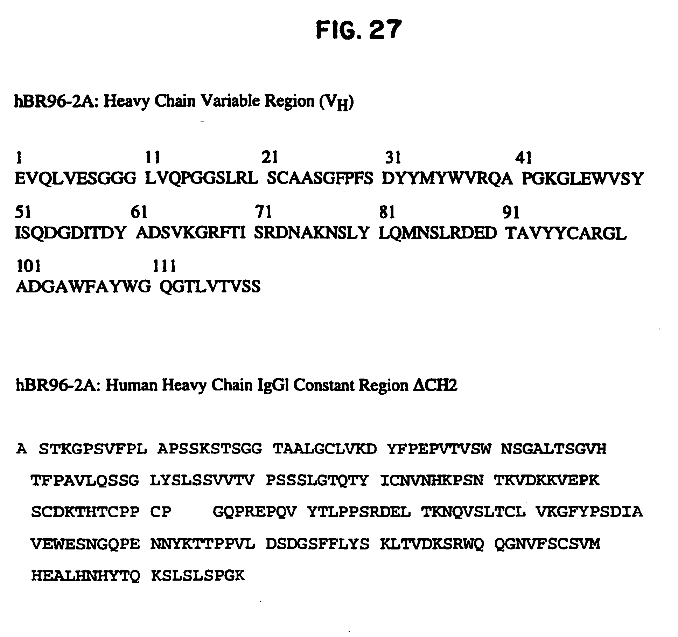

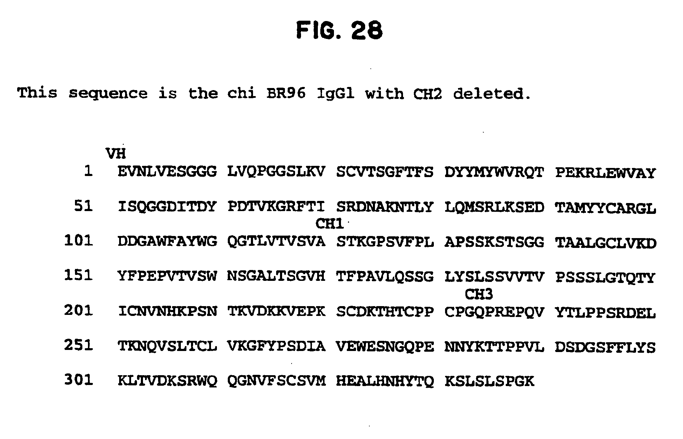

Figures 26-28 provide the amino acid sequences for the heavy chain variable region for both chimeric and humanized BR96 having the H1, H2, and H3 mutations. The amino acid sequence for the light chain variable region is known and methods for generating it are found in PCT Application No. 95/305444. Additionally provided is the amino acid sequence for the IgG1 constant region. Mutations in the constant region are marked.

## Claims

1. A BR96 antibody having a human IgG 1 constant region which has been structurally altered in the CH2 domain wherein leucine at amino acid position 235 is mutated to alanine; glycine at amino acid position 237 is mutated to alanine; glutamic acid at amino acid position 318 is mutated to serine; lysine at amino acid position 320 is mutated to serine; and lysine at amino acid position 322 is mutated to serine.

2. A BR96 antibody having a human IgG 1 constant region which has been structurally altered in the CH2 domain wherein leucine at amino acid position 235 is mutated to alanine; glycine at amino acid position 237 is mutated to alanine; and proline at amino acid position 331 is mutated to alanine.

3. A BR96 antibody having a human IgG1 constant region which has been structurally altered in the CH2 domain wherein leucine at amino acid position 235 is mutated to alanine; glycine at amino acid position 237 is mutated to alanine; glutamic acid at amino acid position 318 is mutated to serine; lysine at amino acid position 320 is mutated to serine; lysine at amino acid position 322 is mutated to serine; and proline at amino acid position 331 is mutated to alanine.

4. A nucleic acid molecule which encodes an antibody selected from the BR96 antibodies of Claims 1-3.

5. A cDNA of Claim 4.

6. A plasmid which comprises the nucleic acid molecule of Claim 4.

7. A host vector system comprising a plasmid of Claim 6 in a suitable host cell.

8. A method for producing a protein comprising growing said host cell of Claim 7 containing said plasmid so as to produce the protein in the host and recovering the protein so produced.

9. A pharmaceutical composition comprising an acceptable carrier and a pharmaceutically effective amount of an immunoglobulin molecule having a variable region and a constant region, the immunoglobulin molecule being modified by structurally altering multiple toxicity-associated domains in the CH2 domain of the constant region of a monoclonal antibody BR96 and said multiple toxicity-associated domains are altered so that toxicity otherwise induced by said monoclonal antibody BR96 is inhibited, wherein said modified immunoglobulin molecule is selected from the antibodies of Claims 1-3.

10. The composition of Claim 9 wherein said structurally altered immunoglobulin molecule recognizes and binds a target associated with cancer.

11. Use of the composition of Claim 10 for manufacturing a medicament for use in a method of treating carcinomas *in vivo* comprising administering a pharmaceutically effective amount of said composition to a subject.

12. The use according to Claim 11 wherein the structurally altered immunoglobulin molecule in the composition is labeled so as to directly or indirectly produce a detectable signal with a compound selected from the group consisting of a radiolabel, an enzyme, a chromophore, a chemiluminescer and a fluorescer.

13. Use of the composition of Claim 10 for manufacturing a medicament for treating a patient suffering from a cancer, the cancer being **characterized** as a group of cells having a tumor-associated antigen on the cell surface, by administration to the subject of a cancer killing amount of said composition comprising immunoglobulin joined to a cytotoxic agent under conditions which permit the molecule so joined to bind the tumor associated antigen on the cell surface so as to kill the cells so bound thereby treating the subject.

14. The use according to Claim 13 wherein said immunoglobulin is conjugated to doxorubicin.

15. Use of an immunoglobulin molecule having a variable region and a constant region, the immunoglobulin molecule being modified by structurally altering multiple toxicity-associated domains in the CH2 domain of the constant region of a monoclonal antibody BR96 and said multiple toxicity-associated domains are altered so that toxicity otherwise induced by said monoclonal antibody BR96 is inhibited, for manufacturing a medicament for inhibiting immunoglobulin-induced toxicity resulting from immunoglobulin immunotherapy in a subject, wherein said modified immunoglobulin molecule is selected from the antibodies of Claims 1-3.

16. The use according to Claim 15 wherein said structurally altered molecule no longer mediates a complement dependent cytotoxicity or antibody dependent cell cytotoxicity.

17. The use according to Claim 15 wherein the immunoglobulin molecule recognizes and binds Le^{y}.

18. A pharmaceutical composition according to Claim 9 for use as a therapeutic agent.

## Patentansprüche

1. BR96-Antikörper, der eine menschliche IgG1-Konstantregion aufweist, die in der CH2-Domäne strukturell geändert worden ist, wobei Leucin an der Aminosäureposition 235 zu Alanin mutiert wird; Glycin an der Aminosäureposition 237 zu Alanin mutiert wird; Glutaminsäure an der Aminosäureposition 318 zu Serin mutiert wird; Lysin an der Aminosäureposition 320 zu Serin mutiert wird; und Lysin an der Aminosäureposition 322 zu Serin mutiert wird.

2. BR96-Antikörper, der eine menschliche IgG1-Konstantregion aufweist, die in der CH2-Domäne strukturell geändert worden ist, wobei Leucin an der Aminosäureposition 235 zu Alanin mutiert wird; Glycin an der Aminosäureposition 237 zu Alanin mutiert wird; und Prolin an der Aminosäureposition 331 zu Alanin mutiert wird.

3. BR96-Antikörper, der eine menschlicher IgG1-Konstantregion aufweist, die in der CH2-Domäne strukturell geändert worden ist, wobei Leucin an der Aminosäureposition 235 zu Alanin mutiert wird; Glycin an der Aminosäureposition 237 zu Alanin mutiert wird; Glutaminsäure an der Aminosäureposition 318 zu Serin mutiert wird; Lysin an der Aminosäureposition 320 zu Serin mutiert wird; Lysin an der Aminosäureposition 322 zu Serin mutiert wird; und Prolin an der Aminosäureposition 331 zu Alanin mutiert wird,

4. Nucleinsäuremolekül, das einen Antikörper ausgewählt unter den BR96.Antikörpem nach den Ansprüchen 1-3 kodiert.

5. cDNA nach Anspruch 4.

6. Plasmid, das das Nucleinsäuremolekül nach Anspruch 4 umfasst.

7. Wirtsvekrorsystem umfassend ein Plasmid nach Anspruch 6 in einer geeigneten Wirtszelle.

8. Methode für die Herstellung eines Proteins, umfassend das Züchten der Wirtszellen nach Anspruch 7, die das Plasmid enthalten, um das Protein in dem Wirt herzustellen, und das Gewinnen des so erzeugten Proteins.

9. Pharmazeutische Zusammensetzung umfassend einen akzeptablen Träger und eine pharmazeutisch wirksame Menge eines Immunoglobulinmoleküls, das eine variable Region und eine Konstantregion aufweist, wobei das Immunoglobulinmolekül durch strukturelles Ändern multipler, mit Toxizität verbundener Domänen in der CH2-Domäne der konstanten Region eines monoklonalen Antikörpers BR96 modifiziert wird und die multiplen, mit Toxizität verbundenen Domänen so geändert werden, dass die Toxizität, die sonst durch den monoklonalen Antikörper BR96 induziert wird, gehemmt wird, wobei das modifizierte Immunoglobulinmolekül unter den Antikörpers nach den Ansprüchen 1-3 ausgewählt wird,

10. Zusammensetzung nach Anspruch 9, wobei das strukturell geänderte Immunoglobulinmolekül ein Target, das mit Krebs verbunden ist, erkennt und bindet.

11. Verwendung der Zusammensetzung nach Anspruch 10 für die Herstellung eines Medikaments zur Verwendung bei einer Methode für die Behandlung von Karzinomen in vivo, umfassend das Verabreichen einer pharmazeutisch wirksamen Menge der Zusammensetzung einem Patienten,

12. Verwendung nach Anspruch 11, wobei das strukturell geänderte Immunoglobulinmolekül in der Zusammensetzung mit einer Verbindung ausgewählt aus der Gruppe bestehend aus einem Radiolabel, einem Enzym, einem Chromophor, einem Chemilumineszenzbildner und einem Fluoreszenzbildner so gelabelt wird, um direkt oder indirekt ein wahrnehmbares Signal zu erzeugen.

13. Verwendung der Zusammensetzung nach Anspruch 10 für die Herstellung eines Medikaments für die Behandlung eines Patienten, der an Krebs leidet, wobei der Krebs als Gruppe von Zellen **gekennzeichnet** ist, die an der Zelloberfläche ein mit Tumor verbundenes Antigen aufweisen, durch Verabreichen dem Patienten einer krebstötenden Menge der Zusammensetzung umfassend ein zytotoxisches Mittel angeknüpftes Immunoglobulin unter Bedingungen, die es dem so angeknüpften Molekül erlauben, das mit dem Tumor verbundene Antigen an der Zelloberfläche so zu binden, dass die so gebundenen Zellen getötet werden, wodurch der Patient behandelt wird.

14. Verwendung nach Anspruch 13, wobei das Immunoglobulin an Doxorubicin konjugiert wird.

15. Verwendung eines Immunoglobulinmoleküls, das eine variable Region und eine Konstantregion aufweist, wobei das Immunoglobulinmolekül durch strukturelles Ändern multipler, mit Toxizität verbundener Domänen in der CH2-Domäne der konstanten Region eines monoklonalen Antikörpers BR96 modifiziert wird und die multiplen, mit Toxizität verbundenen Domänen so geändert werden, dass die Toxizität, die sonst durch den monoklonalen Antikörper BTR96 induziert wird, gehemmt wird, für die Herstellung eines Medikaments für das Hemmen der durch Immunoglobulin induzierten Toxizität, die aus der Immunoglobulinimmuntherapie bei einem Patienten herrührt, wobei das modifizierte Immunoglobulinmolekül unter den Antikörpern nach den Ansprüchen 1-3 ausgewählt wird.

16. Verwendung nach Anspruch 15, wobei das strukturell geänderte Molekül keine komplementabhängige Zytotoxizität oder antikörperabhängige Zellzytotoxizität mehr vermittelt.

17. Verwendung nach Anspruch 15, wobei das Immunoglobulinmolekül Le^{y} erkennt und bindet.

18. Pharmazeutische Zusammensetzung nach Anspruch 9 zur Verwendung als therapeutisches Mittel.

## Revendications

1. Anticorps BR96 possédant la région constante d'une IgGI humaine qui a été modifiée structurellement au niveau du domaine CH2, où la leucine en position 235 est mutée on une alanine; la glycine en position 237 est mutée en une alanine; l'acide glutamique en position 318 est muté en une sérine; la lysine en position 320 est mutée en une sérine; et la lysine en position 322 est mutée en une sérine.

2. Anticorps BR96 possédant la région constante d'une IgG1 humaine qui a été modifiée structurellement au niveau du domaine CH2, où la leucine en position 235 est mutée en une alanine; la glycine en position 237 est mutée en une alanine; et la proline en position 331 est mutée en une alanine.

3. Anticorps BR96 possédant la région constante d'une IgG1 humaine qui a été modifiée structurellement au niveau du domaine CH2, où la leucine en position 235 est mutée en une alanine; la glycine en position 237 est mutée en une alanine; l'acide glutamique en position 318 est muté en une sérine; la lysine en position 320 est mutée en une sérine; la lysine en position 322 est mutée en une sérine; et la proline en position 331 est mutée en une alanine.

4. Molécule d'acide nucléique qui code pour un anticorps sélectionné parmi les anticorps BR96 des revendications 1-3.

5. ADNc de la revendication 4.

6. Plasmide qui comprend la molécule d'acide nucléique de la revendication 4,

7. Système vecteur-hôte qui comprend un plasmide de la revendication 6 dans une cellule hôte appropriée.

8. Procédé de production d'une protéine, qui comprend la culture de ladite cellule hôte de la revendication 7 contenant ledit plasmide de façon à produire la protéine dans l'hôte et à récupérer la protéine ainsi produite.

9. Composition pharmaceutique comprenant un véhicule acceptable et une quantité pharmaceutiquement efficace d'une molécule d'immunoglobuline possédant une région variable et une région constante, la molécule d'immunoglobuline étant modifiée en changeant structurellement des domaines multiples associés à une toxicité du domaine CH2 de la région constante d'un anticorps monoclonal BR96, lesdits domaines multiples associés à une toxicité étant modifiés de telle sorte que la toxicité sinon induite par ledit anticorps monoclonal BR96 est inhibée, et ladite molécule d'immunoglobuline modifiée étant sélectionnée parmi les anticorps des revendications 1-3.

10. Composition de la revendication 9, où ladite molécule d'immunoglobuline structurellement modifiée reconnaît une cible associée à un cancer et s'y lie.

11. Utilisation de la composition de la revendication 10 dans la fabrication d'un médicament conçu pour être employé dans une méthode de traitement de carcinomes in vivo, qui comprend l'administration d'une quantité pharmaceutiquement efficace de ladite composition à un sujet.

12. Utilisation selon la revendication 11, où la molécule d'immunoglobuline structurellement modifiée contenue dans la composition est marquée de façon produire, directement ou indirectement, un signal décelable au moyen d'un composé sélectionné dans le groupe consistant en un radiomarqueur, une enzyme, un chromophore, un agent chimioluminescent ou un agent fluorescent.

13. Utilisation de la composition de la revendication 10 dans la fabrication d'un médicament indiqué dans le traitement d'un patient souffrant d'un cancer, le cancer étant **caractérisé** comme un groupe de cellules qui présentent sur la surface des cellules un antigène associé aux tumeurs, par l'administration au sujet d'une quantité de la dite composition qui tue les cellules cancéreuses, la composition comprenant une immunoglobuline couplée à un agent cytotoxique dans des conditions qui permettent à la molécule ainsi couplée de se lier à l'antigène associé aux tumeurs à la surface des cellules cancéreuses de façon à tuer les cellules liées en traitant par ce biais le sujet et

14. Utilisation selon la revendication 13, où ladite immunoglobuline est conjuguée à la doxonibieine,

15. Utilisation d'une molécule d'immunoglobuline possédant une région variable et une région constante, la molécule d'immunoglobuline étant modifiée en changeant structurellement des domaines multiples associés à une toxicité du domaine CH2 de la région constante d'un anticorps monoclonal BR96 et lesdits domaines multiples associés à une toxicité étant modifiés de telle sorte que la toxicité sinon induite par ledit anticorps monoclonal BR96 est inhibée, dans la fabrication d'un médicament qui inhibe la toxicité induite par l'immunoglobuline en résultat d'une immunothérapie par l'immunoglobuline chez un sujet, où ladite molécule d'immunoglobuline modifiée est sélectionnée parmi les anticorps des revendications 1-3,

16. Utilisation selon la revendication 15, où ladite molécule structurellement modifiée n'intervient plus dans la médiation de la cytotoxicité dépendante du complément ou dans la cytotoxicité dépendante des anticorps.

17. Utilisation selon la revendication 15, où ladite molécule d'immunoglobuline reconnaît l'antigène Le^{y} et s'y lie.

18. Composition pharmaceutique selon la revendication 9, destinée à être utilisée comme agent thérapeutique.


### SEQUENCE LISTING

(1) GENERAL INFORMATION
   (i) APPLICANT: Bristol-Myers Squibb Co.
   (ii) TITLE OF THE INVENTION:
      A METHOD FOR INHIBITING
      IMMUNOGLOBULIN-INDUCED TOXICITY FROM THE USE OF IMMUNOGLOBULINS IN THERAPY AND IN VIVO DIAGNOSIS
   (iii) NUMBER OF SEQUENCES: 13
   (iv) CORRESPONDENCE ADDRESS:
      (A) ADDRESSEE: Merchant & Gould
      (B) STREET: 11150 Santa Monica Blvd., Suite 400
      (C) CITY: Los Angeles
      (D) STATE: CA
      (E) COUNTRY: USA
      (F) ZIP: 90025
   (v) COMPUTER READABLE FORM:
      (A) MEDIUM TYPE: Diskette
      (B) COMPUTER: IBM Compatible
      (C) OPERATING SYSTEM: DOS
      (D) SOFTWARE: FastSEQ Version 2.0
   (vi) CURRENT APPLICATION DATA:
      (A) APPLICATION NUMBER: PCT/US97/ .
      (B) FILING DATE: 01-AUG-1997
      (C) CLASSIFICATION:
   (vii) PRIOR APPLICATION DATA:
      (A) APPLICATION NUMBER: 60/023,033
      (B) FILING DATE: 02-AUG-1996
   (viii) ATTORNEY/AGENT INFORMATION:
      (A) NAME: Adriano, Sarah B
      (B) REGISTRATION NUMBER: 34,470
      (C) REFERENCE/DOCKET NUMBER: 30436.43WOU1
   (ix) TELECOMMUNICATION INFORMATION:
      (A) TELEPHONE: 310-445-1140
      (B) TELEFAX: 310-445-9031
      (C) TELEX:
(2) INFORMATION FOR SEQ ID NO:1:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:1:
      TGGCACCGAA AGCTTTCTGG GGCAGGCCAG GCCTGA 36
(2) INFORMATION FOR SEQ ID NO:2:
   (i) SEQUENCE CHARACTERISTICS:
      (A) LENGTH: 57 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:2:
      TCCGGACATG TTGGTACCCA CGTGGTGGTC GACGCTGAGC CTGGCTTCGA GCAGACA 57
(2) INFORMATION FOR SEQ ID NO:3:
   (i) SEQUENCE CHARACTERISTICS:
      (A) LENGTH: 55 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:3:
      GTCGACCACC ACGTGGGTAC CAACATGTCC GGAGCCACAT GGACAGAGGC CGGCT 55
(2) INFORMATION FOR SEQ ID NO:4:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:4:
      CTGGTTCTTG TTCATCTCCT CTCTAGATGG 30
(2) INFORMATION FOR SEQ ID NO:5:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:5:
      ACCATGGTCG ACCTCAGACC TGCCAAGAGC CATATC 36
(2) INFORMATION FOR SEQ ID NO:6:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:6:
      CATGGTCACG TGGTGTGTCC CTGGATGCAG GCTACTCTA 39
(2) INFORMATION FOR SEQ ID NO:7:
   (i) SEQUENCE CHARACTERISTICS:
      (A) LENGTH: 49 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:7:
      CAGGGAGGGA GGGTGTCTGC TGGAAGCCAG GCTCAGCGCT GACCTCAGA 49
(2) INFORMATION FOR SEQ ID NO:8:
   (i) SEQUENCE CHARACTERISTICS:
      (A) LENGTH: 50 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:8:
      GGAAAGAACC ATCACAGTCT CGCAGGGGCC CAGGGCAGCG CTGGGTGCTT 50
(2) INFORMATION FOR SEQ ID NO:9:
   (i) SEQUENCE CHARACTERISTICS:
      (A) LENGTH: 8691 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:9:
   GACGGATCGG GAGATCTGCT AGGTGACCTG AGGCGCGCCG GCTTCGAATA GCCAGAGTAA 60
   CCTTTTTTTT TAATTTTATT TTATTTTATT TTTGAGATGG AGTTTGGCGC CGATCTCCCG 120
   ATCCCCTATG GTCGACTCTC AGTACAATCT GCTCTGATGC CGCATAGTTA AGCCAGTATC 180
   TGCTCCCTGC TTGTGTGTTG GAGGTCGCTG AGTAGTGCGC GAGCAAAATT TAAGCTACAA 240
   CAAGGCAAGG CTTGACCGAC AATTGCATGA AGAATCTGCT TAGGGTTAGG CGTTTTGCGC 300
   TGCTTCGCGA TGTACGGGCC AGATATACGC GTTGACATTG ATTATTGACT AGTTATTAAT 360
   AGTAATCAAT TACGGGGTCA TTAGTTCATA GCCCATATAT GGAGTTCCGC GTTACATAAC 420
   TTACGGTAAA TGGCCCGCCT GGCTGACCGC CCAACGACCC CCGCCCATTG ACGTCAATAA 480
   TGACGTATGT TCCCATAGTA ACGCCAATAG GGACTTTCCA TTGACGTCAA TGGGTGGACT 540
   ATTTACGGTA AACTGCCCAC TTGGCAGTAC ATCAAGTGTA TCATATGCCA AGTACGCCCC 600
   CTATTGACGT CAATGACGGT AAATGGCCCG CCTGGCATTA TGCCCAGTAC ATGACCTTAT 660
   GGGACTTTCC TACTTGGCAG TACATCTACG TATTAGTCAT CGCTATTACC ATGGTGATGC 720
   GGTTTTGGCA GTACATCAAT GGGCGTGGAT AGCGGTTTGA CTCACGGGGA TTTCCAAGTC 780
   TCCACCCCAT TGACGTCAAT GGGAGTTTGT TTTGGCACCA AAATCAACGG GACTTTCCAA 840
   AATGTCGTAA CAACTCCGCC CCATTGACGC AAATGGGCGG TAGGCGTGTA CGGTGGGAGG 900
   TCTATATAAG CAGAGCTCTC TGGCTAACTA GAGAACCCAC TGCTTACTGG CTTATCGAAA 960
   TTAATACGAC TCACTATAGG GAGACCCAAG CTTGGTACCA ATTTAAATTG ATATCTCCTT 1020
   AGGTCTCGAG TCTCTAGATA ACCGGTCAAT CGATTGGAAT TCTTGCGGCC GCTTGCTAGC 1080
   CACCATGGAG TTGTGGTTAA GCTTGGTCCT TCCTTGTCCT TGTTTTAAAA GGTGTCCAGT 1140
   GTGAAGTGAA TCTGGTGGAG TCTGGGGGAG GCTTAGTGCA GCCTGGAGGG TCCCTGAAAG 1200
   TCTCCTGTGT AACCTCTGGA TTCACTTTCA GTGACTATTA CATGTATTGG GTTCGCCAGA 1260
   CTCCAGAGAA GAGGCTGGAG TGGGTCGCAT ACATTAGTCA AGGTGGTGAT ATAACCGACT 1320
   ATCCAGACAC TGTAAAGGGT CGATTCACCA TCTCCAGAGA CAATGCCAAG AACACCCTGT 1380
   ACCTGCAAAT GAGCCGTCTG AAGTCTGAGG ACACAGCCAT GTATTACTGT GCAAGAGGCC 1440
   TGGACGACGG GGCCTGGTTT GCTTACTGGG GCCAAGGGAC TCTGGTCACG GTCTCTGTAG 1500
   CTAGCACCAA GGGCCCATCG GTCTTCCCCC TGGCACCCTC CTCCAAGAGC ACCTCTGGGG 1560
   GCACAGCGGC CCTGGGCTGC CTGGTCAAGG ACTACTTCCC CGAACCGGTG ACGGTGTCGT 1620
   GGAACTCAGG CGCCCTGACC AGCGGCGTGC ACACCTTCCC GGCTGTCCTA CAGTCCTCAG 1680
   GACTCTACTC CCTCAGCAGC GTGGTCACCG TGCCCTCCAG CAGCTTGGGC ACCCAGACCT 1740
   ACATCTGCAA CGTGAATCAC AAGCCCAGCA ACACCAAGGT GGACAAGAAA GTTGGTGAGA 1800
   GGCCAGCACA GGGAGGGAGG GTGTCTGCTG GAAGCCAGGC TCAGCGCTCC TGCCTGGACG 1860
   CATCCCGGCT ATGCAGCCCC AGTCCAGGGC AGCAAGGCAG GCCCCGTCTG CCTCTTCACC 1920
   CGGAGGCCTC TGCCCGCCCC ACTCATGCTC AGGGAGAGGG TCTTCTGGCT TTTTCCCCAG 1980
   GCTCTGGGCA GGCACAGGCT AGGTGCCCCT AACCCAGGCC CTGCACACAA AGGGGCAGGT 2040
   GCTGGGCTCA GACCTGCCAA GAGCCATATC CGGGAGGACC CTGCCCCTGA CCTAAGCCCA 2100
   CCCCAAAGGC CAAACTCTCC ACTCCCTCAG CTCGGACACC TTCTCTCCTC CCAGATTCCA 2160
   GTAACTCCCA ATCTTCTCTC TGCAGAGCCC AAATCTTGTG ACAAAACTCA CACATGCCCA 2220
   CCGTGCCCAG GTAAGCCAGC CCAGGCCTCG CCCTCCAGCT CAAGGCGGGA CAGGTGCCCT 2280
   AGAGTAGCCT GCATCCAGGG ACAGGCCCCA GCCGGGTGCT GACACGTCCA CCTCCATCTC 2340
   TTCCTCAGCA CCTGAACTCC TGGGGGGACC GTCAGTCTTC CTCTTCCCCC CAAAACCCAA 2400
   GGACACCCTC ATGATCTCCC GGACCCCTGA GGTCACATGC GTGGTGGTGG ACGTGAGCCA 2460
   CGAAGACCCT GAGGTCAAGT TCAACTGGTA CGTGGACGGC GTGGAGGTGC ATAATGCCAA 2520
   GACAAAGCCG CGGGAGGAGC AGTACAACAG CACGTACCGT GTGGTCAGCG TCCTCACCGT 2580
   CCTGCACCAG GACTGGCTGA ATGGCAAGGA GTACAAGTGC AAGGTCTCCA ACAAAGCCCT 2640
   CCCAGCCCCC ATCGAGAAAA CCATCTCCAA AGCCAAAGGT GGGACCCGTG GGGTGCGAGG 2700
   GCCACATGGA CAGAGGCCGG CTCGGCCCAC CCTCTGCCCT GAGAGTGACC GCTGTACCAA 2760
   CCTCTGTCCC TACAGGGCAG CCCCGAGAAC CACAGGTGTA CACCCTGCCC CCATCCCGGG 2820
   ATGAGCTGAC CAAGAACCAG GTCAGCCTGA CCTGCCTGGT CAAAGGCTTC TATCCCAGCG 2880
   ACATCGCCGT GGAGTGGGAG AGCAATGGGC AGCCGGAGAA CAACTACAAG ACCACGCCTC 2940
   CCGTGCTGGA CTCCGACGGC TCCTTCTTCC TCTACAGCAA GCTCACCGTG GACAAGAGCA 3000
   GGTGGCAGCA GGGGAACGTC TTCTCATGCT CCGTGATGCA TGAGGCTCTG CACAACCACT 3060
   ACACGCAGAA GAGCCTCTCC CTGTCTCCGG GTAAATGAGT GCGACGGCCG GCAAGCCCCC 3120
   GCTCCCCGGG CTCTCGCGGT CGCACGAGGA TGCTTGGCAC GTACCCCCTG TACATACTTC 3180
   CCGGGCGCCC AGCATGGAAA TAAAGCACCC AGCGCTGCCC TGGGCCCCTG CGAGACTGTG 3240
   ATGGTTCTTT CCACGGGTCA GGCCGAGTCT GAGGCCTGAG TGGCATGAGG GAGGCAGAGC 3300
   GGGTCCCACT GTCCCCACAC TGGCCCAGGC TGTGCAGGTG TGCCTGGGCC CCCTAGGGTG 3360
   GGGCTCAGCC AGGGGCTGCC CTCGGCAGGG TGGGGGATTT GCCAGCGTGG CCCTCCCTCC 3420
   AGCAGCACCT GCCCTGGGCT GGGCCACGGG AAGCCCTAGG AGCCCCTGGG GACAGACACA 3480
   CAGCCCCTGC CTCTGTAGGA GACTGTCCTG TTCTGTGAGC GCCCCTGTCC TCCCGACCTC 3540
   CATGCCCACT CGGGGGCATG CCTAGTCCAT GTGCGTAGGG ACAGGCCCTC CCTCACCCAT 3600
   CTACCCCCAC GGCACTAACC CCTGGCTGCC CTGCCCAGCC TCGCACCCGC ATGGGGACAC 3660
   AACCGACTCC GGGGACATGC ACTCTCGGGC CCTGTGGAGG GACTGGTGCA GATGCCCACA 3720
   CACACACTCA GCCCAGACCC GTTCAACAAA CCCCGCACTG AGGTTGGCCG GCCACACGGC 3780
   CACCACACAC ACACGTGCAC GCCTCACACA CGGAGCCTCA CCCGGGCGAA CTGCACAGCA 3840
   CCCAGACCAG AGCAAGGTCC TCGCACACGT GAACACTCCT CGGACACAGG CCCCCACGAG 3900
   CCCCACGCGG CACCTCAAGG CCCACGAGCC TCTCGGCAGC TTCTCCACAT GCTGACCTGC 3960
   TCAGACAAAC CCAGCCCTCC TCTCACAAGG GTGCCCCTGC AGCCGCCACA CACACACAGG 4020
   GGATCACACA CCACGTCACG TCCCTGGCCC TGGCCCACTT CCCAGTGCCG CCCTTCCCTG 4080
   CAGGACGGAT CAGCCTCGAC TGTGCCTTCT AGTTGCCAGC CATCTGTTGT TTGCCCCTCC 4140
   CCCGTGCCTT CCTTGACCCT GGAAGGTGCC ACTCCCACTG TCCTTTCCTA ATAAAATGAG 4200
   GAAATTGCAT CGCATTGTCT GAGTAGGTGT CATTCTATTC TGGGGGGTGG GGTGGGGCAG 4260
   GACAGCAAGG GGGAGGATTG GGAAGACAAT AGCAGGCATG CTGGGGATGC GGTGGGCTCT 4320
   ATGGCTTCTG AGGCGGAAAG AACCAGCTGG GGCTCTAGGG GGTATCCCCA CGCGCCCTGT 4380
   AGCGGCGCAT TAAGCGCGGC GGGTGTGGTG GTTACGCGCA GCGTGACCGC TACACTTGCC 4440
   AGCGCCCTAG CGCCCGCTCC TTTCGCTTTC TTCCCTTCCT TTCTCGCCAC GTTCGCCGGG 4500
   CCTCTCAAAA AAGGGAAAAA AAGCATGCAT CTCAATTAGT CAGCAACCAT AGTCCCGCCC 4560
   CTAACTCCGC CCATCCCGCC CCTAACTCCG CCCAGTTCCG CCCATTCTCC GCCCCATGGC 4620
   TGACTAATTT TTTTTATTTA TGCAGAGGCC GAGGCCGCCT CGGCCTCTGA GCTATTCCAG 4680
   AAGTAGTGAG GAGGCTTTTT TGGAGGCCTA GGCTTTTGCA AAAAGCTTGG ACAGCTCAGG 4740
   GCTGCGATTT CGCGCCAAAC TTGACGGCAA TCCTAGCGTG AAGGCTGGTA GGATTTTATC 4800
   CCCGCTGCCA TCATGGTTCG ACCATTGAAC TGCATCGTCG CCGTGTCCCA AAATATGGGG 4860
   ATTGGCAAGA ACGGAGACCT ACCCTGGCCT CCGCTCAGGA ACGAGTTCAA GTACTTCCAA 4920
   AGAATGACCA CAACCTCTTC AGTGGAAGGT AAACAGAATC TGGTGATTAT GGGTAGGAAA 4980
   ACCTGGTTCT CCATTCCTGA GAAGAATCGA CCTTTAAAGG ACAGAATTAA TATAGTTCTC 5040
   AGTAGAGAAC TCAAAGAACC ACCACGAGGA GCTCATTTTC TTGCCAAAAG TTTGGATGAT 5100
   GCCTTAAGAC TTATTGAACA ACCGGAATTG GCAAGTAAAG TAGACATGGT TTGGATAGTC 5160
   GGAGGCAGTT CTGTTTACCA GGAAGCCATG AATCAACCAG GCCACCTTAG ACTCTTTGTG 5220
   ACAAGGATCA TGCAGGAATT TGAAAGTGAC ACGTTTTTCC CAGAAATTGA TTTGGGGAAA 5280
   TATAAACTTC TCCCAGAATA CCCAGGCGTC CTCTCTGAGG TCCAGGAGGA AAAAGGCATC 5340
   AAGTATAAGT TTGAAGTCTA CGAGAAGAAA GACTAACAGG AAGATGCTTT CAAGTTCTCT 5400
   GCTCCCCTCC TAAAGCTATG CATTTTTATA AGACCATGGG ACTTTTGCTG GCTTTAGATC 5460
   TCTTTGTGAA GGAACCTTAC TTCTGTGGTG TGACATAATT GGACAAACTA CCTACAGAGA 5520
   TTTAAAGCTC TAAGGTAAAT ATAAAATTTT TAAGTGTATA ATGTGTTAAA CTACTGATTC 5580
   TAATTGTTTG TGTATTTTAG ATTCCAACCT ATGGAACTGA TGAATGGGAG CAGTGGTGGA 5640
   ATGCCTTTAA TGAGGAAAAC CTGTTTTGCT CAGAAGAAAT GCCATCTAGT GATGATGAGG 5700
   CTACTGCTGA CTCTCAACAT TCTACTCCTC CAAAAAAGAA GAGAAAGGTA GAAGACCCCA 5760
   AGGACTTTCC TTCAGAATTG CTAAGTTTTT TGAGTCATGC TGTGTTTAGT AATAGAACTC 5820
   TTGCTTGCTT TGCTATTTAC ACCACAAAGG AAAAAGCTGC ACTGCTATAC AAGAAAATTA 5880
   TGGAAAAATA TTCTGTAACC TTTATAAGTA GGCATAACAG TTATAATCAT AACATACTGT 5940
   TTTTTCTTAC TCCACACAGG CATAGAGTGT CTGCTATTAA TAACTATGCT CAAAAATTGT 6000
   GTACCTTTAG CTTTTTAATT TGTAAAGGGG TTAATAAGGA ATATTTGATG TATAGTGCCT 6060
   TGACTAGAGA TCATAATCAG CCATACCACA TTTGTAGAGG TTTTACTTGC TTTAAAAAAC 6120
   CTCCCACACC TCCCCCTGAA CCTGAAACAT AAAATGAATG CAATTGTTGT TGTTAACTTG 6180
   TTTATTGCAG CTTATAATGG TTACAAATAA AGCAATAGCA TCACAAATTT CACAAATAAA 6240
   GCATTTTTTT CACTGCATTC TAGTTGTGGT TTGTCCAAAC TCATCAATGT ATCTTATCAT 6300
   GTCTGGATCG GCTGGATGAT CCTCCAGCGC GGGGATCTCA TGCTGGAGTT CTTCGCCCAC 6360
   CCCAACTTGT TTATTGCAGC TTATAATGGT TACAAATAAA GCAATAGCAT CACAAATTTC 6420
   ACAAATAAAG CATTTTTTTC ACTGCATTCT AGTTGTGGTT TGTCCAAACT CATCAATGTA 6480
   TCTTATCATG TCTGTATACC GTCGACCTCT AGCTAGAGCT TGGCGTAATC ATGGTCATAG 6540
   CTGTTTCCTG TGTGAAATTG TTATCCGCTC ACAATTCCAC ACAACATACG AGCCGGAAGC 6600
   ATAAAGTGTA AAGCCTGGGG TGCCTAATGA GTGAGCTAAC TCACATTAAT TGCGTTGCGC 6660
   TCACTGCCCG CTTTCCAGTC GGGAAACCTG TCGTGCCAGC TGCATTAATG AATCGGCCAA 6720
   CGCGCGGGGA GAGGCGGTTT GCGTATTGGG CGCTCTTCCG CTTCCTCGCT CACTGACTCG 6780
   CTGCGCTCGG TCGTTCGGCT GCGGCGAGCG GTATCAGCTC ACTCAAAGGC GGTAATACGG 6840
   TTATCCACAG AATCAGGGGA TAACGCAGGA AAGAACATGT GAGCAAAAGG CCAGCAAAAG 6900
   GCCAGGAACC GTAAAAAGGC CGCGTTGCTG GCGTTTTTCC ATAGGCTCCG CCCCCCTGAC 6960
   GAGCATCACA AAAATCGACG CTCAAGTCAG AGGTGGCGAA ACCCGACAGG ACTATAAAGA 7020
   TACCAGGCGT TTCCCCCTGG AAGCTCCCTC GTGCGCTCTC CTGTTCCGAC CCTGCCGCTT 7080
   ACCGGATACC TGTCCGCCTT TCTCCCTTCG GGAAGCGTGG CGCTTTCTCA ATGCTCACGC 7140
   TGTAGGTATC TCAGTTCGGT GTAGGTCGTT CGCTCCAAGC TGGGCTGTGT GCACGAACCC 7200
   CCCGTTCAGC CCGACCGCTG CGCCTTATCC GGTAACTATC GTCTTGAGTC CAACCCGGTA   7260
   AGACACGACT TATCGCCACT GGCAGCAGCC ACTGGTAACA GGATTAGCAG AGCGAGGTAT   7320
   GTAGGCGGTG CTACAGAGTT CTTGAAGTGG TGGCCTAACT ACGGCTACAC TAGAAGGACA   7380
   GTATTTGGTA TCTGCGCTCT GCTGAAGCCA GTTACCTTCG GAAAAAGAGT TGGTAGCTCT   7440
   TGATCCGGCA AACAAACCAC CGCTGGTAGC GGTGGTTTTT TTGTTTGCAA GCAGCAGATT   7500
   ACGCGCAGAA AAAAAGGATC TCAAGAAGAT CCTTTGATCT TTTCTACGGG GTCTGACGCT   7560
   CAGTGGAACG AAAACTCACG TTAAGGGATT TTGGTCATGA GATTATCAAA AAGGATCTTC   7620
   ACCTAGATCC TTTTAAATTA AAAATGAAGT TTTAAATCAA TCTAAAGTAT ATATGAGTAA   7680
   ACTTGGTCTG ACAGTTACCA ATGCTTAATC AGTGAGGCAC CTATCTCAGC GATCTGTCTA   7740
   TTTCGTTCAT CCATAGTTGC CTGACTCCCC GTCGTGTAGA TAACTACGAT ACGGGAGGGC   7800
   TTACCATCTG GCCCCAGTGC TGCAATGATA CCGCGAGACC CACGCTCACC GGCTCCAGAT   7860
   TTATCAGCAA TAAACCAGCC AGCCGGAAGG GCCGAGCGCA GAAGTGGTCC TGCAACTTTA   7920
   TCCGCCTCCA TCCAGTCTAT TAATTGTTGC CGGGAAGCTA GAGTAAGTAG TTCGCCAGTT   7980
   AATAGTTTGC GCAACGTTGT TGCCATTGCT ACAGGCATCG TGGTGTCACG CTCGTCGTTT   8040
   GGTATGGCTT CATTCAGCTC CGGTTCCCAA CGATCAAGGC GAGTTACATG ATCCCCCATG   8100
   TTGTGCAAAA AAGCGGTTAG CTCCTTCGGT CCTCCGATCG TTGTCAGAAG TAAGTTGGCC   8160
   GCAGTGTTAT CACTCATGGT TATGGCAGCA CTGCATAATT CTCTTACTGT CATGCCATCC   8220
   GTAAGATGCT TTTCTGTGAC TGGTGAGTAC TCAACCAAGT CATTCTGAGA ATAGTGTATG   8280
   CGGCGACCGA GTTGCTCTTG CCCGGCGTCA ATACGGGATA ATACCGCGCC ACATAGCAGA   8340
   ACTTTAAAAG TGCTCATCAT TGGAAAACGT TCTTCGGGGC GAAAACTCTC AAGGATCTTA   8400
   CCGCTGTTGA GATCCAGTTC GATGTAACCC ACTCGTGCAC CCAACTGATC TTCAGCATCT   8460
   TTTACTTTCA CCAGCGTTTC TGGGTGAGCA AAAACAGGAA GGCAAAATGC CGCAAAAAAG   8520
   GGAATAAGGG CGACACGGAA ATGTTGAATA CTCATACTCT TCCTTTTTCA ATATTATTGA   8580
   AGCATTTATC AGGGTTATTG TCTCATGAGC GGATACATAT TTGAATGTAT TTAGAAAAAT   8640
   AAACAAATAG GGGTTCCGCG CACATTTCCC CGAAAAGTGC CACCTGACGT C            8691
(2) INFORMATION FOR SEQ ID NO:10:
   (i) SEQUENCE CHARACTERISTICS:
      (A) LENGTH: 8327 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:10:
   GACGGATCGG GAGATCTGCT AGGTGACCTG AGGCGCGCCG GCTTCGAATA GCCAGAGTAA      60
   CCTTTTTTTT TAATTTTATT TTATTTTATT TTTGAGATGG AGTTTGGCGC CGATCTCCCG     120
   ATCCCCTATG GTCGACTCTC AGTACAATCT GCTCTGATGC CGCATAGTTA AGCCAGTATC     180
   TGCTCCCTGC TTGTGTGTTG GAGGTCGCTG AGTAGTGCGC GAGCAAAATT TAAGCTACAA     240
   CAAGGCAAGG CTTGACCGAC AATTGCATGA AGAATCTGCT TAGGGTTAGG CGTTTTGCGC     300
   TGCTTCGCGA TGTACGGGCC AGATATACGC GTTGACATTG ATTATTGACT AGTTATTAAT     360
   AGTAATCAAT TACGGGGTCA TTAGTTCATA GCCCATATAT GGAGTTCCGC GTTACATAAC     420
   TTACGGTAAA TGGCCCGCCT GGCTGACCGC CCAACGACCC CCGCCCATTG ACGTCAATAA     480
   TGACGTATGT TCCCATAGTA ACGCCAATAG GGACTTTCCA TTGACGTCAA TGGGTGGACT     540
   ATTTACGGTA AACTGCCCAC TTGGCAGTAC ATCAAGTGTA TCATATGCCA AGTACGCCCC     600
   CTATTGACGT CAATGACGGT AAATGGCCCG CCTGGCATTA TGCCCAGTAC ATGACCTTAT     660
   GGGACTTTCC TACTTGGCAG TACATCTACG TATTAGTCAT CGCTATTACC ATGGTGATGC     720
   GGTTTTGGCA GTACATCAAT GGGCGTGGAT AGCGGTTTGA CTCACGGGGA TTTCCAAGTC     780
   TCCACCCCAT TGACGTCAAT GGGAGTTTGT TTTGGCACCA AAATCAACGG GACTTTCCAA     840
   AATGTCGTAA CAACTCCGCC CCATTGACGC AAATGGGCGG TAGGCGTGTA CGGTGGGAGG     900
   TCTATATAAG CAGAGCTCTC TGGCTAACTA GAGAACCCAC TGCTTACTGG CTTATCGAAA     960
   TTAATACGAC TCACTATAGG GAGACCCAAG CTTGGTACCA ATTTAAATTG ATATCTCCTT    1020
   AGGTCTCGAG TCTCTAGATA ACCGGTCAAT CGATTGGAAT TCTTGCGGCC GCTTGCTAGC    1080
   CACCATGGAG TTGTGGTTAA GCTTGGTCCT TCCTTGTCCT TGTTTTAAAA GGTGTCCAGT    1140
   GTGAAGTGAA TCTGGTGGAG TCTGGGGGAG GCTTAGTGCA GCCTGGAGGG TCCCTGAAAG    1200
   TCTCCTGTGT AACCTCTGGA TTCACTTTCA GTGACTATTA CATGTATTGG GTTCGCCAGA    1260
   CTCCAGAGAA GAGGCTGGAG TGGGTCGCAT ACATTAGTCA AGGTGGTGAT ATAACCGACT   1320
   ATCCAGACAC TGTAAAGGGT CGATTCACCA TCTCCAGAGA CAATGCCAAG AACACCCTGT   1380
   ACCTGCAAAT GAGCCGTCTG AAGTCTGAGG ACACAGCCAT GTATTACTGT GCAAGAGGCC   1440
   TGGACGACGG GGCCTGGTTT GCTTACTGGG GCCAAGGGAC TCTGGTCACG GTCTCTGTAG   1500
   CTAGCACCAA GGGCCCATCG GTCTTCCCCC TGGCACCCTC CTCCAAGAGC ACCTCTGGGG   1560
   GCACAGCGGC CCTGGGCTGC CTGGTCAAGG ACTACTTCCC CGAACCGGTG ACGGTGTCGT   1620
   GGAACTCAGG CGCCCTGACC AGCGGCGTGC ACACCTTCCC GGCTGTCCTA CAGTCCTCAG   1680
   GACTCTACTC CCTCAGCAGC GTGGTCACCG TGCCCTCCAG CAGCTTGGGC ACCCAGACCT   1740
   ACATCTGCAA CGTGAATCAC AAGCCCAGCA ACACCAAGGT GGACAAGAAA GTTGGTGAGA   1800
   GGCCAGCACA GGGAGGGAGG GTGTCTGCTG GAAGCCAGGC TCAGCGCTCC TGCCTGGACG   1860
   CATCCCGGCT ATGCAGCCCC AGTCCAGGGC AGCAAGGCAG GCCCCGTCTG CCTCTTCACC   1920
   CGGAGGCCTC TGCCCGCCCC ACTCATGCTC AGGGAGAGGG TCTTCTGGCT TTTTCCCCAG   1980
   GCTCTGGGCA GGCACAGGCT AGGTGCCCCT AACCCAGGCC CTGCACACAA AGGGGCAGGT   2040
   GCTGGGCTCA GACCTGCCAA GAGCCATATC CGGGAGGACC CTGCCCCTGA CCTAAGCCCA   2100
   CCCCAAAGGC CAAACTCTCC ACTCCCTCAG CTCGGACACC TTCTCTCCTC CCAGATTCCA   2160
   GTAACTCCCA ATCTTCTCTC TGCAGAGCCC AAATCTTGTG ACAAAACTCA CACATGCCCA   2220
   CCGTGCCCAG GTAAGCCAGC CCAGGCCTCG CCCTCCAGCT CAAGGCGGGA CAGGTGCCCT   2280
   AGAGTAGCCT GCATCCAGGG ACACACCACG TGGGTACCAA CATGTCCGGA GCCACATGGA   2340
   CAGAGGCCGG CTCGGCCCAC CCTCTGCCCT GAGAGTGACC GCTGTACCAA CCTCTGTCCC   2400
   TACAGGGCAG CCCCGAGAAC CACAGGTGTA CACCCTGCCC CCATCCCGGG ATGAGCTGAC   2460
   CAAGAACCAG GTCAGCCTGA CCTGCCTGGT CAAAGGCTTC TATCCCAGCG ACATCGCCGT   2520
   GGAGTGGGAG AGCAATGGGC AGCCGGAGAA CAACTACAAG ACCACGCCTC CCGTGCTGGA   2580
   CTCCGACGGC TCCTTCTTCC TCTACAGCAA GCTCACCGTG GACAAGAGCA GGTGGCAGCA   2640
   GGGGAACGTC TTCTCATGCT CCGTGATGCA TGAGGCTCTG CACAACCACT ACACGCAGAA   2700
   GAGCCTCTCC CTGTCTCCGG GTAAATGAGT GCGACGGCCG GCAAGCCCCC GCTCCCCGGG   2760
   CTCTCGCGGT CGCACGAGGA TGCTTGGCAC GTACCCCCTG TACATACTTC CCGGGCGCCC   2820
   AGCATGGAAA TAAAGCACCC AGCGCTGCCC TGGGCCCCTG CGAGACTGTG ATGGTTCTTT   2880
   CCACGGGTCA GGCCGAGTCT GAGGCCTGAG TGGCATGAGG GAGGCAGAGC GGGTCCCACT   2940
   GTCCCCACAC TGGCCCAGGC TGTGCAGGTG TGCCTGGGCC CCCTAGGGTG GGGCTCAGCC   3000
   AGGGGCTGCC CTCGGCAGGG TGGGGGATTT GCCAGCGTGG CCCTCCCTCC AGCAGCACCT   3060
   GCCCTGGGCT GGGCCACGGG AAGCCCTAGG AGCCCCTGGG GACAGACACA CAGCCCCTGC   3120
   CTCTGTAGGA GACTGTCCTG TTCTGTGAGC GCCCCTGTCC TCCCGACCTC CATGCCCACT   3180
   CGGGGGCATG CCTAGTCCAT GTGCGTAGGG ACAGGCCCTC CCTCACCCAT CTACCCCCAC   3240
   GGCACTAACC CCTGGCTGCC CTGCCCAGCC TCGCACCCGC ATGGGGACAC AACCGACTCC   3300
   GGGGACATGC ACTCTCGGGC CCTGTGGAGG GACTGGTGCA GATGCCCACA CACACACTCA   3360
   GCCCAGACCC GTTCAACAAA CCCCGCACTG AGGTTGGCCG GCCACACGGC CACCACACAC   3420
   ACACGTGCAC GCCTCACACA CGGAGCCTCA CCCGGGCGAA CTGCACAGCA CCCAGACCAG   3480
   AGCAAGGTCC TCGCACACGT GAACACTCCT CGGACACAGG CCCCCACGAG CCCCACGCGG   3540
   CACCTCAAGG CCCACGAGCC TCTCGGCAGC TTCTCCACAT GCTGACCTGC TCAGACAAAC   3600
   CCAGCCCTCC TCTCACAAGG GTGCCCCTGC AGCCGCCACA CACACACAGG GGATCACACA   3660
   CCACGTCACG TCCCTGGCCC TGGCCCACTT CCCAGTGCCG CCCTTCCCTG CAGGACGGAT   3720
   CAGCCTCGAC TGTGCCTTCT AGTTGCCAGC CATCTGTTGT TTGCCCCTCC CCCGTGCCTT   3780
   CCTTGACCCT GGAAGGTGCC ACTCCCACTG TCCTTTCCTA ATAAAATGAG GAAATTGCAT   3840
   CGCATTGTCT GAGTAGGTGT CATTCTATTC TGGGGGGTGG GGTGGGGCAG GACAGCAAGG   3900
   GGGAGGATTG GGAAGACAAT AGCAGGCATG CTGGGGATGC GGTGGGCTCT ATGGCTTCTG   3960
   AGGCGGAAAG AACCAGCTGG GGCTCTAGGG GGTATCCCCA CGCGCCCTGT AGCGGCGCAT   4020
   TAAGCGCGGC GGGTGTGGTG GTTACGCGCA GCGTGACCGC TACACTTGCC AGCGCCCTAG   4080
   CGCCCGCTCC TTTCGCTTTC TTCCCTTCCT TTCTCGCCAC GTTCGCCGGG CCTCTCAAAA   4140
   AAGGGAAAAA AAGCATGCAT CTCAATTAGT CAGCAACCAT AGTCCCGCCC CTAACTCCGC   4200
   CCATCCCGCC CCTAACTCCG CCCAGTTCCG CCCATTCTCC GCCCCATGGC TGACTAATTT   4260
   TTTTTATTTA TGCAGAGGCC GAGGCCGCCT CGGCCTCTGA GCTATTCCAG AAGTAGTGAG   4320
   GAGGCTTTTT TGGAGGCCTA GGCTTTTGCA AAAAGCTTGG ACAGCTCAGG GCTGCGATTT   4380
   CGCGCCAAAC TTGACGGCAA TCCTAGCGTG AAGGCTGGTA GGATTTTATC CCCGCTGCCA   4440
   TCATGGTTCG ACCATTGAAC TGCATCGTCG CCGTGTCCCA AAATATGGGG ATTGGCAAGA   4500
   ACGGAGACCT ACCCTGGCCT CCGCTCAGGA ACGAGTTCAA GTACTTCCAA AGAATGACCA   4560
   CAACCTCTTC AGTGGAAGGT AAACAGAATC TGGTGATTAT GGGTAGGAAA ACCTGGTTCT   4620
   CCATTCCTGA GAAGAATCGA CCTTTAAAGG ACAGAATTAA TATAGTTCTC AGTAGAGAAC   4680
   TCAAAGAACC ACCACGAGGA GCTCATTTTC TTGCCAAAAG TTTGGATGAT GCCTTAAGAC   4740
   TTATTGAACA ACCGGAATTG GCAAGTAAAG TAGACATGGT TTGGATAGTC GGAGGCAGTT   4800
   CTGTTTACCA GGAAGCCATG AATCAACCAG GCCACCTTAG ACTCTTTGTG ACAAGGATCA 4860
   TGCAGGAATT TGAAAGTGAC ACGTTTTTCC CAGAAATTGA TTTGGGGAAA TATAAACTTC 4920
   TCCCAGAATA CCCAGGCGTC CTCTCTGAGG TCCAGGAGGA AAAAGGCATC AAGTATAAGT 4980
   TTGAAGTCTA CGAGAAGAAA GACTAACAGG AAGATGCTTT CAAGTTCTCT GCTCCCCTCC 5040
   TAAAGCTATG CATTTTTATA AGACCATGGG ACTTTTGCTG GCTTTAGATC TCTTTGTGAA 5100
   GGAACCTTAC TTCTGTGGTG TGACATAATT GGACAAACTA CCTACAGAGA TTTAAAGCTC 5160
   TAAGGTAAAT ATAAAATTTT TAAGTGTATA ATGTGTTAAA CTACTGATTC TAATTGTTTG 5220
   TGTATTTTAG ATTCCAACCT ATGGAACTGA TGAATGGGAG CAGTGGTGGA ATGCCTTTAA 5280
   TGAGGAAAAC CTGTTTTGCT CAGAAGAAAT GCCATCTAGT GATGATGAGG CTACTGCTGA 5340
   CTCTCAACAT TCTACTCCTC CAAAAAAGAA GAGAAAGGTA GAAGACCCCA AGGACTTTCC 5400
   TTCAGAATTG CTAAGTTTTT TGAGTCATGC TGTGTTTAGT AATAGAACTC TTGCTTGCTT 5460
   TGCTATTTAC ACCACAAAGG AAAAAGCTGC ACTGCTATAC AAGAAAATTA TGGAAAAATA 5520
   TTCTGTAACC TTTATAAGTA GGCATAACAG TTATAATCAT AACATACTGT TTTTTCTTAC 5580
   TCCACACAGG CATAGAGTGT CTGCTATTAA TAACTATGCT CAAAAATTGT GTACCTTTAG 5640
   CTTTTTAATT TGTAAAGGGG TTAATAAGGA ATATTTGATG TATAGTGCCT TGACTAGAGA 5700
   TCATAATCAG CCATACCACA TTTGTAGAGG TTTTACTTGC TTTAAAAAAC CTCCCACACC 5760
   TCCCCCTGAA CCTGAAACAT AAAATGAATG CAATTGTTGT TGTTAACTTG TTTATTGCAG 5820
   CTTATAATGG TTACAAATAA AGCAATAGCA TCACAAATTT CACAAATAAA GCATTTTTTT 5880
   CACTGCATTC TAGTTGTGGT TTGTCCAAAC TCATCAATGT ATCTTATCAT GTCTGGATCG 5940
   GCTGGATGAT CCTCCAGCGC GGGGATCTCA TGCTGGAGTT CTTCGCCCAC CCCAACTTGT 6000
   TTATTGCAGC TTATAATGGT TACAAATAAA GCAATAGCAT CACAAATTTC ACAAATAAAG 6060
   CATTTTTTTC ACTGCATTCT AGTTGTGGTT TGTCCAAACT CATCAATGTA TCTTATCATG 6120
   TCTGTATACC GTCGACCTCT AGCTAGAGCT TGGCGTAATC ATGGTCATAG CTGTTTCCTG 6180
   TGTGAAATTG TTATCCGCTC ACAATTCCAC ACAACATACG AGCCGGAAGC ATAAAGTGTA 6240
   AAGCCTGGGG TGCCTAATGA GTGAGCTAAC TCACATTAAT TGCGTTGCGC TCACTGCCCG 6300
   CTTTCCAGTC GGGAAACCTG TCGTGCCAGC TGCATTAATG AATCGGCCAA CGCGCGGGGA 6360
   GAGGCGGTTT GCGTATTGGG CGCTCTTCCG CTTCCTCGCT CACTGACTCG CTGCGCTCGG 6420
   TCGTTCGGCT GCGGCGAGCG GTATCAGCTC ACTCAAAGGC GGTAATACGG TTATCCACAG 6480
   AATCAGGGGA TAACGCAGGA AAGAACATGT GAGCAAAAGG CCAGCAAAAG GCCAGGAACC 6540
   GTAAAAAGGC CGCGTTGCTG GCGTTTTTCC ATAGGCTCCG CCCCCCTGAC GAGCATCACA 6600
   AAAATCGACG CTCAAGTCAG AGGTGGCGAA ACCCGACAGG ACTATAAAGA TACCAGGCGT 6660
   TTCCCCCTGG AAGCTCCCTC GTGCGCTCTC CTGTTCCGAC CCTGCCGCTT ACCGGATACC 6720
   TGTCCGCCTT TCTCCCTTCG GGAAGCGTGG CGCTTTCTCA ATGCTCACGC TGTAGGTATC 6780
   TCAGTTCGGT GTAGGTCGTT CGCTCCAAGC TGGGCTGTGT GCACGAACCC CCCGTTCAGC 6840
   CCGACCGCTG CGCCTTATCC GGTAACTATC GTCTTGAGTC CAACCCGGTA AGACACGACT 6900
   TATCGCCACT GGCAGCAGCC ACTGGTAACA GGATTAGCAG AGCGAGGTAT GTAGGCGGTG 6960
   CTACAGAGTT CTTGAAGTGG TGGCCTAACT ACGGCTACAC TAGAAGGACA GTATTTGGTA 7020
   TCTGCGCTCT GCTGAAGCCA GTTACCTTCG GAAAAAGAGT TGGTAGCTCT TGATCCGGCA 7080
   AACAAACCAC CGCTGGTAGC GGTGGTTTTT TTGTTTGCAA GCAGCAGATT ACGCGCAGAA 7140
   AAAAAGGATC TCAAGAAGAT CCTTTGATCT TTTCTACGGG GTCTGACGCT CAGTGGAACG 7200
   AAAACTCACG TTAAGGGATT TTGGTCATGA GATTATCAAA AAGGATCTTC ACCTAGATCC 7260
   TTTTAAATTA AAAATGAAGT TTTAAATCAA TCTAAAGTAT ATATGAGTAA ACTTGGTCTG 7320
   ACAGTTACCA ATGCTTAATC AGTGAGGCAC CTATCTCAGC GATCTGTCTA TTTCGTTCAT 7380
   CCATAGTTGC CTGACTCCCC GTCGTGTAGA TAACTACGAT ACGGGAGGGC TTACCATCTG 7440
   GCCCCAGTGC TGCAATGATA CCGCGAGACC CACGCTCACC GGCTCCAGAT TTATCAGCAA 7500
   TAAACCAGCC AGCCGGAAGG GCCGAGCGCA GAAGTGGTCC TGCAACTTTA TCCGCCTCCA 7560
   TCCAGTCTAT TAATTGTTGC CGGGAAGCTA GAGTAAGTAG TTCGCCAGTT AATAGTTTGC 7620
   GCAACGTTGT TGCCATTGCT ACAGGCATCG TGGTGTCACG CTCGTCGTTT GGTATGGCTT 7680
   CATTCAGCTC CGGTTCCCAA CGATCAAGGC GAGTTACATG ATCCCCCATG TTGTGCAAAA 7740
   AAGCGGTTAG CTCCTTCGGT CCTCCGATCG TTGTCAGAAG TAAGTTGGCC GCAGTGTTAT 7800
   CACTCATGGT TATGGCAGCA CTGCATAATT CTCTTACTGT CATGCCATCC GTAAGATGCT 7860
   TTTCTGTGAC TGGTGAGTAC TCAACCAAGT CATTCTGAGA ATAGTGTATG CGGCGACCGA 7920
   GTTGCTCTTG CCCGGCGTCA ATACGGGATA ATACCGCGCC ACATAGCAGA ACTTTAAAAG 7980
   TGCTCATCAT TGGAAAACGT TCTTCGGGGC GAAAACTCTC AAGGATCTTA CCGCTGTTGA 8040
   GATCCAGTTC GATGTAACCC ACTCGTGCAC CCAACTGATC TTCAGCATCT TTTACTTTCA 8100
   CCAGCGTTTC TGGGTGAGCA AAAACAGGAA GGCAAAATGC CGCAAAAAAG GGAATAAGGG 8160
   CGACACGGAA ATGTTGAATA CTCATACTCT TCCTTTTTCA ATATTATTGA AGCATTTATC 8220
   AGGGTTATTG TCTCATGAGC GGATACATAT TTGAATGTAT TTAGAAAAAT AAACAAATAG 8280
   GGGTTCCGCG CACATTTCCC CGAAAAGTGC CACCTGACGT CCBRAAG 8327
(2) INFORMATION FOR SEQ ID NO:11:
   (i) SEQUENCE CHARACTERISTICS:
      (A) LENGTH: 8897 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:11:
   GGTACCAATT TAAATTGATA TCTCCTTAGG TCTCGAGCAC CATGAAGTTG CCTGTTAGGC     60
   TGTTGGTGCT GATGTTCTGG ATTCCTGCTT CCAGCAGTGA TGTTTTGATG ACCCAAATTC    120
   CAGTCTCCCT GCCTGTCAGT CTTGGAGATC AAGCGTCCAT CTCTTGCAGA TCTAGTCAGA    180
   TCATTGTACA TAATAATGGC AACACCTATT TAGAATGGTA CCTGCAGAAA CCAGGCCAGT    240
   CTCCACAGCT CCTGATCTAC AAAGTTTCCA ACCGATTTTC TGGGGTCCCA GACAGGTTCA    300
   GCGGCAGTGG ATCAGGGACA GATTTCACAC TCAAGATCAG CAGAGTGGAG GCTGAGGATC    360
   TGGGAGTTTA TTACTGCTTT CAAGGTTCAC ATGTTCCATT CACGTTCGGC TCGGGGACAA    420
   AGTTGGAAAT AAAACGTAAG TCTCGAGTCT CTAGATAACC GGTCAATCGA TTGGAATTCT    480
   AAACTCTGAG GGGGTCGGAT GACGTGGCCA TTCTTTGCCT AAAGCATTGA GTTTACTGCA    540
   AGGTCAGAAA AGCATGCAAA GCCCTCAGAA TGGCTGCAAA GAGCTCCAAC AAAACAATTT    600
   AGAACTTTAT TAAGGAATAG GGGGAAGCTA GGAAGAAACT CAAAACATCA AGATTTTAAA    660
   TACGCTTCTT GGTCTCCTTG CTATAATTAT CTGGGATAAG CATGCTGTTT TCTGTCTGTC    720
   CCTAACATGC CCTTATCCGC AAACAACACA CCCAAGGGCA GAACTTTGTT ACTTAAACAC    780
   CATCCTGTTT GCTTCTTTCC TCAGGAACTG TGGCTGCACC ATCTGTCTTC ATCTTCCCGC    840
   CATCTGATGA GCAGTTGAAA TCTGGAACTG CCTCTGTTGT GTGCCTGCTG AATAACTTCT    900
   ATCCCAGAGA GGCCAAAGTA CAGTGGAAGG TGGATAACGC CCTCCAATCG GGTAACTCCC    960
   AGGAGAGTGT CACAGAGCAG GAGAGCAAGG ACAGCACCTA CAGCCTCAGC AGCACCCTGA   1020
   CGCTGAGCAA AGCAGACTAC GAGAAACACA AAGTCTACGC CTGCGAAGTC ACCCATCAGG   1080
   GCCTGAGCTC GCCCGTCACA AAGAGCTTCA ACAGGGGAGA GTGTTAGAGG GAGAAGTGCC   1140
   CCCACCTGCT CCTCAGTTCC AGCCTGACCC CCTCCCATCC TTTGGCCTCT GACCCTTTTT   1200
   CCACAGGGGA CCTACCCCTA TTGCGGTCCT CCAGCTCATC TTTCACCTCA CCCCCCTCCT   1260
   CCTCCTTGGC TTTAATTATG CTAATGTTGG AGGAGAATGA ATAAATAAAG TGAATCTTTG   1320
   CACCTGTGGT TTCTCTCTTT CCTCATTTAA TAATTATTAT CTGTTGTTTT ACCAACTACT   1380
   CAATTTCTCT TATAAGGGAC TAAATATGTA GTCATCCTAA GGCACGTAAC CATTTATAAA   1440
   AATCATCCTT CATTCTATTT TACCCTATCA TCCTCTGCAA GACAGTCCTC CCTCAAACCC   1500
   ACAAGCCTTC TGTCCTCACA GTCCCCTGGG CCATGGTAGG AGAGACTTGC TTCCTTGTTT   1560
   TCCCCTCCTC AGCAAGCCCT CATAGTCCTT TTTAAGGGTG ACAGGTCTTA CAGTCATATA   1620
   TCCTTTGATT CAATTCCCTG AGAATCAACC AAAGCAAATT TTTCAAAAGA AGAAACCTGC   1680
   TATAAAGAGA ATCATTCATT GCAACATGAT ATAAAATAAC AACACAATAA AAGCAATTAA   1740
   ATAAACAAAC AATAGGGAAA TGTTTAAGTT CATCATGGTA CTTAGACTTA ATGGAATGTC   1800
   ATGCCTTATT TACATTTTTA AACAGGTACT GAGGGACTCC TGTCTGCCAA GGGCCGTATT   1860
   GAGTACTTTC CACAACCTAA TTTAATCCAC ACTATACTGT GAGATTAAAA ACATTCATTA   1920
   AAATGTTGCA AAGGTTCTAT AAAGCTGAGA GACAAATATA TTCTATAACT CAGCAATCCC   1980
   ACTTCTAGAT GACTGAGTGT CCCCACCCAC CAAAAAACTA TGCAAGAATG TTCAAAGCAG   2040
   CTTTATTTAC AAAAGCCAAA AATTGGAAAT AGCCCGATTG TCCAACAATA GAATGAGTTA   2100
   TTAAACTGTG GTATGTTTAT ACATTAGAAT ACCCAATGAG GAGAATTAAC AAGCTACAAC   2160
   TATACCTACT CACACAGATG AATCTCATAA AAATAATGTT ACATAAGAGA AACTCAATGC   2220
   AAAAGATATG TTCTGTATGT TTTCATCCAT ATAAAGTTCA AAACCAGGTA AAAATAAAGT   2280
   TAGAAATTTG GATGGAAATT ACTCTTAGCT GGGGGTGGGC GAGTTAGTGC CTGGGAGAAG   2340
   ACAAGAAGGG GCTTCTGGGG TCTTGGTAAT GTTCTGTTCC TCGTGTGGGG TTGTGCAGTT   2400
   ATGATCTGTG CACTGTTCTG TATACACATT ATGCTTCAAA ATAACTTCAC ATAAAGAACA   2460
   TCTTATACCC AGTTAATAGA TAGAAGAGGA ATAAGTAATA GGTCAAGACC AACGCAGCTG   2520
   GTAAGTGGGG GCCTGGGATC AAATAGCTAC CTGCCTAATC CTGCCCWCTT GAGCCCTGAA   2580
   TGAGTCTGCC TTCCAGGGCT CAAGGTGCTC AACAAAACAA CAGGCCTGCT ATTTTCCTGG   2640
   CATCTGTGCC CTGTTTGGCT AGCTAGGAGC ACACATACAT AGAAATTAAA TGAAACAGAC   2700
   CTTCAGCAAG GGACAGAGGG ACAGAATTAA CCTTGCCCAG ACACTGGAAA CCCATGTATG   2760
   AACACTCACA TGTTTGGGAA GGGGGAAGGG CACATGTAAA TGAGGACTCT TCCTCATTCT 2820
   ATGGGGCACT CTGGCCCTGC CCCTCTCAGC TACTCATCCA TCCAACACAC CTTTCTAAGT 2880
   ACCTCTCTCT GCCTACACTC TGAAGGGGTT CAGGAGTAAC TAACACAGCA TCCCTTCCCT 2940
   CAAATGACTG ACAATCCCTT TGTCCTGCTT TGTTTTTCTT TCCAGTCAGT ACTGGGAAAG 3000
   TGGGGAAGGA CAGTCATGGA GAAACTACAT AAGGAAGCAC CTTGCCCTTC TGCCTCTTGA 3060
   GAATGTTGAT GAGTATCAAA TCTTTCAAAC TTTGGAGGTT TGAGTAGGGG TGAGACTCAG 3120
   TAATGTCCCT TCCAATGACA TGAACTTGCT CACTCATCCC TGGGGGCCAA ATTGAACAAT 3180
   CAAAGGCAGG CATAATCCAG TTATGAATTC TTGCGGCCGC TTGCTAGCTT CACGTGTTGG 3240
   ATCCAACCGC GGAAGGGCCC TATTCTATAG TGTCACCTAA ATGCTAGAGC TCGCTGATCA 3300
   GCCTCGACTG TGCCTTCTAG TTGCCAGCCA TCTGTTGTTT GCCCCTCCCC CGTGCCTTCC 3360
   TTGACCCTGG AAGGTGCCAC TCCCACTGTC CTTTCCTAAT AAAATGAGGA AATTGCATCG 3420
   CATTGTCTGA GTAGGTGTCA TTCTATTCTG GGGGGTGGGG TGGGGCAGGA CAGCAAGGGG 3480
   GAGGATTGGG AAGACAATAG CAGGCATGCT GGGGATGCGG TGGGCTCTAT GGCTTCTGAG 3540
   GCGGAAAGAA CCAGCTGGGG CTCTAGGGGG TATCCCCACG CGCCCTGTAG CGGCGCATTA 3600
   AGCGCGGCGG GTGTGGTGGT TACGCGCAGC GTGACCGCTA CACTTGCCAG CGCCCTAGCG 3660
   CCCGCTCCTT TCGCTTTCTT CCCTTCCTTT CTCGCCACGT TCGCCGGGCC TCTCAAAAAA 3720
   GGGAAAAAAA GCATGCATCT CAATTAGTCA GCAACCATAG TCCCGCCCCT AACTCCGCCC 3780
   ATCCCGCCCC TAACTCCGCC CAGTTCCGCC CATTCTCCGC CCCATGGCTG ACTAATTTTT 3840
   TTTATTTATG CAGAGGCCGA GGCCGCCTCG GCCTCTGAGC TATTCCAGAA GTAGTGAGGA 3900
   GGCTTTTTTG GAGGCCTAGG CTTTTGCAAA AAGCTTGGAC AGCTCAGGGC TGCGATTTCG 3960
   CGCCAAACTT GACGGCAATC CTAGCGTGAA GGCTGGTAGG ATTTTATCCC CGCTGCCATC 4020
   ATGGTTCGAC CATTGAACTG CATCGTCGCC GTGTCCCAAA ATATGGGGAT TGGCAAGAAC 4080
   GGAGACCTAC CCTGGCCTCC GCTCAGGAAC GAGTTCAAGT ACTTCCAAAG AATGACCACA 4140
   ACCTCTTCAG TGGAAGGTAA ACAGAATCTG GTGATTATGG GTAGGAAAAC CTGGTTCTCC 4200
   ATTCCTGAGA AGAATCGACC TTTAAAGGAC AGAATTAATA TAGTTCTCAG TAGAGAACTC 4260
   AAAGAACCAC CACGAGGAGC TCATTTTCTT GCCAAAAGTT TGGATGATGC CTTAAGACTT 4320
   ATTGAACAAC CGGAATTGGC AAGTAAAGTA GACATGGTTT GGATAGTCGG AGGCAGTTCT 4380
   GTTTACCAGG AAGCCATGAA TCAACCAGGC CACCTTAGAC TCTTTGTGAC AAGGATCATG 4440
   CAGGAATTTG AAAGTGACAC GTTTTTCCCA GAAATTGATT TGGGGAAATA TAAACTTCTC 4500
   CCAGAATACC CAGGCGTCCT CTCTGAGGTC CAGGAGGAAA AAGGCATCAA GTATAAGTTT 4560
   GAAGTCTACG AGAAGAAAGA CTAACAGGAA GATGCTTTCA AGTTCTCTGC TCCCCTCCTA 4620
   AAGCTATGCA TTTTTATAAG ACCATGGGAC TTTTGCTGGC TTTAGATCTC TTTGTGAAGG 4680
   AACCTTACTT CTGTGGTGTG ACATAATTGG ACAAACTACC TACAGAGATT TAAAGCTCTA 4740
   AGGTAAATAT AAAATTTTTA AGTGTATAAT GTGTTAAACT ACTGATTCTA ATTGTTTGTG 4800
   TATTTTAGAT TCCAACCTAT GGAACTGATG AATGGGAGCA GTGGTGGAAT GCCTTTAATG 4860
   AGGAAAACCT GTTTTGCTCA GAAGAAATGC CATCTAGTGA TGATGAGGCT ACTGCTGACT 4920
   CTCAACATTC TACTCCTCCA AAAAAGAAGA GAAAGGTAGA AGACCCCAAG GACTTTCCTT 4980
   CAGAATTGCT AAGTTTTTTG AGTCATGCTG TGTTTAGTAA TAGAACTCTT GCTTGCTTTG 5040
   CTATTTACAC CACAAAGGAA AAAGCTGCAC TGCTATACAA GAAAATTATG GAAAAATATT 5100
   CTGTAACCTT TATAAGTAGG CATAACAGTT ATAATCATAA CATACTGTTT TTTCTTACTC 5160
   CACACAGGCA TAGAGTGTCT GCTATTAATA ACTATGCTCA AAAATTGTGT ACCTTTAGCT 5220
   TTTTAATTTG TAAAGGGGTT AATAAGGAAT ATTTGATGTA TAGTGCCTTG ACTAGAGATC 5280
   ATAATCAGCC ATACCACATT TGTAGAGGTT TTACTTGCTT TAAAAAACCT CCCACACCTC 5340
   CCCCTGAACC TGAAACATAA AATGAATGCA ATTGTTGTTG TTAACTTGTT TATTGCAGCT 5400
   TATAATGGTT ACAAATAAAG CAATAGCATC ACAAATTTCA CAAATAAAGC ATTTTTTTCA 5460
   CTGCATTCTA GTTGTGGTTT GTCCAAACTC ATCAATGTAT CTTATCATGT CTGGATCGGC 5520
   TGGATGATCC TCCAGCGCGG GGATCTCATG CTGGAGTTCT TCGCCCACCC CAACTTGTTT 5580
   ATTGCAGCTT ATAATGGTTA CAAATAAAGC AATAGCATCA CAAATTTCAC AAATAAAGCA 5640
   TTTTTTTCAC TGCATTCTAG TTGTGGTTTG TCCAAACTCA TCAATGTATC TTATCATGTC 5700
   TGTATACCGT CGACCTCTAG CTAGAGCTTG GCGTAATCAT GGTCATAGCT GTTTCCTGTG 5760
   TGAAATTGTT ATCCGCTCAC AATTCCACAC AACATACGAG CCGGAAGCAT AAAGTGTAAA 5820
   GCCTGGGGTG CCTAATGAGT GAGCTAACTC ACATTAATTG CGTTGCGCTC ACTGCCCGCT 5880
   TTCCAGTCGG GAAACCTGTC GTGCCAGCTG CATTAATGAA TCGGCCAACG CGCGGGGAGA 5940
   GGCGGTTTGC GTATTGGGCG CTCTTCCGCT TCCTCGCTCA CTGACTCGCT GCGCTCGGTC 6000
   GTTCGGCTGC GGCGAGCGGT ATCAGCTCAC TCAAAGGCGG TAATACGGTT ATCCACAGAA 6060
   TCAGGGGATA ACGCAGGAAA GAACATGTGA GCAAAAGGCC AGCAAAAGGC CAGGAACCGT 6120
   AAAAAGGCCG CGTTGCTGGC GTTTTTCCAT AGGCTCCGCC CCCCTGACGA GCATCACAAA 6180
   AATCGACGCT CAAGTCAGAG GTGGCGAAAC CCGACAGGAC TATAAAGATA CCAGGCGTTT 6240
   CCCCCTGGAA GCTCCCTCGT GCGCTCTCCT GTTCCGACCC TGCCGCTTAC CGGATACCTG 6300
   TCCGCCTTTC TCCCTTCGGG AAGCGTGGCG CTTTCTCAAT GCTCACGCTG TAGGTATCTC   6360
   AGTTCGGTGT AGGTCGTTCG CTCCAAGCTG GGCTGTGTGC ACGAACCCCC CGTTCAGCCC   6420
   GACCGCTGCG CCTTATCCGG TAACTATCGT CTTGAGTCCA ACCCGGTAAG ACACGACTTA   6480
   TCGCCACTGG CAGCAGCCAC TGGTAACAGG ATTAGCAGAG CGAGGTATGT AGGCGGTGCT   6540
   ACAGAGTTCT TGAAGTGGTG GCCTAACTAC GGCTACACTA GAAGGACAGT ATTTGGTATC   6600
   TGCGCTCTGC TGAAGCCAGT TACCTTCGGA AAAAGAGTTG GTAGCTCTTG ATCCGGCAAA   6660
   CAAACCACCG CTGGTAGCGG TGGTTTTTTT GTTTGCAAGC AGCAGATTAC GCGCAGAAAA   6720
   AAAGGATCTC AAGAAGATCC TTTGATCTTT TCTACGGGGT CTGACGCTCA GTGGAACGAA   6780
   AACTCACGTT AAGGGATTTT GGTCATGAGA TTATCAAAAA GGATCTTCAC CTAGATCCTT   6840
   TTAAATTAAA AATGAAGTTT TAAATCAATC TAAAGTATAT ATGAGTAAAC TTGGTCTGAC   6900
   AGTTACCAAT GCTTAATCAG TGAGGCACCT ATCTCAGCGA TCTGTCTATT TCGTTCATCC   6960
   ATAGTTGCCT GACTCCCCGT CGTGTAGATA ACTACGATAC GGGAGGGCTT ACCATCTGGC   7020
   CCCAGTGCTG CAATGATACC GCGAGACCCA CGCTCACCGG CTCCAGATTT ATCAGCAATA   7080
   AACCAGCCAG CCGGAAGGGC CGAGCGCAGA AGTGGTCCTG CAACTTTATC CGCCTCCATC   7140
   CAGTCTATTA ATTGTTGCCG GGAAGCTAGA GTAAGTAGTT CGCCAGTTAA TAGTTTGCGC   7200
   AACGTTGTTG CCATTGCTAC AGGCATCGTG GTGTCACGCT CGTCGTTTGG TATGGCTTCA   7260
   TTCAGCTCCG GTTCCCAACG ATCAAGGCGA GTTACATGAT CCCCCATGTT GTGCAAAAAA   7320
   GCGGTTAGCT CCTTCGGTCC TCCGATCGTT GTCAGAAGTA AGTTGGCCGC AGTGTTATCA   7380
   CTCATGGTTA TGGCAGCACT GCATAATTCT CTTACTGTCA TGCCATCCGT AAGATGCTTT   7440
   TCTGTGACTG GTGAGTACTC AACCAAGTCA TTCTGAGAAT AGTGTATGCG GCGACCGAGT   7500
   TGCTCTTGCC CGGCGTCAAT ACGGGATAAT ACCGCGCCAC ATAGCAGAAC TTTAAAAGTG   7560
   CTCATCATTG GAAAACGTTC TTCGGGGCGA AAACTCTCAA GGATCTTACC GCTGTTGAGA   7620
   TCCAGTTCGA TGTAACCCAC TCGTGCACCC AACTGATCTT CAGCATCTTT TACTTTCACC   7680
   AGCGTTTCTG GGTGAGCAAA AACAGGAAGG CAAAATGCCG CAAAAAAGGG AATAAGGGCG   7740
   ACACGGAAAT GTTGAATACT CATACTCTTC CTTTTTCAAT ATTATTGAAG CATTTATCAG   7800
   GGTTATTGTC TCATGAGCGG ATACATATTT GAATGTATTT AGAAAAATAA ACAAATAGGG   7860
   GTTCCGCGCA CATTTCCCCG AAAAGTGCCA CCTGACGTCG ACGGATCGGG AGATCTGCTA   7920
   GCCCGGGTGA CCTGAGGCGC GCCGGCTTCG AATAGCCAGA GTAACCTTTT TTTTTAATTT   7980
   TATTTTATTT TATTTTTGAG ATGGAGTTTG GCGCCGATCT CCCGATCCCC TATGGTCGAC   8040
   TCTCAGTACA ATCTGCTCTG ATGCCGCATA GTTAAGCCAG TATCTGCTCC CTGCTTGTGT   8100
   GTTGGAGGTC GCTGAGTAGT GCGCGAGCAA AATTTAAGCT ACAACAAGGC AAGGCTTGAC   8160
   CGACAATTGC ATGAAGAATC TGCTTAGGGT TAGGCGTTTT GCGCTGCTTC GCGATGTACG   8220
   GGCCAGATAT ACGCGTTGAC ATTGATTATT GACTAGTTAT TAATAGTAAT CAATTACGGG   8280
   GTCATTAGTT CATAGCCCAT ATATGGAGTT CCGCGTTACA TAACTTACGG TAAATGGCCC   8340
   GCCTGGCTGA CCGCCCAACG ACCCCCGCCC ATTGACGTCA ATAATGACGT ATGTTCCCAT   8400
   AGTAACGCCA ATAGGGACTT TCCATTGACG TCAATGGGTG GACTATTTAC GGTAAACTGC   8460
   CCACTTGGCA GTACATCAAG TGTATCATAT GCCAAGTACG CCCCCTATTG ACGTCAATGA   8520
   CGGTAAATGG CCCGCCTGGC ATTATGCCCA GTACATGACC TTATGGGACT TTCCTACTTG   8580
   GCAGTACATC TACGTATTAG TCATCGCTAT TACCATGGTG ATGCGGTTTT GGCAGTACAT   8640
   CAATGGGCGT GGATAGCGGT TTGACTCACG GGGATTTCCA AGTCTCCACC CCATTGACGT   8700
   CAATGGGAGT TTGTTTTGGC ACCAAAATCA ACGGGACTTT CCAAAATGTC GTAACAACTC   8760
   CGCCCCATTG ACGCAAATGG GCGGTAGGCG TGTACGGTGG GAGGTCTATA TAAGCAGAGC   8820
   TCTCTGGCTA ACTAGAGAAC CCACTGCTTA CTGGCTTATC GAAATTAATA CGACTCACTA   8880
   TAGGGAGACC CAAGCTT                                                  8897
(2) INFORMATION FOR SEQ ID NO:12:
   (i) SEQUENCE CHARACTERISTICS:
      (A) LENGTH: 8321 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:12:
   GGTACCAATT TAAATTGATA TCTCCTTAGG TCTCGAGTCT CTAGATAACC GGTCAATCGA     60
   TTGGAATTCT TGCGGCCGCT TGCTAGCCAC CATGGAGTTG TGGTTAAGCT TGGTCTTCCT    120
   TGTCCTTGTT TTAAAAGGTG TCCAGTGTGA AGTGCAACTG GTGGAGTCTG GGGGAGGCTT 180
   AGTGCAGCCT GGAGGGTCCC TGCGACTTTC CTGTGCTGCA TCTGGATTCC CGTTCAGTGA 240
   CTATTACATG TATTGGGTTC GCCAGGCTCC AGGCAAGGGA CTGGAGTGGG TCTCATACAT 300
   TAGTCAAGAT GGTGATATAA CCGACTATGC AGACTCCGTA AAGGGTCGAT TCACCATCTC 360
   CAGAGACAAT GCAAAGAACA GCCTGTACCT GCAAATGAAC AGCCTGAGGG ACGAGGACAC 420
   AGCCGTGTAT TACTGTGCAA GAGGCCTGGC GGACGGGGCC TGGTTTGCTT ACTGGGGCCA 480
   AGGGACTCTG GTCACGGTCT CTTCCGCTAG CACCAAGGGC CCATCGGTCT TCCCCCTGGC 540
   ACCCTCCTCC AAGAGCACCT CTGGGGGCAC AGCGGCCCTG GGCTGCCTGG TCAAGGACTA 600
   CTTCCCCGAA CCGGTGACGG TGTCGTGGAA CTCAGGCGCC CTGACCAGCG GCGTGCACAC 660
   CTTCCCGGCT GTCCTACAGT CCTCAGGACT CTACTCCCTC AGCAGCGTGG TCACCGTGCC 720
   CTCCAGCAGC TTGGGCACCC AGACCTACAT CTGCAACGTG AATCACAAGC CCAGCAACAC 780
   CAAGGTGGAC AAGAAAGTTG GTGAGAGGCC AGCACAGGGA GGGAGGGTGT CTGCTGGAAG 840
   CCAGGCTCAG CGCTCCTGCC TGGACGCATC CCGGCTATGC AGCCCCAGTC CAGGGCAGCA 900
   AGGCAGGCCC CGTCTGCCTC TTCACCCGGA GGCCTCTGCC CGCCCCACTC ATGCTCAGGG 960
   AGAGGGTCTT CTGGCTTTTT CCCCAGGCTC TGGGCAGGCA CAGGCTAGGT GCCCCTAACC 1020
   CAGGCCCTGC ACACAAAGGG GCAGGTGCTG GGCTCAGACC TGCCAAGAGC CATATCCGGG 1080
   AGGACCCTGC CCCTGACCTA AGCCCACCCC AAAGGCCAAA CTCTCCACTC CCTCAGCTCG 1140
   GACACCTTCT CTCCTCCCAG ATTCCAGTAA CTCCCAATCT TCTCTCTGCA GAGCCCAAAT 1200
   CTTGTGACAA AACTCACACA TGCCCACCGT GCCCAGGTAA GCCAGCCCAG GCCTCGCCCT 1260
   CCAGCTCAAG GCGGGACAGG TGCCCTAGAG TAGCCTGCAT CCAGGGACAC ACCACGTGGG 1320
   TACCAACATG TCCGGAGCCA CATGGACAGA GGCCGGCTCG GCCCACCCTC TGCCCTGAGA 1380
   GTGACCGCTG TACCAACCTC TGTCCCTACA GGGCAGCCCC GAGAACCACA GGTGTACACC 1440
   CTGCCCCCAT CCCGGGATGA GCTGACCAAG AACCAGGTCA GCCTGACCTG CCTGGTCAAA 1500
   GGCTTCTATC CCAGCGACAT CGCCGTGGAG TGGGAGAGCA ATGGGCAGCC GGAGAACAAC 1560
   TACAAGACCA CGCCTCCCGT GCTGGACTCC GACGGCTCCT TCTTCCTCTA CAGCAAGCTC 1620
   ACCGTGGACA AGAGCAGGTG GCAGCAGGGG AACGTCTTCT CATGCTCCGT GATGCATGAG 1680
   GCTCTGCACA ACCACTACAC GCAGAAGAGC CTCTCCCTGT CTCCGGGTAA ATGAGTGCGA 1740
   CGGCCGGCAA GCCCCCGCTC CCCGGGCTCT CGCGGTCGCA CGAGGATGCT TGGCACGTAC 1800
   CCCCTGTACA TACTTCCCGG GCGCCCAGCA TGGAAATAAA GCACCCAGCG CTGCCCTGGG 1860
   CCCCTGCGAG ACTGTGATGG TTCTTTCCAC GGGTCAGGCC GAGTCTGAGG CCTGAGTGGC 1920
   ATGAGGGAGG CAGAGCGGGT CCCACTGTCC CCACACTGGC CCAGGCTGTG CAGGTGTGCC 1980
   TGGGCCCCCT AGGGTGGGGC TCAGCCAGGG GCTGCCCTCG GCAGGGTGGG GGATTTGCCA 2040
   GCGTGGCCCT CCCTCCAGCA GCACCTGCCC TGGGCTGGGC CACGGGAAGC CCTAGGAGCC 2100
   CCTGGGGACA GACACACAGC CCCTGCCTCT GTAGGAGACT GTCCTGTTCT GTGAGCGCCC 2160
   CTGTCCTCCC GACCTCCATG CCCACTCGGG GGCATGCCTA GTCCATGTGC GTAGGGACAG 2220
   GCCCTCCCTC ACCCATCTAC CCCCACGGCA CTAACCCCTG GCTGCCCTGC CCAGCCTCGC 2280
   ACCCGCATGG GGACACAACC GACTCCGGGG ACATGCACTC TCGGGCCCTG TGGAGGGACT 2340
   GGTGCAGATG CCCACACACA CACTCAGCCC AGACCCGTTC AACAAACCCC GCACTGAGGT 2400
   TGGCCGGCCA CACGGCCACC ACACACACAC GTGCACGCCT CACACACGGA GCCTCACCCG 2460
   GGCGAACTGC ACAGCACCCA GACCAGAGCA AGGTCCTCGC ACACGTGAAC ACTCCTCGGA 2520
   CACAGGCCCC CACGAGCCCC ACGCGGCACC TCAAGGCCCA CGAGCCTCTC GGCAGCTTCT 2580
   CCACATGCTG ACCTGCTCAG ACAAACCCAG CCCTCCTCTC ACAAGGGTGC CCCTGCAGCC 2640
   GCCACACACA CACAGGGGAT CACACACCAC GTCACGTCCC TGGCCCTGGC CCACTTCCCA 2700
   GTGCCGCCCT TCCCTGCAGG ACGGATCAGC CTCGACTGTG CCTTCTAGTT GCCAGCCATC 2760
   TGTTGTTTGC CCCTCCCCCG TGCCTTCCTT GACCCTGGAA GGTGCCACTC CCACTGTCCT 2820
   TTCCTAATAA AATGAGGAAA TTGCATCGCA TTGTCTGAGT AGGTGTCATT CTATTCTGGG 2880
   GGGTGGGGTG GGGCAGGACA GCAAGGGGGA GGATTGGGAA GACAATAGCA GGCATGCTGG 2940
   GGATGCGGTG GGCTCTATGG CTTCTGAGGC GGAAAGAACC AGCTGGGGCT CTAGGGGGTA 3000
   TCCCCACGCG CCCTGTAGCG GCGCATTAAG CGCGGCGGGT GTGGTGGTTA CGCGCAGCGT 3060
   GACCGCTACA CTTGCCAGCG CCCTAGCGCC CGCTCCTTTC GCTTTCTTCC CTTCCTTTCT 3120
   CGCCACGTTC GCCGGGCCTC TCAAAAAAGG GAAAAAAAGC ATGCATCTCA ATTAGTCAGC 3180
   AACCATAGTC CCGCCCCTAA CTCCGCCCAT CCCGCCCCTA ACTCCGCCCA GTTCCGCCCA 3240
   TTCTCCGCCC CATGGCTGAC TAATTTTTTT TATTTATGCA GAGGCCGAGG CCGCCTCGGC 3300
   CTCTGAGCTA TTCCAGAAGT AGTGAGGAGG CTTTTTTGGA GGCCTAGGCT TTTGCAAAAA 3360
   GCTTGGACAG CTCAGGGCTG CGATTTCGCG CCAAACTTGA CGGCAATCCT AGCGTGAAGG 3420
   CTGGTAGGAT TTTATCCCCG CTGCCATCAT GGTTCGACCA TTGAACTGCA TCGTCGCCGT 3480
   GTCCCAAAAT ATGGGGATTG GCAAGAACGG AGACCTACCC TGGCCTCCGC TCAGGAACGA 3540
   GTTCAAGTAC TTCCAAAGAA TGACCACAAC CTCTTCAGTG GAAGGTAAAC AGAATCTGGT 3600
   GATTATGGGT AGGAAAACCT GGTTCTCCAT TCCTGAGAAG AATCGACCTT TAAAGGACAG 3660
   AATTAATATA GTTCTCAGTA GAGAACTCAA AGAACCACCA CGAGGAGCTC ATTTTCTTGC 3720
   CAAAAGTTTG GATGATGCCT TAAGACTTAT TGAACAACCG GAATTGGCAA GTAAAGTAGA 3780
   CATGGTTTGG ATAGTCGGAG GCAGTTCTGT TTACCAGGAA GCCATGAATC AACCAGGCCA 3840
   CCTTAGACTC TTTGTGACAA GGATCATGCA GGAATTTGAA AGTGACACGT TTTTCCCAGA 3900
   AATTGATTTG GGGAAATATA AACTTCTCCC AGAATACCCA GGCGTCCTCT CTGAGGTCCA 3960
   GGAGGAAAAA GGCATCAAGT ATAAGTTTGA AGTCTACGAG AAGAAAGACT AACAGGAAGA 4020
   TGCTTTCAAG TTCTCTGCTC CCCTCCTAAA GCTATGCATT TTTATAAGAC CATGGGACTT 4080
   TTGCTGGCTT TAGATCTCTT TGTGAAGGAA CCTTACTTCT GTGGTGTGAC ATAATTGGAC 4140
   AAACTACCTA CAGAGATTTA AAGCTCTAAG GTAAATATAA AATTTTTAAG TGTATAATGT 4200
   GTTAAACTAC TGATTCTAAT TGTTTGTGTA TTTTAGATTC CAACCTATGG AACTGATGAA 4260
   TGGGAGCAGT GGTGGAATGC CTTTAATGAG GAAAACCTGT TTTGCTCAGA AGAAATGCCA 4320
   TCTAGTGATG ATGAGGCTAC TGCTGACTCT CAACATTCTA CTCCTCCAAA AAAGAAGAGA 4380
   AAGGTAGAAG ACCCCAAGGA CTTTCCTTCA GAATTGCTAA GTTTTTTGAG TCATGCTGTG 4440
   TTTAGTAATA GAACTCTTGC TTGCTTTGCT ATTTACACCA CAAAGGAAAA AGCTGCACTG 4500
   CTATACAAGA AAATTATGGA AAAATATTCT GTAACCTTTA TAAGTAGGCA TAACAGTTAT 4560
   AATCATAACA TACTGTTTTT TCTTACTCCA CACAGGCATA GAGTGTCTGC TATTAATAAC 4620
   TATGCTCAAA AATTGTGTAC CTTTAGCTTT TTAATTTGTA AAGGGGTTAA TAAGGAATAT 4680
   TTGATGTATA GTGCCTTGAC TAGAGATCAT AATCAGCCAT ACCACATTTG TAGAGGTTTT 4740
   ACTTGCTTTA AAAAACCTCC CACACCTCCC CCTGAACCTG AAACATAAAA TGAATGCAAT 4800
   TGTTGTTGTT AACTTGTTTA TTGCAGCTTA TAATGGTTAC AAATAAAGCA ATAGCATCAC 4860
   AAATTTCACA AATAAAGCAT TTTTTTCACT GCATTCTAGT TGTGGTTTGT CCAAACTCAT 4920
   CAATGTATCT TATCATGTCT GGATCGGCTG GATGATCCTC CAGCGCGGGG ATCTCATGCT 4980
   GGAGTTCTTC GCCCACCCCA ACTTGTTTAT TGCAGCTTAT AATGGTTACA AATAAAGCAA 5040
   TAGCATCACA AATTTCACAA ATAAAGCATT TTTTTCACTG CATTCTAGTT GTGGTTTGTC 5100
   CAAACTCATC AATGTATCTT ATCATGTCTG TATACCGTCG ACCTCTAGCT AGAGCTTGGC 5160
   GTAATCATGG TCATAGCTGT TTCCTGTGTG AAATTGTTAT CCGCTCACAA TTCCACACAA 5220
   CATACGAGCC GGAAGCATAA AGTGTAAAGC CTGGGGTGCC TAATGAGTGA GCTAACTCAC 5280
   ATTAATTGCG TTGCGCTCAC TGCCCGCTTT CCAGTCGGGA AACCTGTCGT GCCAGCTGCA 5340
   TTAATGAATC GGCCAACGCG CGGGGAGAGG CGGTTTGCGT ATTGGGCGCT CTTCCGCTTC 5400
   CTCGCTCACT GACTCGCTGC GCTCGGTCGT TCGGCTGCGG CGAGCGGTAT CAGCTCACTC 5460
   AAAGGCGGTA ATACGGTTAT CCACAGAATC AGGGGATAAC GCAGGAAAGA ACATGTGAGC 5520
   AAAAGGCCAG CAAAAGGCCA GGAACCGTAA AAAGGCCGCG TTGCTGGCGT TTTTCCATAG 5580
   GCTCCGCCCC CCTGACGAGC ATCACAAAAA TCGACGCTCA AGTCAGAGGT GGCGAAACCC 5640
   GACAGGACTA TAAAGATACC AGGCGTTTCC CCCTGGAAGC TCCCTCGTGC GCTCTCCTGT 5700
   TCCGACCCTG CCGCTTACCG GATACCTGTC CGCCTTTCTC CCTTCGGGAA GCGTGGCGCT 5760
   TTCTCAATGC TCACGCTGTA GGTATCTCAG TTCGGTGTAG GTCGTTCGCT CCAAGCTGGG 5820
   CTGTGTGCAC GAACCCCCCG TTCAGCCCGA CCGCTGCGCC TTATCCGGTA ACTATCGTCT 5880
   TGAGTCCAAC CCGGTAAGAC ACGACTTATC GCCACTGGCA GCAGCCACTG GTAACAGGAT 5940
   TAGCAGAGCG AGGTATGTAG GCGGTGCTAC AGAGTTCTTG AAGTGGTGGC CTAACTACGG 6000
   CTACACTAGA AGGACAGTAT TTGGTATCTG CGCTCTGCTG AAGCCAGTTA CCTTCGGAAA 6060
   AAGAGTTGGT AGCTCTTGAT CCGGCAAACA AACCACCGCT GGTAGCGGTG GTTTTTTTGT 6120
   TTGCAAGCAG CAGATTACGC GCAGAAAAAA AGGATCTCAA GAAGATCCTT TGATCTTTTC 6180
   TACGGGGTCT GACGCTCAGT GGAACGAAAA CTCACGTTAA GGGATTTTGG TCATGAGATT 6240
   ATCAAAAAGG ATCTTCACCT AGATCCTTTT AAATTAAAAA TGAAGTTTTA AATCAATCTA 6300
   AAGTATATAT GAGTAAACTT GGTCTGACAG TTACCAATGC TTAATCAGTG AGGCACCTAT 6360
   CTCAGCGATC TGTCTATTTC GTTCATCCAT AGTTGCCTGA CTCCCCGTCG TGTAGATAAC 6420
   TACGATACGG GAGGGCTTAC CATCTGGCCC CAGTGCTGCA ATGATACCGC GAGACCCACG 6480
   CTCACCGGCT CCAGATTTAT CAGCAATAAA CCAGCCAGCC GGAAGGGCCG AGCGCAGAAG 6540
   TGGTCCTGCA ACTTTATCCG CCTCCATCCA GTCTATTAAT TGTTGCCGGG AAGCTAGAGT 6600
   AAGTAGTTCG CCAGTTAATA GTTTGCGCAA CGTTGTTGCC ATTGCTACAG GCATCGTGGT 6660
   GTCACGCTCG TCGTTTGGTA TGGCTTCATT CAGCTCCGGT TCCCAACGAT CAAGGCGAGT 6720
   TACATGATCC CCCATGTTGT GCAAAAAAGC GGTTAGCTCC TTCGGTCCTC CGATCGTTGT 6780
   CAGAAGTAAG TTGGCCGCAG TGTTATCACT CATGGTTATG GCAGCACTGC ATAATTCTCT 6840
   TACTGTCATG CCATCCGTAA GATGCTTTTC TGTGACTGGT GAGTACTCAA CCAAGTCATT 6900
   CTGAGAATAG TGTATGCGGC GACCGAGTTG CTCTTGCCCG GCGTCAATAC GGGATAATAC 6960
   CGCGCCACAT AGCAGAACTT TAAAAGTGCT CATCATTGGA AAACGTTCTT CGGGGCGAAA 7020
   ACTCTCAAGG ATCTTACCGC TGTTGAGATC CAGTTCGATG TAACCCACTC GTGCACCCAA 7080
   CTGATCTTCA GCATCTTTTA CTTTCACCAG CGTTTCTGGG TGAGCAAAAA CAGGAAGGCA 7140
   AAATGCCGCA AAAAAGGGAA TAAGGGCGAC ACGGAAATGT TGAATACTCA TACTCTTCCT 7200
   TTTTCAATAT TATTGAAGCA TTTATCAGGG TTATTGTCTC ATGAGCGGAT ACATATTTGA 7260
   ATGTATTTAG AAAAATAAAC AAATAGGGGT TCCGCGCACA TTTCCCCGAA AAGTGCCACC 7320
   TGACGTCGAC GGATCGGGAG ATCTGCTAGG TGACCTGAGG CGCGCCGGCT TCGAATAGCC 7380
   AGAGTAACCT TTTTTTTTAA TTTTATTTTA TTTTATTTTT GAGATGGAGT TTGGCGCCGA 7440
   TCTCCCGATC CCCTATGGTC GACTCTCAGT ACAATCTGCT CTGATGCCGC ATAGTTAAGC 7500
   CAGTATCTGC TCCCTGCTTG TGTGTTGGAG GTCGCTGAGT AGTGCGCGAG CAAAATTTAA 7560
   GCTACAACAA GGCAAGGCTT GACCGACAAT TGCATGAAGA ATCTGCTTAG GGTTAGGCGT 7620
   TTTGCGCTGC TTCGCGATGT ACGGGCCAGA TATACGCGTT GACATTGATT ATTGACTAGT 7680
   TATTAATAGT AATCAATTAC GGGGTCATTA GTTCATAGCC CATATATGGA GTTCCGCGTT 7740
   ACATAACTTA CGGTAAATGG CCCGCCTGGC TGACCGCCCA ACGACCCCCG CCCATTGACG 7800
   TCAATAATGA CGTATGTTCC CATAGTAACG CCAATAGGGA CTTTCCATTG ACGTCAATGG 7860
   GTGGACTATT TACGGTAAAC TGCCCACTTG GCAGTACATC AAGTGTATCA TATGCCAAGT 7920
   ACGCCCCCTA TTGACGTCAA TGACGGTAAA TGGCCCGCCT GGCATTATGC CCAGTACATG 7980
   ACCTTATGGG ACTTTCCTAC TTGGCAGTAC ATCTACGTAT TAGTCATCGC TATTACCATG 8040
   GTGATGCGGT TTTGGCAGTA CATCAATGGG CGTGGATAGC GGTTTGACTC ACGGGGATTT 8100
   CCAAGTCTCC ACCCCATTGA CGTCAATGGG AGTTTGTTTT GGCACCAAAA TCAACGGGAC 8160
   TTTCCAAAAT GTCGTAACAA CTCCGCCCCA TTGACGCAAA TGGGCGGTAG GCGTGTACGG 8220
   TGGGAGGTCT ATATAAGCAG AGCTCTCTGG CTAACTAGAG AACCCACTGC TTACTGGCTT 8280
   ATCGAAATTA ATACGACTCA CTATAGGGAG ACCCAAGCTT G 8321
(2) INFORMATION FOR SEQ ID NO:13:
   (i) SEQUENCE CHARACTERISTICS:
      (A) LENGTH: 8897 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:13:
   GACGGATCGG GAGATCTGCT AGCCCGGGTG ACCTGAGGCG CGCCGGCTTC GAATAGCCAG     60
   AGTAACCTTT TTTTTTAATT TTATTTTATT TTATTTTTGA GATGGAGTTT GGCGCCGATC    120
   TCCCGATCCC CTATGGTCGA CTCTCAGTAC AATCTGCTCT GATGCCGCAT AGTTAAGCCA    180
   GTATCTGCTC CCTGCTTGTG TGTTGGAGGT CGCTGAGTAG TGCGCGAGCA AAATTTAAGC    240
   TACAACAAGG CAAGGCTTGA CCGACAATTG CATGAAGAAT CTGCTTAGGG TTAGGCGTTT    300
   TGCGCTGCTT CGCGATGTAC GGGCCAGATA TACGCGTTGA CATTGATTAT TGACTAGTTA    360
   TTAATAGTAA TCAATTACGG GGTCATTAGT TCATAGCCCA TATATGGAGT TCCGCGTTAC    420
   ATAACTTACG GTAAATGGCC CGCCTGGCTG ACCGCCCAAC GACCCCCGCC CATTGACGTC    480
   AATAATGACG TATGTTCCCA TAGTAACGCC AATAGGGACT TTCCATTGAC GTCAATGGGT    540
   GGACTATTTA CGGTAAACTG CCCACTTGGC AGTACATCAA GTGTATCATA TGCCAAGTAC    600
   GCCCCCTATT GACGTCAATG ACGGTAAATG GCCCGCCTGG CATTATGCCC AGTACATGAC    660
   CTTATGGGAC TTTCCTACTT GGCAGTACAT CTACGTATTA GTCATCGCTA TTACCATGGT    720
   GATGCGGTTT TGGCAGTACA TCAATGGGCG TGGATAGCGG TTTGACTCAC GGGGATTTCC    780
   AAGTCTCCAC CCCATTGACG TCAATGGGAG TTTGTTTTGG CACCAAAATC AACGGGACTT    840
   TCCAAAATGT CGTAACAACT CCGCCCCATT GACGCAAATG GGCGGTAGGC GTGTACGGTG    900
   GGAGGTCTAT ATAAGCAGAG CTCTCTGGCT AACTAGAGAA CCCACTGCTT ACTGGCTTAT    960
   CGAAATTAAT ACGACTCACT ATAGGGAGAC CCAAGCTTGG TACCAATTTA AATTGATATC   1020
   TCCTTAGGTC TCGAGCACCA TGAAGTTGCC TGTTAGGCTG TTGGTGCTGA TGTTCTGGAT   1080
   TCCTGCTTCC AGCAGTGATG TTGTCATGAC CCAAACCCCA CTGTCCAGTC CTGTCACGCT   1140
   TGGACAACCT GCGTCCATCT CTTGCAGATC TAGTCAGATC ATTGTACATA ATAATGGCAA   1200
   CACCTATCTG GAATGGTACC AGCAGAGACC AGGGCAGTCT CCACGGCTCC TGATCTACAA   1260
   AGTTTCCAAC CGATTTTCTG GGGTCCCAGA CAGGTTCAGC GGCAGTGGAG CTGGGACAGA   1320
   TTTCACACTC AAGATCAGCA GAGTGGAGGC TGAGGATGTG GGAGTTTACT ACTGCTTCCA   1380
   GGGTTCACAT GTTCCATTCA CGTTCGGCCA AGGGACAAAG TTGGAAATCA AACGTAAGTC   1440
   TCGAGTCTCT AGATAACCGG TCAATCGATT GGAATTCTAA ACTCTGAGGG GGTCGGATGA   1500
   CGTGGCCATT CTTTGCCTAA AGCATTGAGT TTACTGCAAG GTCAGAAAAG CATGCAAAGC   1560
   CCTCAGAATG GCTGCAAAGA GCTCCAACAA AACAATTTAG AACTTTATTA AGGAATAGGG   1620
   GGAAGCTAGG AAGAAACTCA AAACATCAAG ATTTTAAATA CGCTTCTTGG TCTCCTTGCT 1680
   ATAATTATCT GGGATAAGCA TGCTGTTTTC TGTCTGTCCC TAACATGCCC TTATCCGCAA 1740
   ACAACACACC CAAGGGCAGA ACTTTGTTAC TTAAACACCA TCCTGTTTGC TTCTTTCCTC 1800
   AGGAACTGTG GCTGCACCAT CTGTCTTCAT CTTCCCGCCA TCTGATGAGC AGTTGAAATC 1860
   TGGAACTGCC TCTGTTGTGT GCCTGCTGAA TAACTTCTAT CCCAGAGAGG CCAAAGTACA 1920
   GTGGAAGGTG GATAACGCCC TCCAATCGGG TAACTCCCAG GAGAGTGTCA CAGAGCAGGA 1980
   GAGCAAGGAC AGCACCTACA GCCTCAGCAG CACCCTGACG CTGAGCAAAG CAGACTACGA 2040
   GAAACACAAA GTCTACGCCT GCGAAGTCAC CCATCAGGGC CTGAGCTCGC CCGTCACAAA 2100
   GAGCTTCAAC AGGGGAGAGT GTTAGAGGGA GAAGTGCCCC CACCTGCTCC TCAGTTCCAG 2160
   CCTGACCCCC TCCCATCCTT TGGCCTCTGA CCCTTTTTCC ACAGGGGACC TACCCCTATT 2220
   GCGGTCCTCC AGCTCATCTT TCACCTCACC CCCCTCCTCC TCCTTGGCTT TAATTATGCT 2280
   AATGTTGGAG GAGAATGAAT AAATAAAGTG AATCTTTGCA CCTGTGGTTT CTCTCTTTCC 2340
   TCATTTAATA ATTATTATCT GTTGTTTTAC CAACTACTCA ATTTCTCTTA TAAGGGACTA 2400
   AATATGTAGT CATCCTAAGG CACGTAACCA TTTATAAAAA TCATCCTTCA TTCTATTTTA 2460
   CCCTATCATC CTCTGCAAGA CAGTCCTCCC TCAAACCCAC AAGCCTTCTG TCCTCACAGT 2520
   CCCCTGGGCC ATGGTAGGAG AGACTTGCTT CCTTGTTTTC CCCTCCTCAG CAAGCCCTCA 2580
   TAGTCCTTTT TAAGGGTGAC AGGTCTTACA GTCATATATC CTTTGATTCA ATTCCCTGAG 2640
   AATCAACCAA AGCAAATTTT TCAAAAGAAG AAACCTGCTA TAAAGAGAAT CATTCATTGC 2700
   AACATGATAT AAAATAACAA CACAATAAAA GCAATTAAAT AAACAAACAA TAGGGAAATG 2760
   TTTAAGTTCA TCATGGTACT TAGACTTAAT GGAATGTCAT GCCTTATTTA CATTTTTAAA 2820
   CAGGTACTGA GGGACTCCTG TCTGCCAAGG GCCGTATTGA GTACTTTCCA CAACCTAATT 2880
   TAATCCACAC TATACTGTGA GATTAAAAAC ATTCATTAAA ATGTTGCAAA GGTTCTATAA 2940
   AGCTGAGAGA CAAATATATT CTATAACTCA GCAATCCCAC TTCTAGATGA CTGAGTGTCC 3000
   CCACCCACCA AAAAACTATG CAAGAATGTT CAAAGCAGCT TTATTTACAA AAGCCAAAAA 3060
   TTGGAAATAG CCCGATTGTC CAACAATAGA ATGAGTTATT AAACTGTGGT ATGTTTATAC 3120
   ATTAGAATAC CCAATGAGGA GAATTAACAA GCTACAACTA TACCTACTCA CACAGATGAA 3180
   TCTCATAAAA ATAATGTTAC ATAAGAGAAA CTCAATGCAA AAGATATGTT CTGTATGTTT 3240
   TCATCCATAT AAAGTTCAAA ACCAGGTAAA AATAAAGTTA GAAATTTGGA TGGAAATTAC 3300
   TCTTAGCTGG GGGTGGGCGA GTTAGTGCCT GGGAGAAGAC AAGAAGGGGC TTCTGGGGTC 3360
   TTGGTAATGT TCTGTTCCTC GTGTGGGGTT GTGCAGTTAT GATCTGTGCA CTGTTCTGTA 3420
   TACACATTAT GCTTCAAAAT AACTTCACAT AAAGAACATC TTATACCCAG TTAATAGATA 3480
   GAAGAGGAAT AAGTAATAGG TCAAGACCAA CGCAGCTGGT AAGTGGGGGC CTGGGATCAA 3540
   ATAGCTACCT GCCTAATCCT GCCCWCTTGA GCCCTGAATG AGTCTGCCTT CCAGGGCTCA 3600
   AGGTGCTCAA CAAAACAACA GGCCTGCTAT TTTCCTGGCA TCTGTGCCCT GTTTGGCTAG 3660
   CTAGGAGCAC ACATACATAG AAATTAAATG AAACAGACCT TCAGCAAGGG GACAGAGGAC 3720
   AGAATTAACC TTGCCCAGAC ACTGGAAACC CATGTATGAA CACTCACATG TTTGGGAAGG 3780
   GGGAAGGGCA CATGTAAATG AGGACTCTTC CTCATTCTAT GGGGCACTCT GGCCCTGCCC 3840
   CTCTCAGCTA CTCATCCATC CAACACACCT TTCTAAGTAC CTCTCTCTGC CTACACTCTG 3900
   AAGGGGTTCA GGAGTAACTA ACACAGCATC CCTTCCCTCA AATGACTGAC AATCCCTTTG 3960
   TCCTGCTTTG TTTTTCTTTC CAGTCAGTAC TGGGAAAGTG GGGAAGGACA GTCATGGAGA 4020
   AACTACATAA GGAAGCACCT TGCCCTTCTG CCTCTTGAGA ATGTTGATGA GTATCAAATC 4080
   TTTCAAACTT TGGAGGTTTG AGTAGGGGTG AGACTCAGTA ATGTCCCTTC CAATGACATG 4140
   AACTTGCTCA CTCATCCCTG GGGGCCAAAT TGAACAATCA AAGGCAGGCA TAATCCAGTT 4200
   ATGAATTCTT GCGGCCGCTT GCTAGCTTCA CGTGTTGGAT CCAACCGCGG AAGGGCCCTA 4260
   TTCTATAGTG TCACCTAAAT GCTAGAGCTC GCTGATCAGC CTCGACTGTG CCTTCTAGTT 4320
   GCCAGCCATC TGTTGTTTGC CCCTCCCCCG TGCCTTCCTT GACCCTGGAA GGTGCCACTC 4380
   CCACTGTCCT TTCCTAATAA AATGAGGAAA TTGCATCGCA TTGTCTGAGT AGGTGTCATT 4440
   CTATTCTGGG GGGTGGGGTG GGGCAGGACA GCAAGGGGGA GGATTGGGAA GACAATAGCA 4500
   GGCATGCTGG GGATGCGGTG GGCTCTATGG CTTCTGAGGC GGAAAGAACC AGCTGGGGCT 4560
   CTAGGGGGTA TCCCCACGCG CCCTGTAGCG GCGCATTAAG CGCGGCGGGT GTGGTGGTTA 4620
   CGCGCAGCGT GACCGCTACA CTTGCCAGCG CCCTAGCGCC CGCTCCTTTC GCTTTCTTCC 4680
   CTTCCTTTCT CGCCACGTTC GCCGGGCCTC TCAAAAAAGG GAAAAAAAGC ATGCATCTCA 4740
   ATTAGTCAGC AACCATAGTC CCGCCCCTAA CTCCGCCCAT CCCGCCCCTA ACTCCGCCCA 4800
   GTTCCGCCCA TTCTCCGCCC CATGGCTGAC TAATTTTTTT TATTTATGCA GAGGCCGAGG 4860
   CCGCCTCGGC CTCTGAGCTA TTCCAGAAGT AGTGAGGAGG CTTTTTTGGA GGCCTAGGCT 4920
   TTTGCAAAAA GCTTGGACAG CTCAGGGCTG CGATTTCGCG CCAAACTTGA CGGCAATCCT 4980
   AGCGTGAAGG CTGGTAGGAT TTTATCCCCG CTGCCATCAT GGTTCGACCA TTGAACTGCA 5040
   TCGTCGCCGT GTCCCAAAAT ATGGGGATTG GCAAGAACGG AGACCTACCC TGGCCTCCGC 5100
   TCAGGAACGA GTTCAAGTAC TTCCAAAGAA TGACCACAAC CTCTTCAGTG GAAGGTAAAC 5160
   AGAATCTGGT GATTATGGGT AGGAAAACCT GGTTCTCCAT TCCTGAGAAG AATCGACCTT 5220
   TAAAGGACAG AATTAATATA GTTCTCAGTA GAGAACTCAA AGAACCACCA CGAGGAGCTC 5280
   ATTTTCTTGC CAAAAGTTTG GATGATGCCT TAAGACTTAT TGAACAACCG GAATTGGCAA 5340
   GTAAAGTAGA CATGGTTTGG ATAGTCGGAG GCAGTTCTGT TTACCAGGAA GCCATGAATC 5400
   AACCAGGCCA CCTTAGACTC TTTGTGACAA GGATCATGCA GGAATTTGAA AGTGACACGT 5460
   TTTTCCCAGA AATTGATTTG GGGAAATATA AACTTCTCCC AGAATACCCA GGCGTCCTCT 5520
   CTGAGGTCCA GGAGGAAAAA GGCATCAAGT ATAAGTTTGA AGTCTACGAG AAGAAAGACT 5580
   AACAGGAAGA TGCTTTCAAG TTCTCTGCTC CCCTCCTAAA GCTATGCATT TTTATAAGAC 5640
   CATGGGACTT TTGCTGGCTT TAGATCTCTT TGTGAAGGAA CCTTACTTCT GTGGTGTGAC 5700
   ATAATTGGAC AAACTACCTA CAGAGATTTA AAGCTCTAAG GTAAATATAA AATTTTTAAG 5760
   TGTATAATGT GTTAAACTAC TGATTCTAAT TGTTTGTGTA TTTTAGATTC CAACCTATGG 5820
   AACTGATGAA TGGGAGCAGT GGTGGAATGC CTTTAATGAG GAAAACCTGT TTTGCTCAGA 5880
   AGAAATGCCA TCTAGTGATG ATGAGGCTAC TGCTGACTCT CAACATTCTA CTCCTCCAAA 5940
   AAAGAAGAGA AAGGTAGAAG ACCCCAAGGA CTTTCCTTCA GAATTGCTAA GTTTTTTGAG 6000
   TCATGCTGTG TTTAGTAATA GAACTCTTGC TTGCTTTGCT ATTTACACCA CAAAGGAAAA 6060
   AGCTGCACTG CTATACAAGA AAATTATGGA AAAATATTCT GTAACCTTTA TAAGTAGGCA 6120
   TAACAGTTAT AATCATAACA TACTGTTTTT TCTTACTCCA CACAGGCATA GAGTGTCTGC 6180
   TATTAATAAC TATGCTCAAA AATTGTGTAC CTTTAGCTTT TTAATTTGTA AAGGGGTTAA 6240
   TAAGGAATAT TTGATGTATA GTGCCTTGAC TAGAGATCAT AATCAGCCAT ACCACATTTG 6300
   TAGAGGTTTT ACTTGCTTTA AAAAACCTCC CACACCTCCC CCTGAACCTG AAACATAAAA 6360
   TGAATGCAAT TGTTGTTGTT AACTTGTTTA TTGCAGCTTA TAATGGTTAC AAATAAAGCA 6420
   ATAGCATCAC AAATTTCACA AATAAAGCAT TTTTTTCACT GCATTCTAGT TGTGGTTTGT 6480
   CCAAACTCAT CAATGTATCT TATCATGTCT GGATCGGCTG GATGATCCTC CAGCGCGGGG 6540
   ATCTCATGCT GGAGTTCTTC GCCCACCCCA ACTTGTTTAT TGCAGCTTAT AATGGTTACA 6600
   AATAAAGCAA TAGCATCACA AATTTCACAA ATAAAGCATT TTTTTCACTG CATTCTAGTT 6660
   GTGGTTTGTC CAAACTCATC AATGTATCTT ATCATGTCTG TATACCGTCG ACCTCTAGCT 6720
   AGAGCTTGGC GTAATCATGG TCATAGCTGT TTCCTGTGTG AAATTGTTAT CCGCTCACAA 6780
   TTCCACACAA CATACGAGCC GGAAGCATAA AGTGTAAAGC CTGGGGTGCC TAATGAGTGA 6840
   GCTAACTCAC ATTAATTGCG TTGCGCTCAC TGCCCGCTTT CCAGTCGGGA AACCTGTCGT 6900
   GCCAGCTGCA TTAATGAATC GGCCAACGCG CGGGGAGAGG CGGTTTGCGT ATTGGGCGCT 6960
   CTTCCGCTTC CTCGCTCACT GACTCGCTGC GCTCGGTCGT TCGGCTGCGG CGAGCGGTAT 7020
   CAGCTCACTC AAAGGCGGTA ATACGGTTAT CCACAGAATC AGGGGATAAC GCAGGAAAGA 7080
   ACATGTGAGC AAAAGGCCAG CAAAAGGCCA GGAACCGTAA AAAGGCCGCG TTGCTGGCGT 7140
   TTTTCCATAG GCTCCGCCCC CCTGACGAGC ATCACAAAAA TCGACGCTCA AGTCAGAGGT 7200
   GGCGAAACCC GACAGGACTA TAAAGATACC AGGCGTTTCC CCCTGGAAGC TCCCTCGTGC 7260
   GCTCTCCTGT TCCGACCCTG CCGCTTACCG GATACCTGTC CGCCTTTCTC CCTTCGGGAA 7320
   GCGTGGCGCT TTCTCAATGC TCACGCTGTA GGTATCTCAG TTCGGTGTAG GTCGTTCGCT 7380
   CCAAGCTGGG CTGTGTGCAC GAACCCCCCG TTCAGCCCGA CCGCTGCGCC TTATCCGGTA 7440
   ACTATCGTCT TGAGTCCAAC CCGGTAAGAC ACGACTTATC GCCACTGGCA GCAGCCACTG 7500
   GTAACAGGAT TAGCAGAGCG AGGTATGTAG GCGGTGCTAC AGAGTTCTTG AAGTGGTGGC 7560
   CTAACTACGG CTACACTAGA AGGACAGTAT TTGGTATCTG CGCTCTGCTG AAGCCAGTTA 7620
   CCTTCGGAAA AAGAGTTGGT AGCTCTTGAT CCGGCAAACA AACCACCGCT GGTAGCGGTG 7680
   GTTTTTTTGT TTGCAAGCAG CAGATTACGC GCAGAAAAAA AGGATCTCAA GAAGATCCTT 7740
   TGATCTTTTC TACGGGGTCT GACGCTCAGT GGAACGAAAA CTCACGTTAA GGGATTTTGG 7800
   TCATGAGATT ATCAAAAAGG ATCTTCACCT AGATCCTTTT AAATTAAAAA TGAAGTTTTA 7860
   AATCAATCTA AAGTATATAT GAGTAAACTT GGTCTGACAG TTACCAATGC TTAATCAGTG 7920
   AGGCACCTAT CTCAGCGATC TGTCTATTTC GTTCATCCAT AGTTGCCTGA CTCCCCGTCG 7980
   TGTAGATAAC TACGATACGG GAGGGCTTAC CATCTGGCCC CAGTGCTGCA ATGATACCGC 8040
   GAGACCCACG CTCACCGGCT CCAGATTTAT CAGCAATAAA CCAGCCAGCC GGAAGGGCCG 8100
   AGCGCAGAAG TGGTCCTGCA ACTTTATCCG CCTCCATCCA GTCTATTAAT TGTTGCCGGG 8160
   AAGCTAGAGT AAGTAGTTCG CCAGTTAATA GTTTGCGCAA CGTTGTTGCC ATTGCTACAG 8220
   GCATCGTGGT GTCACGCTCG TCGTTTGGTA TGGCTTCATT CAGCTCCGGT TCCCAACGAT 8280
   CAAGGCGAGT TACATGATCC CCCATGTTGT GCAAAAAAGC GGTTAGCTCC TTCGGTCCTC 8340
   CGATCGTTGT CAGAAGTAAG TTGGCCGCAG TGTTATCACT CATGGTTATG GCAGCACTGC 8400
   ATAATTCTCT TACTGTCATG CCATCCGTAA GATGCTTTTC TGTGACTGGT GAGTACTCAA 8460
   CCAAGTCATT CTGAGAATAG TGTATGCGGC GACCGAGTTG CTCTTGCCCG GCGTCAATAC 8520
   GGGATAATAC CGCGCCACAT AGCAGAACTT TAAAAGTGCT CATCATTGGA AAACGTTCTT 8580
   CGGGGCGAAA ACTCTCAAGG ATCTTACCGC TGTTGAGATC CAGTTCGATG TAACCCACTC 8640
   GTGCACCCAA CTGATCTTCA GCATCTTTTA CTTTCACCAG CGTTTCTGGG TGAGCAAAAA 8700
   CAGGAAGGCA AAATGCCGCA AAAAAGGGAA TAAGGGCGAC ACGGAAATGT TGAATACTCA   8760
   TACTCTTCCT TTTTCAATAT TATTGAAGCA TTTATCAGGG TTATTGTCTC ATGAGCGGAT   8820
   ACATATTTGA ATGTATTTAG AAAAATAAAC AAATAGGGGT TCCGCGCACA TTTCCCCGAA   8880
   AAGTGCCACC TGACGTC                                                  8897